(19) Europäisches Patentamt
European Patent Office
Office européen des brevets

(11) Publication number: **0 300 688 A1**

# (12) EUROPEAN PATENT APPLICATION

(21) Application number: 88306464.4

(22) Date of filing: 14.07.88

(51) Int. Cl.[4]: C07D 207/34 , C07D 207/333 , C07D 403/06 , C07D 405/06 , C07D 409/06 , A61K 31/40 , C07D 207/00 , C07D 401/00 , C07D 405/00 , C07D 409/00

The title of the invention has been amended (Guidelines for Examination in the EPO, A-III, 7.3).

(30) Priority: 21.07.87 GB 8717193
24.12.87 GB 8730116

(43) Date of publication of application: 25.01.89 Bulletin 89/04

(84) Designated Contracting States: AT BE CH DE ES FR GB GR IT LI LU NL SE

(71) Applicant: FISONS plc
Fison House Princes Street
Ipswich Suffolk IP1 1QH(GB)

(72) Inventor: Dixon, John
Church Farmhouse Main Street Great Dalby
Melton Mowbray Leicestershire(GB)
Inventor: Baxter, Andrew John Gilby
Flat 4,6 Western Terrace
The Park Nottingham(GB)
Inventor: Manners, Carol Nancy
6 Cohen Close
Arnold Nottingham(GB)
Inventor: Teague, Simon
87 Victor Crescent
Sandiacre Nottingham(GB)

(74) Representative: Wright, Robert Gordon McRae
Fisons plc 12 Derby Road
Loughborough, Leicestershire LE11 0BB(GB)

## (54) Pyrrole derivatives, process for their preparation and pharmaceutical compositions containing them.

(57) Compounds of formula I,

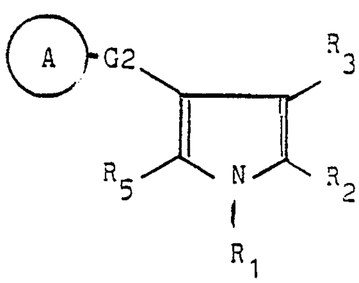

I

wherein
$R_1$, $R_2$, $R_3$ and $R_5$ have the meanings defined in the specification,

EP 0 300 688 A1

G2 represents a chain $-(CH_2)_z(W)_y-$, in which W has various meanings including C = O, and up to two of the methylene segments in the chain $(CH_2)_z$ are optionally replaced by -NH- and one segment is optionally replaced by -O- or $-(C=NR_{40})-$, and the chain is optionally unsaturated and optionally substituted, and

A is a 5- or 6-membered ring or a bicyclic or tricyclic fused ring system, A being optionally substituted by various substituents,

possess useful pharmacological properties, in particular as cardiotonics.

Also described are processes for the preparation of compounds of formula I, pharmaceutical compositions containing them and methods of treatment involving their use.

## COMPOUNDS

This invention relates to new compounds having a common pyrrole structure, new compositions containing the compounds and processes for their preparation.

According to the invention there are provided compounds of formula I,

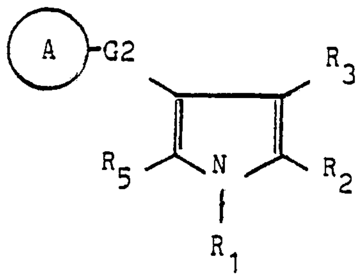

I

wherein

$R_1$ represents $-R_{11}$, $-NHR_{11}$ or $-NHCOOR_{11}$ in which $R_{11}$ is hydrogen or alkyl $C_{1-6}$,

$R_2$ and $R_5$, which may be the same or different, represent hydrogen, $-R_{21}$, -OH, $-OR_{21}$, $-NR_{22}R_{23}$, halogen, -CH, $-NO_2$, $-S(O)_qR_{21}$, $-N=NR_{24}$, $-COOR_{22}$, $-CH=R_{25}$, phenyl or pyridyl,

in which $R_{21}$ is alkyl or alkenyl $C_{1-6}$ optionally interrupted by O or S and optionally substituted by -OH, halogen, -CN, $-NO_2$, $-NR_{22}-R_{23}$, $-COOR_{22}$, phenyl or $-OOCR_{26}$,

$R_{22}$ and $R_{23}$, which may be the same or different, are hydrogen or alkyl $C_{1-6}$,

$R_{24}$ is phenyl optionally substituted by $-NO_2$,

$R_{25}$ is =O, =CHCN, =NOH or $=NNHCONH_2$,

$R_{26}$ is alkyl $C_{1-6}$, and

q is O, 1 or 2,

G2 represents a chain $-(CH_2)_z(W)_y-$, in which

W represents C=O, $S(O)_q$ or $C=NR_{40}$,

in which $R_{40}$ is hydrogen, -OH, cycloalkyl $C_{3-8}$ or alkyl $C_{1-6}$ optionally substituted by phenyl,

y is 0, 1 or, provided W is C=O, 2,

z is 0, 1, 2 or 3, and

up to two of the methylene segments in the chain $(CH_2)_z$ are optionally replaced by -NH- and one segment is optionally replaced by -O- or $-(C=NR_{40})-$, and the chain is optionally unsaturated and optionally substituted by alkyl $C_{1-6}$ or alkoxy $C_{1-6}$,

A is a 5- or 6-membered ring or a bicyclic or tricyclic fused ring system, A being optionally substituted by one or more of halogen, -OH, $-NO_2$, $-NR_{43}R_{45}$, $-COOR_{43}$, $-S(O)_qR_{44}$, $-CONR_{43}R_{45}$, $-NHCOR_{45}$, alkyl, alkylene or alkoxy $C_{1-6}$ optionally substituted by halogen, phenyl, $-COOR_{43}$ or $-NR_{43}R_{45}$, or a group D-G3-, in which

$R_{43}$ and $R_{45}$, which may be the same or different, are hydrogen or alkyl $C_{1-6}$,

$R_{44}$ is alkyl $C_{1-6}$,

G3 is a single bond, -NH-, -O-, -CO-, $-OCH_2-$, -O-CO-, $-S(O)_q-$, $-(CH_2)_p-$, $-CH_2S(O)_q-$, -CO-S-, $-SO_2-O-$ or $-CH_2NH-$, in which p is 1,2 or 3, and

D represents a 5- or 6-membered ring optionally substituted by -OH, halogen, $-NO_2$, $-NR_{43}R_{45}$, alkyl $C_{1-6}$, alkoxy $C_{1-6}$ or $-SCH_2C_6H_5$, and

$R_3$ represents hydrogen, $-S(O)_qR_{31}$, $-NO_2$, -CN, -halogen, $-CH_2NR_{32}R_{33}$, $-(CO)_rR_{34}$ or an aryl group, in which

$R_{31}$ is alkyl $C_{1-6}$,

$R_{32}$ and $R_{33}$, which may be the same or different, are hydrogen, alkyl $C_{1-6}$ optionally substituted by phenyl, or phenyl optionally substituted by halogen or trihalomethyl,

r is 1 or 2,

$R_{34}$ is hydrogen, $-NR_{32}-R_{33}$, alkyl $C_{1-6}$, -OH, $-OR_{35}$ or $-SR_{35}$,

$R_{35}$ is cycloalkyl $C_{3-8}$ or alkyl $C_{1-6}$ optionally substituted by alkoxy $C_{1-6}$, halogen, -OH, cycloalkyl $C_{3-8}$, $-NR_{32}R_{33}$ or $-COOR_{36}$, and

$R_{36}$ is hydrogen or alkyl $C_{1-6}$, or $R_3$ and $R_2$ together form a chain -N=CH-NH-CO-,

provided that

a) when $R_3$ represents hydrogen, then A is substituted by D-G3- where, if A is a 5-membered heterocyclic ring, D-G3- is other than phenyl, benzyl or phenyl substituted by chlorine, or, if A is phenyl or naphthyl and D is phenyl, G3 is other than $-CH_2-O-$, -S-, -O-or -NH-;

b) when $R_2$ and $R_5$ both represent hydrogen, then A is substituted by D-G3-;

c) when $R_1$ represents alkyl $C_{1-6}$ or $-NHCOOR_{11}$, and $R_2$ and $R_5$ are both alkyl $C_{1-6}$, and $R_3$ is $-COOR_{35}$ or $-NO_2$, then A is other than unsubstituted phenyl or phenyl substituted by fluorine;

d) when $R_2$ and $R_3$ both represent halogen, and A is phenyl, then A is substituted by D-G3-;

e) when one or both or $R_2$ and $R_5$ represents phenyl, and $R_3$ represents $-COOR_{35}$, $-COCH_3$, phenyl or $-NO_2$, then A is other than unsubstituted phenyl, isoxazolyl substituted by alkyl $C_{1-6}$ or phenyl, or a group

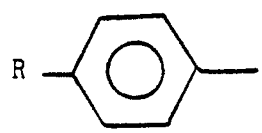

where R is halogen, alkyl $C_{1-6}$ or alkoxy $C_{1-6}$

f) when $R_5$ represents hydrogen, and $R_2$ represents alkyl $C_{1-6}$, and $R_3$ represents $-COOR_{35}$, and A is a 5- or 6-membered ring, then A is substituted by D-G3-; and

g) when $R_2$ and $R_5$ are both selected from hydrogen, $-COOR_{22}$ and alkyl $C_{1-6}$ optionally substituted by halogen, -OH, $-NR_{22}-R_{23}$ or $-OOCR_{26}$ and optionally interrupted by O or S, and $R_3$ is selected from $-NO_2$, halogen, -CN, $-COR_{34}$ or $-CH_2NR_{32}R_{34}$, where $R_{34}$ is hydrogen, $-NR_{32}R_{34}$, alkyl $C_{1-6}$, -OH or $-OR_{35}$ where $R_{35}$ is alkyl $C_{1-6}$ optionally substituted by an aryl group, and $R_1$ represents hydrogen or alkyl $C_{1-6}$, and G2 represents $-(CH_2)_z(W)_y-$ in which z represents 0, 1 or 2 and none of the methylene segments are replaced by -NH-, -O- or $-(C=NR_{40})-$ and the chain is neither unsaturated not substituted by alkoxy $C_{1-6}$, then A is other than phenyl, naphthyl or benzofuranzanyl optionally substituted only by halogen, -OH, $-NR_{34}R_{45}$, $-NO_2$, -COOH, alkyl or alkoxy $C_{1-6}$ optionally substituted by halogen, or D-G3- where G3 is $-SCH_2-$, $-OCH_2-$ or -O-CO- and D is phenyl optionally substituted by -OH, halogen or alkyl or alkoxy $C_{1-6}$;

and pharmaceutically acceptable derivatives thereof.

Compounds of formula I, and pharmaceutically acceptable derivatives thereof, may be prepared by the following processes:

a) producing a compound of formula I in which $R_3$ or G2 includes a carbonyl group adjacent to the pyrrole ring by reacting a compound of formula IIa or IIb,

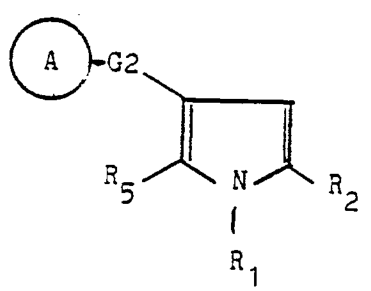

IIa

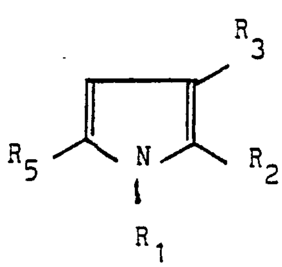

IIb

with a compound of formula IIIa or IIIb respectively,

$R_{3a}$-CO-$L_a$ IIIa

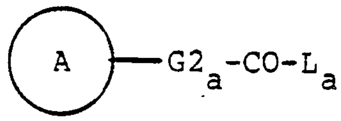

IIIb

in which $R_{3a}CO$- and G2-$_aCO$- represent groups $R_3$ and G2 respectively and $L_a$ is a leaving group, or

b) producing a compound of formula I which is substituted by D-G3c- or alkylene $C_{1-6}$ optionally substituted by -$COOR_{41}$ or -$NR_{43}R_{45}$ in which -G3c-represents -NH-, -S-, -$CH_2NH$- or alkylene $C_{1-6}$, by reacting a compound of formula I substituted by a leaving group with a compound of formula IV,

$Ar_c$-H IV

in which $Ar_c$- represents D-G3c- or alkylene $C_{1-6}$ optionally substituted by -$COOR_{43}$ or -$NR_{43}$-$R_{45}$, or

c) producing a compound of formula I which contains a substituent halogen, -CHO, -CH, -$NO_2$, -S(O)-$_qR_{21}$, -$CONH_2$, -$CH_2NR_{32}R_{33}$ or -N=N-$R_{24}$,
by reacting a corresponding compound containing a hydrogen atom with an appropriate electrophile and, where necessary, appropriate transformation of the product, or

d) producing a compound of formula I in which one or both of $R_2$ and $R_5$ represents hydrogen,
by reacting a compound of formula V

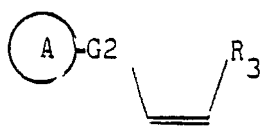

V

with a compound of formula VI

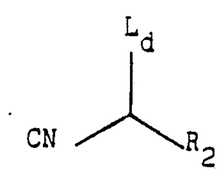

VI

in which $L_d$ represents a leaving group,

e) reacting a compound of formula VII

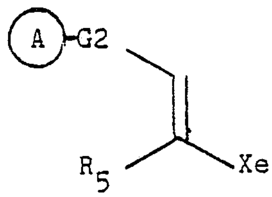

VII

with a compound of formula VIII

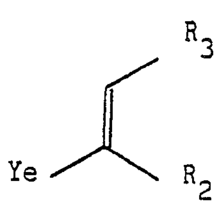

VIII

in which one of $X_e$ and $Y_e$ represents a leaving group, and the other represents $-NHR_1$, and where necessary, working up the reaction product,

f) reacting a compound of formula IX,

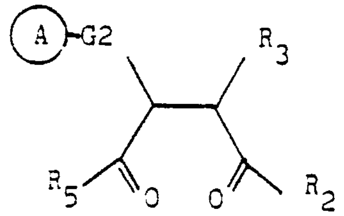

IX

with a compound of formula X,

$R_1-NH_2$ X

g) removal of a protecting group from a compound of formula I which bears a protecting group,

h) producing a compound of formula I in which G2 includes a group $-(CO)_y-$ adjacent to the pyrrole ring,
by reacting a compound of formula XI,

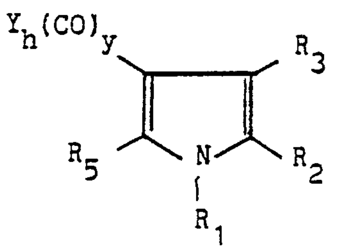

XI

with a compound of formula XII

A $G2_h-X_h$ XII

in which $G2_h$ is a group which, in combination with the group $-(CO)_y-$ forms G2 and one of $X_h$ and $Y_h$ is a leaving group and the other is hydrogen, and, if necessary, working up the reaction product,

i) producing a compound of formula I in which A is a 5-or 6-membered ring or a bicyclic or tricyclic fused ring system by appropriate treatment of a corresponding compound in which

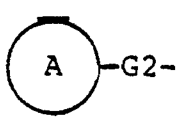

contains groups cyclisable to form such a ring or fused ring system respectively,

j) producing a compound of formula I in which G2 represents $-NHC(=NH)NH-(CO)_y-$
by reacting a compound of formula XIII,

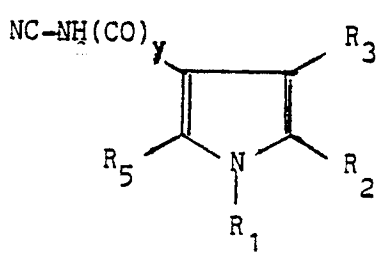

XIII

with a compound of formula XIV,

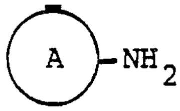

XIV

and, where necessary or desired thereafter, converting the compound of formula I formed in any of the above reactions to a pharmaceutically acceptable derivative thereof, or vice versa.

In addition to the processes described above, further compounds of formula I may be prepared by processes involving substitution or other modification of other compounds of formula I. Such processes include:

k) producing a compound of formula I containing a group $-COOR_k$ or $-COSR_k$ in which $R_k$ represents cycloalkyl $C_{3-8}$ or alkyl $C_{1-6}$ optionally substituted by halogen, $-NR_{32}R_{33}$ or $-COOR_{35}$,
by esterification or thioesterification respectively of the corresponding compound containing a group -COOH,

l) producing a compound of formula I containing a substituent $-NH_2$,
by reduction of the corresponding compound containing a substituent $-NO_2$,

m) producing a compound of formula I containing a group -COOH
by hydrolysis of a corresponding ester,

n) deamination of a compound of formula I containing a substituent $-NH_2$,

o) producing a compound of formula I containing an alkyl $C_{1-6}$ group substituted by a group $-OOCR_{26}$,
by alkanoyloxylation of the alkyl $C_{1-6}$ group,

p) producing a compound of formula I containing a hydroxy group,
by reacting the corresponding compound containing a group $-OOCR_{26}$ with a hydroxylic base,

q) producing a compound of formula I containing an amido group,
by acylation of the corresponding amine or by reacting a corresponding carboxylic acid or carboxylic acid derivative with an appropriate amine,

r) producing a compound of formula I containing a group $-SR_r$ in which $R_r$ represents alkyl $C_{1-6}$ by diazotisation of the corresponding compound containing a group $-NH_2$ and subsequent displacement,

s) producing a compound of formula I containing an aliphatic -CH=N- group by condensation of the appropriate aldehyde and amine,

t) producing a compound of formula I containing a group -SO- or $-SO_2-$,
by oxidation of the corresponding compound containing a group -S- or -SO-, and, where necessary or desired thereafter, converting the compound of formula I formed in any of the above reactions to a pharmaceutically acceptable derivative thereof, or vice versa.

The reaction conditions which may be employed in the above processes are illustrated, though not limited, in the Examples described below. However, the following general comments may be made:

The reaction of process a) is most conveniently carried out under Friedel-Crafts acylation conditions ie in the presence of a Lewis acid, eg aluminium chloride, in a solvent which is inert to the reaction conditions, eg a halogenated hydrocarbon solvent, at a temperature of from about 0 to 75 °C. Leaving groups that $L_a$ may represent include halide, particularly chloride.

Alternatively, the reaction of process a) may proceed via an organometallic intermediate. For example, the compound of formula IIa or IIb may be treated with an alkyl magnesium halide (a Grignard reagent), eg ethyl magnesium bromide, prior to reaction with the compound of formula IIIa or IIIb.

The reaction of process b) is preferably carried out in the presence of a catalyst at a temperature of from about 0 to 150 °C. Suitable catalysts include copper (I) oxide, copper bronze, nickel (II) bromide and various palladium (0) species. The reaction may be performed in the compound of formula IV as solvent.

The reaction of process c) may be carried out using conventional electrophilic substitution techniques. For example, when the species to be introduced is:-

i. halogen, a suitable electrophile is $Hal^+$ in which Hal represents halogen. Bromination, for example, may be carried out using pyridinium bromide perbromide.

ii. -CHO, the reaction may be carried out under Vilsmeier-Haack conditions ie using phosphorous oxychloride and a disubstituted formamide.

iii. -CH, the reaction may be performed using chlorosulphonyl isocyanate followed by treatment with dimethylformamide.

iv. $-CONH_2$, the reaction may be performed using chlorosulphonyl isocyanate followed by treatment with sodium sulphate.

v. $-NO_2$, a suitable electrophile is the nitronium ion $NO_2^+$. For example, the reaction may be carried out using acetyl nitrate in a polar solvent, eg acetic anhydride, or using a nitronium salt, eg nitronium tetrafluoroborate.

vi. $-SR_{2'}$, the reaction may be carried out using the appropriate alkyl thiohalide eg the thiochloride.

vii. $-N=N-R_{24}$, the reaction may be carried out using a diazonium salt such as the tetrafluoroborate. The reaction is preferably carried out at a temperature of from about 0 to 50 °C.

viii. $-CH_2NR_{32}R_{33}$, the reaction may be performed by treatment with formaldehyde and an amine $HNR_{32}R_{33}$.

The reaction of process d) is preferably carried out in a solvent which is inert to the reaction conditions at a temperature of from about 0 to 100 °C. A preferred leaving group which $L_d$ may represent is (4-methylphenyl)sulphonyl.

The reaction of process e) is preferably carried out at a temperature of from about 50 to 150 °C. Leaving groups that $X_e$ and $Y_e$ may represent include hydroxy, halide and nitro. Where the leaving group is hydroxy, the compound of formula VII or VIII may exist as the corresponding ketone tautomer.

The reaction of process f) is preferably carried out in a solvent which is inert to the reaction conditions at a temperature of from about 0 to 150 °C.

Protecting groups which may be removed in process g) include, for example, groups protecting the pyrrole nitrogen and groups protecting hydroxy groups. The method used to remove the protecting group will depend on the nature of the protecting group, but conventional techniques may generally be employed, including acidic and basic cleavage, and hydrogenolysis.

The reaction of process h) is preferably carried out in an inert solvent at a temperature of from about 0 to 100 °C. Leaving groups which may be employed include trihalomethyl, eg trichloromethyl, and alkoxy $C_{1-6}$, eg ethoxy.

The conditions used in the reaction of process i) will depend on the particular compound to be synthesised. However, in the case of heterocyclic ring formation, the reaction will generally involved nucleophilic attack on a carbonyl group with subsequent loss of $H_2O$ leading to formation of a double bond.

The reaction of process j) is preferably carried out at a temperature of from about 0 to 150°. In some cases, the reaction may be performed in the compound of formula XIV as solvent.

The reaction of process k) may be carried out using conventional esterification (or thioesterification) techniques. For example, the carboxylic acid may be treated with an inorganic halide followed by addition of the appropriate alcohol or thiol. The inorganic halide is preferably phosphorous pentachloride and the reaction is preferably carried out in an inert solvent at a temperature of from about 0 to 50 °C.

The reaction of process 1) may be performed by catalytic hydrogenation, preferably using a metal or metal oxide catalyst, eg $PtO_2$ or Pd.

The reaction of process m) may be performed using conventional hydrolysis techniques, preferably at a temperature of from about -50° to 50 °C in an inert solvent.

The reaction of process n) is preferably carried out by diazotisation followed by treatment with hypophosphorous acid. The reaction is preferably carried out in a polar solvent at a temperature of from about -50° to 50 °C.

The alkanoyloxylation of process o) may be carried out using a metallic alkanoate. For example, when $R_{25}$ represents methyl, lead tetraacetate can be used. The reaction is preferably carried out at a temperature of from about 50 to 150 °C.

Suitable hydroxylic bases for use in process p) include lithium hydroxide hydrate. The temperature at which the reaction is performed is preferably from about 0 to 100 °C.

The reaction of process q) may be carried out using conventional techniques eg acylation using the appropriate acid chloride or anhydride.

The diazotisation of process r) may be performed by conventional means. The subsequent displacement may be accomplished by treatment with, for example, a dialkyldisulphide.

The reaction of process s) may be carried out using conventional techniques, preferably in a polar solvent at a temperature of from about 0 to 150 °C.

The reaction of process t) is preferably performed in an inert solvent at a temperature of from about 0 to 100 °C, more preferably 0 to 50 °C. Oxidising agents which may be used include 3-chlorobenzeneperoxoic acid. Compounds of formula I containing a group -SO- or $-SO_2-$may be produced from the corresponding compound containing the group -S- by using about 1.1 and about 3 equivalents of oxidising agent respectively.

The compounds of formulae II - XIV are either known or may be prepared by known methods, for

example the methods described in the Examples.

When any of the starting materials or intermediates contain a chiral centre they may be resolved using conventional techniques.

The processes as described above may produce the compound of formula I or a derivative thereof. It is also within the scope of this invention to treat any derivative so produced to liberate the free compound of formula I, or to convert one derivative into another.

The compounds of formula I and the intermediates thereto may be isolated from their reaction mixtures using conventional techniques.

Pharmaceutically acceptable derivatives of the compounds of formula I include derivatives of the pyrrole nitrogen, eg compounds in which $R_1$ represents $-CH_2OCOR_{10}$, wherein $R_{10}$ represents alkyl, aryl or N-$(alkyl)_2$.

Alkyl groups which the following groups may represent include:

$R_1$ and $R_{11}$ : methyl, ethyl, n-propyl, iso-propyl, n-butyl, s-butyl and tert-butyl.

$R_{21}$, $R_{22}$, $R_{23}$, $R_{26}$, $R_{31}$, $R_{32}$, $R_{33}$, $R_{34}$, $R_{35}$, $R_{36}$, $R_{43}$, $R_{44}$ and $R_{45}$ : methyl, ethyl, n-propyl and iso-propyl.

Cycloalkyl $C_{3-8}$ groups which may occur in the compound of formula I include cyclopropyl, cyclobutyl, cyclopentyl and cyclohexyl.

The term 'halogen' wherever used herein includes fluorine, chlorine, bromine and iodine.

Aryl groups that $R_3$ may represent include carbocyclic groups, eg phenyl, and heterocyclic groups, including N-, O- and S-heterocycles and rings containing two or three like or unlike heteroatoms, eg oxadiazolyl. The carbocyclic or heterocyclic groups may be 5- or 6-membered.

We prefer compounds in which $R_1$ represents hydrogen.

We prefer compounds in which $R_2$ and $R_5$ do not both represent phenyl.

We prefer compounds in which $R_2$ and $R_5$ are selected from the group consisting of hydrogen, $-R_{21}$, -OH, $-OR_{21}$, $-NR_{22}R_{23}$, halogen, -CN, $-NO_2$, $-S(O)_qR_{21}$, $-N=NR_{24}$, $-COOR_{22}$, $-CH=R_{25}$ or pyridyl, more preferably from the group comprising hydrogen, $-R_{21}$, $-NR_{22}R_{23}$, halogen, -CN, $-NO_2$, $-S(O)_qR_{21}$, $-N=NR_{24}$, $-CH=R_{25}$ or pyridyl. Compounds in which $R_2$ and $R_5$ are selected from hydrogen, $-R_{21}$ (especially methyl, ethyl, thiomethyl and ethenyl, any of which being optionally substituted by, for example, -CN or $-OOCR_{26}$) and halogen are particularly preferred.

$R_3$ is preferably selected from the group consisting of hydrogen, $-S(O)_qR_{31}$, $-NO_2$, -CN, -halogen, $-CH_2NR_{32}R_{33}$ and $-(CO)_rR_{34}$, more preferably from the group consisting of $-NO_2$, -CN, -halogen and $-(CO)_rR_{34}$. Compounds in which $R_3$ represents $-COOR_{35}$ or $-NO_2$ are especially preferred.

We prefer no more than two, and more particularly no more than one, of $R_2$, $R_3$ and $R_5$ to represent halogen.

We prefer no more than two, and more particularly no more than one, of $R_2$, $R_3$ and $R_5$ to represent hydrogen.

Preferred definitions of G2 are those in which z is zero, y is 1 and W represents C=O or $S(O)_q$, especially where q is zero, and where both y and z are zero.

Where A represents a ring, it is preferably a carbocyclic ring, more preferably a 6-membered ring, especially phenyl.

Heterocyclic rings which A may represent include O-, S- and, more preferably, N-heterocycles. Where A is a heterocyclic ring, it is preferably a 5-membered ring, especially pyrrolyl.

Where A is a fused ring system, it may be carbocyclic, eg naphthyl, fluorenyl or anthracenyl, or heterocyclic, eg quinolyl, isoquinolyl, benzimidazolyl, benzothiazolyl or dibenzazepinyl.

Where A is a 5- or 6-membered ring, it is preferably substituted, more preferably by only 1 substituent. The ring A is preferably substituted by D-G3-.

D may represent a carbocyclic or heterocyclic ring, eg an O-, S- or N-heterocycle. D is preferably a 6-membered ring, eg phenyl or pyridyl.

G3 is preferably a chain of two or, more preferably, one segment. Particularly preferred definitions of G3 are -NH- and, especially, $-CH_2-$.

As a specific group of compounds we provide compounds of formula I in which

$R_1$ is as first defined above,

$R_2$ and $R_5$, which may be the same or different, represent

$-R_{21}$ (where $R_{21}$ represents alkyl $C_{1-6}$ optionally substituted by -OH, fluorine, $-NO_2$, $-COOR_{21}$, $-NR_{22}R_{23}$ or $-OOCR_{26}$ where $R_{26}$ is alkyl $C_{1-6}$), -OH, $-OR_{21}$ (where $R_{21}$ represents alkyl $C_{1-6}$), $-NR_{22}R_{23}$, halogen, -CN, $-NO_2$, $-S(O)_qR_{21}$ (where $R_{21}$ represents alkyl $C_{1-6}$), $-N=NR_{24}$, $-COOR_{22}$ (where $R_{22}$ represents hydrogen or alkyl $C_{1-6}$) or phenyl,

G2 represents C=O,

A is a 5- or 6-membered ring optionally substituted by alkyl $C_{1-6}$, halogen, $-COOR_{43}$, $-S(O)_qR_{44}$,

$-CONHR_{43}$, $-NHCOR_{45}$, alkoxy $C_{1-6}$ or a group D-G3-, in which
G3 is a single bond, -NH-, -O-, -CO-, $-S(O)_q-$, $-(CH_2)_p-$, $-S(O)_qCH_2-$ or -CO-S-, in which p is 1 or 2, and
D represents a 6-membered ring optionally substituted by $-NO_2$, $-NH_2$, alkyl $C_{1-6}$ or $-SCH_2C_6H_5$, and
$R_3$ represents $-S(O)_qR_{31}$, $-NO_2$, an aryl group or $-(CO)_rR_{34}$ (where $R_{34}$ is -OH, $-OR_{35}$ or $-SR_{35}$, $R_{35}$ being cycloalkyl $C_{3-8}$ or alkyl $C_{1-6}$ optionally substituted by alkoxy $C_{1-6}$, halogen, -OH, cycloalkyl $C_{3-8}$ or $-NR_{32}R_{33}$ where $R_{32}$ and $R_{33}$ are hydrogen or alkyl $C_{1-6}$).

As a second specific group, there are provided compounds of formula I in which
$R_1$ is as first defined above,
$R_2$ and $R_5$, which may be the same or different, represent hydrogen, $-R_{21}$ (where $R_{21}$ represents alkyl $C_{1-6}$ optionally substituted by -OH, fluorine, $-NO_2$, $-COOR_{21}$, $-NR_{22}R_{23}$ or $-OOCR_{26}$ where $R_{26}$ is alkyl $C_{1-6}$), -OH, $-OR_{21}$ (where $R_{21}$ represents alkyl $C_{1-6}$), $-NR_{22}R_{23}$, halogen, -CN, $-NO_2$, $-S(O)_qR_{21}$ (where $R_{21}$ represents alkyl $C_{1-6}$), $-N=NR_{24}$, $-COOR_{22}$ (where $R_{22}$ represents hydrogen or alkyl $C_{1-6}$) or phenyl,
G2 represents C=O,
A is a 5- or 6-membered ring optionally substituted by alkyl $C_{1-6}$, halogen, $-COOR_{43}$, $-S(O)_qR_{44}$, $-CONHR_{43}$, $-NHCOR_{45}$, alkoxy $C_{1-6}$ or a group D-G3-, in which
G3 is a single bond, -NH-, -O-, -CO-, $-S(O)_q-$, $-(CH_2)_p-$, $-S(O)_qCH_2-$ or -CO-S-, in which p is 1 or 2, and
D represents a 6-membered ring optionally substituted by $-NO_2$, $-NH_2$, alkyl $C_{1-6}$ or $-SCH_2C_6H_5$, and
$R_3$ represents $-S(O)_qR_{31}$, $-NO_2$, an aryl group, hydrogen, $-CONH_2$, -CN, halogen or $-(CO)_rR_{34}$ (where $R_{34}$ is -OH, $-OR_{35}$ or $-SR_{35}$, $R_{35}$ being cycloalkyl $C_{3-8}$ or alkyl $C_{1-6}$ optionally substituted by alkoxy $C_{1-6}$, halogen, -OH, cycloalkyl $C_{3-8}$ or $-NR_{32}R_{33}$ where $R_{32}$ and $R_{33}$ are hydrogen or alkyl $C_{1-6}$).

As a third specific group, there are provided compounds of formula I in which
$R_1$ is as first defined above,
$R_2$ and $R_5$, which may be the same or different, represent hydrogen, $-R_{21}$, or halogen,
$R_3$ represents $-NO_2$ or $-COOR_{35}$,
G2 represents C=O, -S- or a single bond,
A represents phenyl substituted by a group D-G3-, in which
G3 is as first defined above, and
D is as first defined above.

As a fourth specific group, there are provided compounds of formula I in which
$R_1$ represents hydrogen,
$R_2$ and $R_5$, which may be the same or different, represent hydrogen, $-R_{21}$, or halogen,
$R_3$ represents $-NO_2$ or $-COOR_{35}$,
G2 represents C=O,
A represents phenyl substituted by a group D-G3-, in which
G3 represents -NH- or $-CH_2-$, and
D is as first defined above.

The compounds of formula I and pharmaceutically acceptable derivatives thereof are useful because they possess pharmacological activity in animals. The compounds are suitable as cardiotonics. In particular, the compounds are beneficial inotropic agents, eg positive inotropic agents, and are able to augment contractility of smooth and cardiac muscle.

Thus, according to the invention, we further provide a method of increasing the force of contraction of the heart in an animal, either human or non-human, which method comprises administering to the animal an effective amount of one or more compounds of the invention. We also provide the use of one or more compounds of the invention in the manufacture of a medicament for increasing the force of contraction of the heart in an animal.

The compounds of the invention are also suitable for the treatment of hypotonic circulatory conditions, for the depression of blood sugar, for decreasing the swelling of mucous membranes, as nasal decongestants and for influencing the salt and fluid balance. The compounds may also be beneficial in the treatment of psychiatric illness, migraine and depression.

Activity of the compounds has been observed in the following assay systems:

1. Guinea Pig Atria: Force and Rate - R M Kennedy and E Seifer, Eur. J. Pharmac. (1985), 107, 209-14;

2. Embryonic chick heart cell reaggregate model -W H Barry, J Pober, J D Marsh, S R Frankel and T W Smith, Am J Physiol (1980) 239, H651-H657; L Patmore and R L Whiting, Br J Pharmacol (1985) 86, 817P.

3. Accumulation of $^{45}Ca$ in GH3 cells: G A Shangold, S Kongsamut and R J Miller, Life Sciences (1985), 36,2209-15.

The dosage administered will naturally depend on the compound employed, the mode of administration and the desired effect. However in general satisfactory results are obtained when the compounds are administered at a dosage of from 0.05µg to 3.5g, which may be administered in divided doses of, for example 1µg to 750mg.

The compounds of formula I, and pharmaceutically acceptable derivatives thereof, have the advantage that they are more efficacious or produce less undesirable side effects in certain pharmacological models, or are longer acting than compounds of similar structure to the compounds of formula I.

The compounds of the invention may be administered by a wide variety of routes and may act systemically or locally. Thus the compounds may be administered by oral or nasal inhalation to the lung, to the buccal cavity, oesophageally, rectally, topically to the skin or to other available surfaces of the body by injection, eg intravenously, intramuscularly, intraperitoneally, or by surgical implant.

According to our invention we also provide a pharmaceutical composition comprising preferably less than 80% and more preferably less than 50% by weight of a compound of formula I, or a pharmaceutically acceptable derivative thereof, in admixture with a pharmaceutically acceptable adjuvant, diluent or carrier. Examples of suitable adjuvants, diluents or carriers are:
For tablets, capsules and dragees: microcrystalline cellulose, calcium phosphate, diatomaceous earth, a sugar such as lactose, dextrose or mannitol, talc, stearic acid, starch, sodium bicarbonate and/or gelatin;
for suppositories: natural or hardened oil or waxes; and
for inhalation compositions: coarse lactose.

When the compounds are to be used in aqueous solution it may be necessary to incorporate a chelating or sequestering agent, eg sodium edetate, an antioxidant, eg sodium metabisulphite or buffering agents, eg sodium hydrogen phosphate and sodium phosphate. Aqueous solutions typically contain up to about 10% w/w of the new compound and may be used for intravenous injections.

The invention is illustrated, but in no way limited by the following Examples. All temperatures quoted are in °C.

## Example 1

### 1.1 Methyl 2,5-Dimethyl-4-(2-((4-nitrophenyl)thio) benzoyl)-1H-pyrrole-3-carboxylate

2-((4-Nitrophenyl)thio)benzoic acid (5.5g, 20mmol) and thionyl chloride (5.5ml) in chloroform (50ml) were heated at reflux for 4 hours. The solvent was evaporated and the residue azeotroped with toluene to remove excess thionyl chloride. The acid chloride was dissolved in dichloromethane (50ml) and methyl 2,5-dimethyl-1H-pyrrole-3-carboxylate (3.06g, 20mmol) in dichloromethane (100ml) was added. The mixture was cooled to -70° and, with stirring, aluminium chloride (4g) was added. After stirring at room temperature for 16 hours, the reaction mixture was poured onto ice/water/chloroform with stirring. After 2 hours, the solid was filtered off, washed with water, methanol and dichloromethane and dried in vacuo to give the title compound (5g). A microanalytically pure sample was prepared by crystallisation from dichloromethane/methanol, mp 224-225°.

### 1.2 i) Methyl 2,5-Dimethyl-4-(2-((4-nitrophenyl)methyl) benzoyl)-1H-pyrrole-3-carboxylate

### ii) Methyl 2,5-Dimethyl-4-(2-((2-nitrophenyl)methyl) benzoyl)-1H-pyrrole-3-carboxylate

2-(Phenylmethyl)benzoic acid (5g, 2.35mmol) was added with stirring to nitric acid (75ml, d = 1.42) at room temperature. After 4 hours, the reaction mixture was poured onto ice/water with stirring. The solid was filtered off, washed with water and then dissolved in ether. The ethereal solution was washed with brine, dried ($Na_2SO_4$) and the solvent evaporated to give a mixture of the 2′- and 4′-nitro acids. Chloroform (30ml) and thionyl chloride (5ml) were added and the mixture was heated at reflux for 4 hours. The solvent was evaporated and excess thionyl chloride removed by azeotroping with toluene. Methyl 2,5-dimethyl-1H-pyrrole-3-carboxylate (3.6g, 2.35mmol) in dichloromethane (100ml) was added and the mixture cooled with stirring to -70°. Aluminium chloride (4g) was added and the reaction mixture was allowed to reach room temperature overnight. After heating at reflux for 1 hour, the cooled reaction mixture was poured onto ice/water. The organic layer was separated and washed with water, saturated sodium bicarbonate and brine. After drying ($Na_2SO_4$), the solvent was evaporated. HPLC on silica eluting with ethyl acetate/petroleum ether bp 60-80° followed by recrystallisation three times from toluene gave the 4-nitrophenyl isomer (1.0g), mp 187-188°.

The 2-nitrophenyl isomer was obtained from the first recrystallisation liquors by reverse phase HPLC eluting with methanol/water mixtures. Crystallisation from dichloromethane/cyclohexane gave the 2-nitrophenyl isomer (0.28g), mp 147-148°.

1.3 Methyl 2,5-Dimethyl-4-(2-(2-phenylethyl)benzoyl)-1H-pyrrole-3-carboxylate

Aluminium chloride (4g) was added to a stirred solution at -78° of methyl 2,5-dimethyl-1H-pyrrole -3-carboxylate (2g, 13mmol) and 2-(2-phenylethyl)benzoyl chloride (3.5g, 14mmol) in dichloromethane (170ml). The reaction was allowed to warm to -30°, stirred at this temperature for 3 hours, then poured onto ice. After dilution with chloroform, the organic layer was separated, dried ($MgSO_4$ and evaporated. Chromatography on silica eluting with ethyl acetate-hexane mixtures gave the title compound (2.1g) as a white solid, mp 42-48°.

Compounds 1.4-1.21 were prepared from appropriate starting materials by methods analogous to those used in the preparation of Compounds 1.1 to 1.3.

1.4 Methyl 2,5-Dimethyl-4-(2-(4-nitrophenoxy)benzoyl)-1H-pyrrole-3-carboxylate

mp 141-142.5°.

1.5 Methyl 2,5-Dimethyl-4-(2-(3-nitrobenzoyl)benzoyl)-1H-pyrrole-3-carboxylate

mp 243-244°.

1.6 Methyl 2,5-Dimethyl-4-(2-(((4-methylphenyl) sulphonyl)oxy)benzoyl)-1H-pyrrole-3-carboxylate

mp 111-113°.

1.7 Methyl 4-(2-Chlorobenzoyl)-2-methyl-5-(methylthio)-1H-pyrrole-3-carboxylate

a) Methyl 2-Methyl-5-(methylthio)-1H-pyrrole-3-carboxylate

Sulphuryl chloride (2.0g, 15mmol) was added to a solution of dimethyldisulphide (1.4g, 15mmol) in dichloromethane (30ml). After 30 minutes this solution was added dropwise to a solution of methyl 2-methyl-1H-pyrrole-3-carboxylate (2.78g, 20mmol) in dichloromethane (60ml). After stirring for 16 hours, the solution was washed with water and brine, dried ($MgSO_4$) and the solvent evaporated. Chromatography on silica eluting with ethyl acetate/petroleum ether (60-80°) mixtures gave the sub-title compound (0.85g). $M^+$ 185; nmr delta ($CDCl_3$) 2.32 (s,3H), 2.51 (s,3H), 3.80 (s,3H).

b) Methyl 4-(2-Chlorobenzoyl)-2-methyl-5-(methylthio)-1H-pyrrole-3-carboxylate

Prepared from the product of step a), mp 164-165°.

1.8 Methyl 2,5-Dimethyl-4-(2-pyridinylamino)benzoyl) -1H-pyrrole-3-carboxylate

mp 240° (dec).

1.9 Methyl 4-(2-(Cyclohexylmethyl)benzoyl)-2,5-dimethyl -1H-pyrrole-3-carboxylate

a) 2-(2-(Cyclohexylmethyl)phenyl)-4,5-dihydro-4,4-dimethyloxazole

2-(2-Fluorophenyl)-4,5-dihydro-4,4-dimethyloxazole (5.4g, 28mmol) was added to a stirred solution of cyclohexylmagnesium bromide (28mmol) in tetrahydrofuran (25ml) at room temperature. After 4 hours, saturated ammonium chloride solution was added and the mixture stirred for a further 2 hours. The tetrahydrofuran was removed by evaporation and the aqueous residue was extracted with ethyl acetate. The organic extract was dried ($MgSO_4$) and the solvent was evaporated. Chromatography on silica eluting with ethyl acetate/petroleum ether (60-80°) gave the sub-title compound (1.9g).
$M^+$ 271; nmr delta ($CDCl_3$) 2.84 (d,2H), 4.06 (s,2H).

b) 2-(Cyclohexylmethyl)benzoic acid

2-(2-(Cyclohexylmethyl)phenyl)-4,5-dihydro-4,4-dimethyloxazole (1.4g, 5.16mmol) was heated at reflux in iodomethane (25ml) for 8 hours. The excess iodomethane was evaporated and the residue was heated at reflux for 10 hours in 20% aqueous sodium hydroxide solution. The cooled solution was extracted with dichloromethane, acidified with hydrochloric acid and extracted with ethyl acetate (x3). The combined ethyl acetate extracts were dried ($MgSO_4$) and the solvent evaporated to give the sub-title acid (0.47g).
$M^+$ 218; nmr delta ($CDCl_3$) 2.93 (d,2H).

c) Methyl 4-(2-Cyclohexylmethyl)benzoyl)-2,5-dimethyl-1H-pyrrole-3-carboxylate

Prepared from the product of step b), mp 161-162°.

1.10 Methyl 4-(2-Chlorobenzoyl)-2-methyl-5-phenyl-1H- pyrrole-3-carboxylate

mp 159-160°.

1.11 Methyl 4-(2-Chlorobenzoyl)-2,5-dimethyl-α-oxo-1H-pyrrole-3-acetate

Prepared using methyl chloro-oxoacetate and (2-chlorophenyl) (2,4-dimethyl-1H-pyrrol-3-yl)methanone, mp 209-211°.

1.12 (2-Chlorophenyl) (2.5-Dimethyl-4-(5-methyl-1,3,4-oxadiazol-2-yl)-1H-pyrrol-3-yl)methanone

a) 2-(2,5-Dimethyl-1H-Pyrrol-3-yl)-5-methyl-1,3,4-oxadiazole

Methyl 2,5-dimethyl-1H-pyrrole-3-carboxylate (7.5g, 50mmol) and hydrazine hydrate (10ml) were heated at 120° for 16 hours. The reaction mixture was filtered off and the filtrate evaporated to dryness. The residue was crystallised from ethanol/water and the solid was filtered off. The combined solids were dried in vacuo and then heated at reflux under nitrogen in triethyl orthoacetate (25ml) for 24 hours. On cooling the sub-title oxadizole crystallised out and was filtered off (5.5g).
M/e 177; nmr ($D_6$DMSO) delta 2.15(s,3H), 2.42 (s,3H), 2.47 (s,3H), 6.04 (s,1H) and 11.04(s,1H).

b) (2-Chlorophenyl) (2,5-dimethyl-4-(5-methyl-1,3,4-oxadiazol-2-yl)-1H-pyrrol-3-yl)methanone

Prepared from the product of step a), mp 207-209°.

1.13 Methyl 4-(2-(2-(Dimethylamino)ethoxy)benzoyl)-2,5-dimethyl-1H-pyrrole-3-carboxylate maleate salt

mp 149-150°.

1.14 Methyl 2,5-Dimethyl-4-(2-((2-((phenylmethyl)thio) benzoyl)thio)benzoyl)-1H-pyrrole-3-carboxylate

Obtained as a by-product from the reaction of 2-((phenylmethyl)thio)benzoyl chloride with methyl 2,5-dimethyl-1H-pyrrole-3-carboxylate, mp 115°.

1.15 Methyl 4-(2-(Methoxycarbonyl)benzoyl)-2,5-dimethyl-1H-pyrrole-3-carboxylate

mp 131-132°.

1.16 Methyl 3-(2-Chlorobenzoyl)-4-(methoxycarbonyl)-5-methyl-1H-pyrrole-2-propanoate

a) Methyl 4-(methoxycarbonyl)-5-methyl-1H-pyrrole-2-propanoate

Methyl 2-methyl-1H-pyrrole-3carboxylate (2g, 14.4mmol) and methyl 2-propenoate (1.6g, 18.7mmol) in benzene (70ml) was stirred for 7 days. Boron trifluoride etherate (1ml) was added and the reaction mixture was stirred for a further 2 days. The reaction mixture was poured on to water and chloroform. The organic layer was separated, dried ($MgSO_4$) and the solvent exaporated. Chromatography on silica eluting with hexane/tetrahydrofuran gave the sub-title ester (0.25g).
$M^+$ 225; nmr ($CDCl_3$) delta 2.48(s,3H), 3.70(s,3H), 3.77(s,3H).

b) Methyl 3-(2-chlorobenzoyl)-4-(methoxycarbonyl)-5-methyl-1H-pyrrole-2-propanoate

Prepared from the product of step a), mp 108-109°.

1.17 Methyl 4-(2-Chlorobenzoyl)-5-methyl-2-(2-nitroethyl) -1H-pyrrole-3-carboxylate

a) Methyl 5-methyl-2-(2-nitroethyl)-1H-pyrrole-3-carboxylate

Nitroethylene (excess) and methyl 5-methyl-1H-pyrrole-3-carboxylate (2g, 14.4mmol) in benzene (30ml) was left for 16 hours. The solvent was exaporated and the residue chromatographed on silica eluting with ethyl acetate/dichloromethane to give the sub-title compound (1.1g).
$m^{+}$ 212; nmr ($CDCl_3$) delta 2.27(s,3H) and 3.79(s,3H).

b) Methyl 4-(2-chlorobenzoyl)-5-methyl-2-(2-nitroethyl)-1H-pyrrole-3-carboxylate

Prepared using the product of step a), mp 171-172°.

1.18 Methyl 2,5-Dimethyl-4-(3-pyridinylcarbonyl)-1H-pyrrole-3-carboxylate

mp 184-186°.

1.19 Methyl 2,5-Dimethyl-4-(4-pyridinylcarbonyl)-1H-pyrrole-3-carboxylate

mp 157-159°.

1.20 5-(2-Chlorobenzoyl)-3,4-dihydro-6-methyl-7H-pyrrolo(2,3-d)pyrimidin-4-one

mp>260°

1.21 (E)Methyl 4-(3-methoxy-1-oxo-4-phenyl-2-butenyl)-2,5-dimethyl-1H-pyrrole-3-carboxylate

Prepared by the method of Compound 1.1 using 3-methoxy-4-phenyl-2-butenoyl chloride which was obtained in turn from methyl 4-phenyl-2-butynoate by reaction with aqueous sodium hydroxide in methanol followed by thionyl chloride, mp 140-141°

1.22 (2,5-Dimethyl-1H-pyrrol-3-yl) (2-(phenylmethyl) phenyl)methanone

2,5-Dimethyl-1H-pyrrole (10g,0.105mol) in ether (50ml) was added dropwise to a stirred solution of ethyl magnesium bromide (16.6g, 42mls of 3M in ether, 0.125mol) in benzene (85ml). After 20 minutes, 2-(phenylmethyl)benzoyl chloride (29g, 0.125 mol) in ether/benzene was added over 35 minutes. After 3 hours, saturated ammonium chloride (12.5g in 50ml water) was added followed 10 minutes later by water. The product was extracted with ethyl acetate (x3). The organic extract was washed with saturated sodium

bicarbonate, water and brine, dried (MgS)₄) and the solvent was evaporated. Purification by HPLC eluting with tetrahydrofuran/petroleum ether (60-80°) followed by crystallisation from hexane/2-propanol gave the title compound (16g), mp 110-112°.

Example 2

2.1 Methyl 2,5-Dimethyl-4-(2-(phenylthio)benzoyl)-1H-pyrrole-3-carboxylate

a) Methyl 4-(2-Iodobenzoyl)-2,5-dimethyl-1H-pyrrole -3-carboxylate

Aluminium chloride (12g) was added to a stirred solution at -70° of methyl 2,5-dimethyl-1H-pyrrole -3-carboxylate (9.18g, 60mmol) and 2-iodobenzoyl chloride (16g, 60mmol) in dichloromethane (150ml). The reaction mixture was allowed to reach room temperature, stirred overnight and then poured onto ice. After dilution with chloroform, the organic layer was separated and washed with water, saturated sodium bicarbonate (twice) and brine, dried ($Na_2SO_4$) and the solvent evaporated. Crystallisation from toluene gave the sub-title compound (6.6g), mp 125-126°.

b) Methyl 2,5-Dimethyl-4-(2-(phenylthio)benzoyl)-1H-pyrrole-3-carboxylate

The product of step a) (0.85g, 2.2mmol), sodium hydroxide (0.09g, 2.2mmol), benzenethiol (0.24g, 2.2mmol) and copper (I) oxide (0.15g, 1.1mmol) were heated at 100° in dimethylformamide (5ml) under $N_2$ for 16 hours. The cooled reaction mixture was poured onto dilute hydrochloric acid (50ml) and the product extracted with ethyl acetate (2 x 50ml). The organic layers were washed with dilute hydrochloric acid, water and saturated brine, dried ($Na_2SO_4$) and the solvent was evaporated. Chromatography on silica eluting with chloroform/ethyl acetate mixtures followed by crystallisation from toluene/chloroform gave the title compound (0.23g), mp 177-178°.

2.2 Methyl 2,5-Dimethyl-4-(2-(phenylamino)benzoyl)-1H-pyrrole-3-carboxylate

Copper bronze (2.5g) was added to methyl 4-(2-iodobenzoyl)-2,5-dimethyl-1H-pyrrole-3-carboxylate (2.5g, 6.5mmol) and aniline (7.5ml). The reaction mixture was heated at 110° under $N_2$ for 5 hours, cooled, diluted with chloroform and filtered. The solvent was evaporated and the residue chromatographed on silica eluting with ethyl acetate/dichloromethane mixtures. Rechromatography on silica eluting with ether/hexane followed by partial removal of solvent and filtration gave the title compound (0.8g), mp 140-142°, as yellow crystals.

Compounds 2.3-2.4 were prepared from appropriate starting materials by methods analogous to those used in the preparation of Compounds 2.1 and 2.2.

2.3 Methyl 2,5-Dimethyl-4-(2-(2-pyridinylamino)benzoyl) -1H-pyrrole-3-carboxylate

Obtained as the maleate propanolate 1:1:1, mp 113-115°.

2.4 Methyl 5-Ethyl-2-methyl-4-(2-(phenylamino)benzoyl)-1H-pyrrole-3-carboxylate

a) Methyl 5-ethyl-4-(2-iodobenzoyl)-2-methyl-1H-pyrrole-3-carboxylate

Obtained by method of Example 2.1, mp 149-150°.

b) Methyl 5-Ethyl-2-methyl-4-(2-(phenylamino)benzoyl)-1H-pyrrole-3-carboxylate

Obtained from product of step a), mp 158-159° (dec).

2.5 Methyl 2,5-dimethyl-4-(2-(cyclohexylamino)benzoyl)-1H-pyrrole-3-carboxylate

Methyl 4-(2-iodobenzoyl)-2,5-dimethyl-1H-pyrrole-3-carboxylate (1.1g, 2.9mmol) was dissolved in cyclohexylamine (10ml) at 100° under $N_2$. Anhydrous nickel (II) bromide (0.1g) was added and the mixture heated at reflux for 3 hours. The excess amine was evaporated and the residue chromatographed on silica eluting with ethyl acetate/petroleum ether (60-80°) (x2). Crystallisation from dichloromethane/cyclohexane gave the title compound as yellow crystals, mp 180-182°.

2.6 Methyl 2,5-dimethyl-4-(2-((phenylmethyl)amino)benzoyl) -1H-pyrrole-3-carboxylate

Prepared by the method of Example 2.5
$M^+$ 451, nmr delta 1.94, 2.57, 3.50, 4.46

2.7 Methyl 2,5-dimethyl-4-(2-((phenylmethyl)amino)phenyl) ((phenylmethyl)imino)methyl)-1H-pyrrole-3-carboxylate

Obtained as by-product in preparation of Compound 2.6, mp 151-153°.

2.8 Methyl 4-((2-(cyclohexylamino)phenyl)(cyclohexylimino)methyl)-2,5-dimethyl-1H-pyrrole-3-carboxylate

Obtained as by-product in preparation of Compound 2.5, mp 174-176°.

2.9 (E)-Methyl 2,5-dimethyl-4-(2-(2-phenylethenyl) benzoyl)-1H-pyrrole-3-carboxylate

Methyl 4-(2-iodobenzoyl)-2,5-dimethyl-1H-pyrrole-3-carboxylate (0.7g, 1.82mmol), styrene (5ml), triethylamine (0.51g, 5mmol), triphenylphosphine (0.1g) and palladium (II) acetate (catalytic) in dimethylformamide (1ml) were heated in a sealed tube at 80° for 16 hours. The reaction mixture was poured onto dilute hydrochloric acid and ethyl acetate. The organic layer was separated, dried ($MgSO_4$ and the solvent was evaporated. Chromatography on silica eluting with ethyl acetate/petroleum ether (60-80°) mixtures followed by trituration with ether/hexane gave the title compound (0.17g) mp 76-80°.

2.10 E-3-(2-((4-(Methoxycarbonyl)-2,5-dimethyl-1H-pyrrol-3-yl)carbonyl)phenyl)-2-propenoic acid

Prepared by the method of Example 2.9, mp 225° (dec)

2.11 Methyl 5-(2-(methoxycarbonyl)ethenyl)-2-methyl-4-(2- (phenylmethyl)benzoyl)-1H-pyrrole-3-carboxylate

Prepared by the method of Example 2.9 using the Compound of Example 3.21, mp 212-213°.

2.12 Methyl 2-methyl-5-phenyl-4-(2-(phenylmethyl)benzoyl) -1H-pyrrole-3-carboxylate

Methyl 5-iodo-2-methyl-4-(2-(phenylmethyl)benzoyl) -1H-pyrrole-3-carboxylate (1.15g, 2.2mmol), tetraphenyltin (2.15g,5mmol) and chloro(phenylmethyl)bis(triphenyl phosphine) palladium II (50ng) were heated for 2 days at 60° in hexamethylphosphoric triamide (20ml). The cooled reaction mixture was poured onto dilute hydrochloric acid and ethyl acetate. The organic layer was separated, washed with water, dried ($MgSO_4$) and the solvent was evaporated. Chromatography on silica eluting with ethyl acetate/petroleum ether (60-80°) mixtures followed by HPLC on silica eluting with ethyl acetate/dichloromethane gave the title compound (0.26g), mp 149°.

2.13 Methyl 2-methyl-5-(phenylmethyl)-4-(2-(phenylmethyl) benzoyl)-1H-pyrrole-3-carboxylate

Prepared by the method of Example 2.12, mp 181°.

Example 3

3.1 Methyl 2-Bromo-4-(2-chlorobenzoyl)-5-methyl-1H-pyrrole-3-carboxylate

a) Methyl 4-(2-Chlorobenzoyl)-5-methyl-1H-pyrrole-3-carboxylate

Prepared using process a), mp 153-154°.

b) Methyl 2-Bromo-4-(2-chlorobenzoyl)-5-methyl-1H-pyrrole-3-carboxylate

Pyridinium bromide perbromide (0.62g, 1.65mmol) was added to a stirred solution of methyl 4-(2-chlorobenzoyl)-5-methyl-1H-pyrrole-3-carboxylate (0.415g, 1.5mmol) in dichloromethane (10ml). After 3 hours, the reaction mixture was washed with water and brine, dried ($MgSO_4$) and the solvent evaporated. Chromatography on silica eluting with ethyl acetate/petroleum ether (60-80°) followed by crystallisation from cyclohexane/ether gave the title compound (0.21g), mp 122-123°.

3.2 (4-Bromo-2,5-dimethyl-1H-pyrrol-3-yl) (2-(phenylmethyl) phenyl)methanone

Prepared by an analogous method to Compound 3.1, mp 151° (dec).

3.3 Methyl 2-Bromo-5-methyl-4-(2-(phenylmethyl)benzoyl)-1H-pyrrole-3-carboxylate

Bromine (3.3ml of 1M in carbon tetrachloride, 3.3mmol) was added to a solution at -78° of methyl 5-methyl-4-(2-(phenylmethyl)benzoyl)-1H-pyrrole-3-carboxylate(1.11g, 3.3mmol) and triethylamine (0.33g, 3.3mmol) in dichloromethane (20ml). After 1 hour the reaction was warmed to room temperature and water was added. The organic layer was dried ($MgSO_4$) and the solvent was evaporated. Chromatography on silica eluting with ethyl acetate/petroleum ether (60-80°) followed by crystallisation from toluene gave the title bromide (0.65g), mp 140-141°.

3.4 Methyl 5-Bromo-2-methyl-4-(2-(phenylmethyl)benzoyl)-1H-pyrrole-3-carboxylate

Prepared by an analogous method to Compound 3.3, mp 196-197°.

3.5 Methyl 4-(2-Chlorobenzoyl)-2-cyano-5-methyl-1H-pyrrole-3-carboxylate

Chlorosulphonyl isocyanate (0.74g, 5.2mmol) was added to a stirred solution at -78° of methyl 4-(2-chlorobenzoyl)-5-methyl-1H-pyrrole-3-carboxylate (1.11g, 4mmol) in dichloromethane (10ml). The reaction mixture was allowed to reach room temperature and then dimethylformamide (1.2ml) was added. After stirring for 16 hours, ice and dichloromethane were added. The organic layer was separated, dried ($MgSO_4$) and the solvent evaporated. Chromatography on silica eluting with ethyl acetate/dichloromethane followed by crystallisation from toluene gave the title compound (0.3g), mp 143-144°.
Compounds 3.6-3.8 were prepared by methods analogous to that used for Compound 3.5.

3.6 2,5-Dimethyl-4-(2-(phenylmethyl)benzoyl)-1H-pyrrole-3-carbonitrile

mp 190-191°.

3.7 Methyl 2-Cyano-5-methyl-4-(2-(phenylmethyl)benzoyl)-1H-pyrrole-3-carboxylate

mp 176°.

3.8 Methyl 5-Cyano-2-methyl-4-(2-(phenylmethyl)benzoyl)-1H-pyrrole-3-carboxylate

mp 197° (dec)

3.9 2,5-Dimethyl-4-(2-(phenylmethyl)benzoyl)-1H-pyrrole-3-carboxamide

Chlorosulphonyl isocyanate (1.52g, 10.8mmol) was added to a solution at -40° of (2,5-dimethyl-1H-pyrrol-3-yl) (2-(phenylmethyl)phenyl) methanone (3g, 10.4mmol) in dichloromethane (50ml). After 1 hour at room temperature, sodium sulphate solution was added. The organic layer was separated, washed with water and brine, dried ($MgSO_4$) and the solvent evaporated. Chromatography on silica eluting with methanol/dichloromethane followed by crystallisation from acetone and recrystallisation from methanol/acetonitrile gave the title amide (0.11g), mp 235-237°.

3.10 Methyl 4-(2-Chlorobenzoyl)-2-formyl-5-methyl-1H-pyrrole-3-carboxylate

Phosphorous oxychloride (0.85g, 5.5mmol) was added with stirring to dimethylformamide (0.4g, 5.5mmol) at 0°. After 10 minutes, dichloromethane (5ml) was added followed by a solution of methyl 4-(2-chlorobenzoyl)-5-methyl-1H-pyrrole-3-carboxylate (1.39g, 5mmol) in dichloromethane (10ml). After 3 days at room temperature, sodium acetate (2g) in water (5ml) was added. After 3 hours, the reaction mixture was washed with water and brine, dried ($MgSO_4$) and the solvent evaporated. Chromatography on silica eluting with ethyl acetate/petroleum either (60-80°) gave the title compound (0.75g), mp 137-138° (toluene).
Compounds 3.11-3.13 were prepared by methods analogous to that used for Compound 3.10.

3.11 Methyl 5-Formyl-2-methyl-4-(2-(phenylmethyl)benzoyl)-1H-pyrrole-3-carboxylate

mp 186°.

3.12 Methyl 2-Formyl-5-methyl-4-(2-(phenylmethyl)benzoyl)-1H-pyrrole-3-carboxylate

mp 138-139°.

3.13 2,5-Dimethyl-4-(2-phenylmethyl)benzoyl)-1H-pyrrole-3-carboxaldehyde

mp 167-168°.

3.14 Methyl 4-(2-Chlorobenzoyl)-5-methyl-2-nitro-1H-pyrrole-3-carboxylate

A solution of nitric acid (0.5ml of 98%) in acetic anhydride (2ml) was added at -25° to a solution of methyl 4-(2-chlorobenzoyl)-5-methyl-1H-pyrrole-3- carboxylate (0.56g, 2mmol) in acetic anhydride (5ml). After 30 minutes at -25°, the reaction mixture was stirred at 0° for 30 minutes. The reaction was quenched with ice and ethyl acetate was added. The organic layer was separated, dried ($MgSO_4$) and the solvent was evaporated. Chromatography on silica eluting with ethyl acetate/petroleum ether (60-80°) gave the title compound (0.12g), mp 189°.

3.15 (2,5-Dimethyl-4-nitro-1H-pyrrol-3-yl) (2-(phenylmethyl)phenyl)methanone

A solution of (2,5-dimethyl-1H-pyrrol-3-yl) (2-(phenylmethyl)phenyl)methanone (4.5g, 15.6mmol) in acetonitrile (80ml) was added dropwise to a solution at -40° of nitronium tetrafluoroborate (2.25g, 16.9mmol) in acetonitrile (100ml). The reaction mixture was allowed to reach room temperature slowly and then left for a further 1.5 hours. Water and ethyl acetate were added. The organic layer was separated, washed with water and brine, dried ($MgSO_4$) and the solvent was evaporated. Purification by HPLC on silica eluting with hexane/tetrahydrofuran followed by chromatography on silica eluting with ether/hexane and the crystallisation from 2-propanol/hexane gave the title compound (0.24g), mp 151-152°.

3.16 Methyl 4-(2-Chlorobenzoyl)-5-methyl-2-((4-nitrophenyl) azo)-1H-pyrrole-3-carboxylate

4-Nitrobenzenediazonium tetrafluoroborate (0.47g, 2mmol) was added to a stirred solution of methyl 4-(2-chlorobenzoyl)-5-methyl-1H-pyrrole-3-carboxylate (0.556g, 2mmol) in acetonitrile (15ml). A further portion of diazonium compound (0.3g) was added after 4 hours and the mixture left stirring for 16 hours. The solvent was evaporated and the residue purified by chromatography on silica eluting with ethyl acetate/petroleum ether (60-80°) mixtures to give the title compound (0.23g), mp 230°.

3.17 (2-Chlorophenyl)(2,5-Dimethyl-4-(methylthio)-1H-pyrrol-3-yl)methanone

Prepared by the method of Example 1.7a, mp 216-217°.

3.18 Methyl 2-methyl-5-((4-nitrophenyl)azo)-4-(2-(phenylmethyl)benzoyl)-1H-pyrrole-3-carboxylate

Prepared by the method of Compound 3.16.

3.19 Methyl 5-methyl-2-((4-nitrophenyl)azo)-4-(2-(phenylmethyl)benzoyl)-1H-pyrrole-3-carboxylate

Prepared by the method of Compound 3.16.
$M+I^+$ 483; nmr ($CDCl_3$) delta 4.27(s,2H), 3.36(s,3H) and 2.36(s,3H).

3.20 (4-((Dimethylamino)methyl)-2,5-dimethyl-1H-pyrrol-3-yl) ((2-phenylmethyl)phenyl)methanone

A solution of (2,5-dimethyl-1H-pyrrol-3yl) (2- phenylmethyl)phenyl)methanone (2g, 7mmol), dimethylamine hydrochloride (0.56g, 7mmol) and paraformaldehyde (0.28g) in ethanol (40ml) was heated at reflux for 4 days. The solvent was evaporated and the residue was chromatographed on silica eluting with dichloromethane/methanol mixtures to give the title compound (0.2g), mp 95-100°.

3.21 Methyl 5-iodo-2-methyl-4-(2-(phenylmethyl)benzoyl)-1H-pyrrole-3-carboxylate

Iodine monochloride (1g, 6.2mmol) was added to a stirred solution of methyl 2-methyl-4-(2-(phenylmethyl) benzoyl)-1H-pyrrole-3-carboxylate (1.2g, 3.6mmol) and triethylamine (1.05ml, 3.6mmol) in dichloromethane (40ml). After stirring for 16 hours, further iodine monochloride (0.6g) was added. After 1 hour, the reaction mixture was poured onto water and ethyl acetate. The organic layer was separated, washed with water, dried ($MgSO_4$) and the solvent was evaporated. Chromatography on silica eluting with ethyl acetate/dichloromethane gave the title compound (1.33g). $M^+$ 359; nmr ($CDCl_3$) delta 4.38(S,2H), 3.29(S,3H) and 2.49(S.3H).

Example 4

4.1 Methyl 2-Methyl-4-(2-(phenylmethyl)benzoyl)-1H-pyrrole-3-carboxylate

a) 1-(2-(Phenylmethyl)phenyl)ethanone

Tetramethyltin (20ml, 0.14mol) and benzyl(chloro)bis(triphenylphosphine)palladium(II) (catalytic) were added to a stirred solution of 2-(phenylmethyl)benzoyl chloride (0.14mol) in hexamethylphosphoric triamide (30ml). After heating at 60° for 10 minutes, the reaction mixture was cooled, diluted with water and extracted with ether. The organic extract was washed with water, dried ($Na_2SO_4$) and the solvent evaporated to give the sub-title ketone (33.7g).
$M^+$ 210; nmr ($CDCl_3$) delta 2.45(s,3H) and 4.28(s.2H).

b) 2-Bromo-1-(2-(phenylmethyl)phenyl)ethanone

Bromine (7.6g, 0.048mol) was added dropwise to a solution of 1-(2-(phenylmethyl)phenyl)ethanone (10g, 0.048mol) in acetic acid (800ml). After 1 hour, the solvent was evaporated and the residue dissolved in sodium bicarbonate solution and ether. The organic extract was dried ($MgSO_4$) and the solvent exaporated to give the sub-title bromoketone (12.2g).
$M^+$ 288/90; nmr ($CDCl_3$) 4.23(s,2H) and 4.24(s,2H).

c) Methyl 4-oxo-4-(2-(phenylmethyl)phenyl)-2-butenoate

Triphenylphosphine (9g, 34.6mmol) and 2-bromo-1-(2-(phenylmethyl)phenyl)ethanone (10g, 34.6 mmol) in ether (700ml) were stirred at room temperature under nitrogen for 3 days. The reaction mixture was cooled to -78° and butyl lithium (40ml of IM, 40mmol) was added. Methyl oxoacetate (6ml, excess) was added and the mixture allowed to reach room temperature. The solvent was exaporated and the residue extracted with petroleum ether (60-80°). The petrol extracts were evaporated to dryness and the residue chromatographed on silica eluting with ether/hexane to give the sub-title ester (3.1g)
$M^+$ 280; nmr ($CDCl_3$) delta 3.43(s,3H), 4.23(s,2H), 6.50(d,1H) and 7.45(d,1H)

d) Methyl 2-methyl-4-(2-(phenylmethyl) benzoyl)-1H-pyrrole-3-carboxylate

A solution of 1-((1-isocyanoethyl)sulphonyl)-4-methylbenzene (4.4g, 0.021mol) and methyl 4-oxo-4-(2-(phenylmethyl)phenyl)-2-butenoate (5.9g, 0.021mol) in ether/dimethylsulphoxide (100ml, 2:1) was added dropwise to a stirred suspension of sodium hydride (1g of 80%, 0.033mol) in ether (100ml). After stirring for 1 hour, the reaction mixture was poured onto 2% sodium chloride solution (1000ml). The aqueous layer was extracted with ether (4 times) and the ethereal extracts were dried ($MgSO_4$) and the solvent was evaporated. Chromatography on silica eluting with ethyl acetate/dichloromethane followed by crystallisation from toluene gave the title compound (3.2g), mp 98-99°.

4.2 Methyl 4-(2-(phenylmethyl)benzoyl)-1H-pyrrole-3-carboxylate

Prepared by a method analogous to that used in the preparation of Compound 4.1, mp 159-160°.

Example 5

5.1 Methyl 3-(Methoxycarbonyl)-5-methyl-4-(2-(phenylmethyl)benzoyl)-1H-pyrrole-2-acetate

a) Dimethyl 2-Chloro-3-(((1,1-dimethylethoxy)carbonyl) hydrazono)pentanedioate

1,1-Dimethylethyl hydrazinecarboxylate (6.5g, 49mmol) and dimethyl 2-chloro-3-oxopentanedioate (10.3g, 49mmol) in ether (150ml) was stirred for 16 hours. The title compound (9.7g) was filtered off.
$(M+I)^+$ 323/5; nmr delta ($CDCl_3$) 5.22 (s,1H), 3.785 (s,3H), 3.775 (s,3H), 1.52 (s,9H).

b) 2-(Phenylmethyl)benzoyl chloride

N,N-Dimethylformamide (2 drops) was added to a stirred mixture of 2-(phenylmethyl)benzoic acid (11.5g, 54mmol) and thionyl chloride (5ml) in benzene (100ml). After 2 hours at reflux the solvent was evaporated and the residue azeotroped twice with toluene to give the title acid chloride.

c) 1-(2-(Phenylmethyl)phenyl)-1,3-butanedione

Methyl lithium (49ml of 0.84M, 41mmol) was added to a stirred solution at -78° of trimethyl(1-methyl ethenyloxy)silane (6g, 46mmol) in tetrahydrofuran (60ml). After 1 hour, 2-(phenylmethyl)benzoyl chloride (7.3g, 31.6mmol) in tetrahydrofuran (20ml) was added slowly. The reaction mixture was allowed to warm up to -30° and then quenched into dilute hydrochloric acid and ethyl acetate. The organic layer was separated, washed with brine, dried ($MgSO_4$) and the solvent evaporated. Purification by HPLC eluting with tetrahydrofuran/petroleum ether (60-80°) mixtures gave the sub-title dione (4.4g).
$M^+$ 252; nmr ($CDCl_3$) delta 5.75 (s,1H), 4.23 (s,2H), 2.10 (s,3H).

d) Methyl 1-(((1,1-Dimethylethoxy)carbonyl)amino)-3-(methoxycarbonyl)-5-methyl-4-(2-(phenylmethyl)-benzoyl)-1H-pyrrole-2-acetate

Saturated sodium bicarbonate (50ml) was added to a mixture of dimethyl 2-chloro-3-(((1,1-dimethylethoxy) carbonyl)hydrazono)pentanedioate (2.2g, 6.6mmol) and ether (50ml). After stirring for 20 minutes, the organic layer was separated, dried ($MgSO_4$) and the solvent removed to give dimethyl 3-(((1,1-dimethylethoxy)carbonyl)azo)-2-pentenedioate (1.9g). This compound was added to 1-(2-(phenylmethyl)-phenyl)-1,3-butanedione (1.5g, 6mmol) in tert-butanol (70ml) and the mixture heated at reflux for 15 hours. The solvent was evaporated and the residue chromatographed on silica eluting with tetrahydrofuran/petroleum ether (60-80°) mixtures to give the sub-title compound (1.35g).
$(M+I)^+$ 520; nmr ($CDCl_3$) delta 3.74 (s,3H), 3.18 (s,3H), 2.10 (s,3H), 1.50 (s,9H).

e) Methyl 3-(Methoxycarbonyl)-5-methyl-4-(2-(phenylmethyl)benzoyl)-1H-pyrrole-2-acetate

Gaseous hydrogen chloride was bubbled through a refluxing solution of methyl 1-(((1,1-dimethylethoxy) carbonyl)amino)-3-(methoxycarbonyl)-5-methyl-4-(2-(phenyl methyl)benzoyl)-1H-pyrrole-2-acetate (1.3g, 2.5mmol) in methanol (60ml) for 8 minutes. The methanol was partially removed and the residue dissolved in ethyl acetate. The organic layer was washed with dilute sodium bicarbonate, water and brine, dried ($MgSO_4$) and the solvent was evaporated to give the 1-amino intermediate as an oil.
This oil was dissolved in ethanol (80ml) and then 2M hydrochloric acid(10ml) was added. The solution was cooled to 12° and then sodium nitrite (0.8g) in water (10ml) was added over about 2 minutes. After 10 minutes, ethyl acetate and water were added. The organic layer was washed with water (3 times) and brine, dried ($MgSO_4$) and the solvent was evaporated. Chromatography on silica eluting with ethyl acetate/dichloromethane mixtures followed by crystallisation from methanol gave the title compound (0.46g), mp 152-153°.

5.2 Methyl 5-Ethyl-2-methyl-4-(2-(phenylmethyl)benzoyl)- 1H-pyrrole-3-carboxylate

a) Methyl 2-Chloro-3-(((1,1-dimethylethoxy)carbonyl) hydrazono)butanoate

1,1-Dimethylethyl hydrazinecarboxylate (7g, 53mmol) was added portionwise to a solution at room temperature of methyl 2-chloro-3-oxobutanoate (7.5g, 50mmol) in ether (200ml). After stirring for 18 hours, the solid was filtered off to give the sub-title hydrazone (13.7g), mp 143-144° (dec).

b) 4-(2-(Phenylmethyl)benzoyl)-3,5-heptanedione

Sodium hydride (4.5g, 0.094moles) was added to a solution of 3,5-heptanedione (6g, 0.047moles) in dimethoxyethane (100ml). After 1 hour, 2-(phenylmethyl) benzoyl chloride (10.8g, 0.047moles) in dimethoxyethane (30ml) was added. After 16 hours, water and hexane were added. The separated aqueous extract was acidified with 2N hydrochloric acid and extracted with ether. The ethereal extract was washed with water and brine, dried ($MgSO_4$) and the solvent evaporated. HPLC on silica eluting with tetrahydrofuran/petroleum ether (60-80°) mixtures gave the trione (8.1g).
$M^+$ 322; nmr delta ($CDCl_3$) 0.91 (t,6H), 2.01 (q,4H), 4.40 (s,2H).

c) 1-(2-(phenylmethyl)phenyl)-1,3-pentanedione

The product of step b) (7.5g, 23mmol) was heated at 80° in acetic acid (100ml) containing concentrated sulphuric acid (3ml) for 2.5 hours. After cooling, water and ethyl acetate were added. The organic layer was separated, washed three times with water, dried ($MgSO_4$) and the solvent was evaporated. HPLC on silica eluting with tetrahydrofuran/ petroleum ether (60-80°) mixtures gave the sub-title dione (2.2g).
$M^+$ 266; nmr delta ($CDCl_3$) 1.3(t,3H), 2.37 (q,2H), 4.23 (s,2H).

d) Methyl 1-(((1,1-Dimethylethoxy)carbonyl)amino)-5-ethyl-2-methyl-4-(2-(phenylmethyl)benzoyl)-1H-pyrrole-3-carboxylate

Saturated sodium bicarbonate (100ml) was added to a vigorously stirred mixture of methyl 2-chloro-3-(((1,1-dimethylethoxy)carbonyl)hydrazono)butanoate (3.6g, 15.8mmol) and ether (100ml) containing tetrahydrofuran (5ml). After 2 hours, the organic layer was separated, washed with water, dried ($MgSO_4$) and the solvent was evaporated. 1-(2-(Phenylmethyl)phenyl)-1,3-pentanedione (2.2g, 8.3mmol) in t-butanol (150ml) was added and the mixture heated at reflux for 17 hours. The solvent was evaporated and crystallised from ethyl acetate/hexane to give the sub-title compound (3.0g), mp 162-164° (dec).

e) Methyl 1-Amino-5-ethyl-2-methyl-4-(2-(phenylmethyl) benzoyl)-1H-pyrrole-3-carboxylate

HC1 gas was bubbled through a solution of the product of step d) (3g, 6.3mmol) in methanol at reflux for 7 minutes. After cooling, the methanol was partially evaporated and then ethyl acetate and water were added. The organic layer was washed with water, dried ($MgSO_4$) and the solvent evaporated. Crystallisation from methanol gave the sub-title compound (2.1g), mp 143-144°.

f) Methyl 5-Ethyl-2-methyl-4-(2-(phenylmethyl)benzoyl)-1H-pyrrole-3-carboxylate

The produce of step e) (2g, 5.3mmol) was dissolved in hot ethanol (250ml) and cooled to 10°. Concentrated hydrochloric acid (3ml) and water (10ml) were added followed by a solution of sodium nitrite (1.8g, 26mmol) in water (10ml). After 20 minutes, the solvent was evaporated and the residue dissolved in ethyl acetate and water. The organic layer was washed with water (x2) and brine, dried ($MgSO_4$) and the solvent was evaporated. Crystallisation from ether/hexane and recrystallisation from 2-propanol gave the title compound (0.25g), mp 152-153°.

Compounds 5.3-5.12 were prepared from appropriate starting materials using methods analogous to those used in the preparation of Compounds 5.1 and 5.2.

5.3 Methyl 2,5-Dimethyl-4-((1-(phenylmethyl)-1H-pyrrol-2-yl)carbonyl)-1H-pyrrole-3-carboxylate

a) Methyl 1-(((1,1-Dimethylethoxy)carbonyl)amino)-2,5-dimethyl-4-((1-(phenylmethyl)-1H-pyrrol-2-yl)-carbonyl)-1H-pyrrole-3-carboxylate

Prepared by the method of Compound 5.1d), mp 178-180°.

b) Methyl 2,5-Dimethyl-4-((1-(phenylmethyl)-1H-pyrrol-2-yl)carbonyl)-1H-pyrrole-3-carboxylate

Prepared from the product of step a) by the method of Compound 5.1e) and 5.1f), mp 182-183°.

5.4 Ethyl 4-(2-Chlorobenzoyl)-1-((methoxycarbonyl)amino)-2,5-dimethyl-1H-pyrrole-3-carboxylate

mp 138-140°.

5.5 Methyl 4-(2-Chlorobenzoyl)-1-(((1,1-dimethylethoxy) carbonyl)amino)-2,5-dimethyl-1H-pyrrole-3-carboxylate

mp 163-164°.

5.6 Methyl 1-Amino-4-(2-chlorobenzoyl)-2,5-dimethyl-1H-pyrrole-3-carboxylate

Prepared by the method of Compound 5.1e) and purifying the intermediate 1-amino compound, mp 167-168°c.

5.7 Methyl 2,5-Dimethyl-4-((2-methyl-1-piperidinyl) carbonyl)-1H-pyrrole-3-carboxylate

a) Methyl 1-(((1,1-dimethylethoxy)carbonyl)amino)-2,5-dimethyl-4-((2-methyl-1-piperidinyl)carbonyl)-1H-pyrrole-3-carboxylate

Prepared by the method of Compound 5.1d)
$M^+$ 393; nmr ($CDCL_3$) delta 2.2(s,3H), 1.88(s,3H) and 1.5(s,9H).

b) Methyl 1-amino-2,5-dimethyl-4-((2-methyl-1-piperidinyl)carbonyl)-1H-pyrrole-3-carboxylate

Prepared by the method of Compound 5.6.
$M^+$ 293; nmr ($CDCl_3$) delta 3.59(2s, total 3H), 2.50(2s, total 3H) and 2.40(2s, total 3H).

c) Methyl 2,5-dimethyl-4-((2-methyl-1-piperidinyl)carbonyl)-1H-pyrrole-3-carboxylate

Prepared by the method of Compound 5.1f), mp 170-172°.

5.8 Methyl 2,5-Dimethyl-4-((1-(phenylmethyl)-1H-imidazol-2-yl)carbonyl)1H-pyrrole-3-carboxylate

a) Methyl 1-(((1,1-dimethylethoxy)carbonyl)amino) -2,5-dimethyl-4-((1-(phenylmethyl)-1H-imidazol-2-yl) carbonyl)-1H-pyrrole-3-carboxylate

Prepared by the method of Compound 5.1d).
$M^+$ 452; nmr ($CDCl_3$) delta 1.49(s,9H), 2.16(s,3H), 2.27(s,3H) and 3.00(s,3H)

b) Methyl 2,5-dimethyl-4-((1-(phenylmethyl) -1H-imidazol-2-yl)carbonyl-1H-pyrrole-3-carboxylate

Prepared from the product of step a) by the method of Compound 5.1e) and 5.1f), mp 214-215° (dec).

5.9 Methyl 5-Ethyl-2-methyl-4-((1-(phenylmethyl)-1H-pyrrol-2-yl)carbonyl)-1H-pyrrole-3-carboxylate

a) Methyl 1-(((1,1-dimethylethoxy)carbonyl)amino)-5-ethyl-2-methyl-4-((1-phenylmethyl-1H-pyrrol-2-yl)-carbonyl)-1H-pyrrole-3-carboxylate

Prepared by the method of Compound 5.1d).
$M+1^+$ 466; nmr ($CDCl_3$) delta 3.25(s,3H), 6.07(dd,1H) and 6.95(t,1H)

b) Methyl 5-ethyl-2-methyl-4-((1-(phenylmethyl)-1H-pyrrol-2-yl)carbonyl)-1H-pyrrole-3-carboxylate

Prepared from the product of step a) by the method of Compound 5.1e) and 5.1f), mp 151-152°.

5.10 Methyl 2-methyl-4-(2-phenylmethyl)benzoyl)-5-trifluoromethyl-1H-pyrrole-3-carboxylate

mp 193-194°

5.11 Methyl 4-(2-((4-methoxyphenyl)methyl)benzoyl)-2,5-dimethyl-1H-pyrrole-3-carboxylate

mp 169-170°

5.12 Methyl 4-((9H-fluoren-1-yl)carbonyl)-2,5-dimethyl-1H-pyrrole-3-carboxylate

mp 171-173°.

5.13 Methyl 2-methyl-4,5-di(2-pyridinyl)-1H-pyrrole-3-carboxylate

2-Pyridinecarboxaldehyde (10.7g, 0.1mol) and methyl 3-amino-2-butenoate (11.5g, 0.1mol) were heated at reflux in nitroethane (7.5g) and t-butanol (50ml) for 36 hours. The solvent was evaporated and the residue chromatographed on silica eluting with ethylacetate/petroleum ether (60-80°) mixtures followed by further chromatography on silica eluting with tetrahydrofuran/hexane. Crystallisation from ether/cyclohexane gave the title compound (0.35g), mp 114-116°.

5.14 (2-Chlorophenyl) (2,5-Dimethyl-4-phenyl-1H-pyrrol-3-yl)methanone

a) 1-(2-Chlorophenyl)-1,3-butanedione

Boron triflouride was bubbled through a solution at -78° of 1-(2-chlorophenyl)ethanone (14.5g, 94mmol) and acetic anhydride (20ml) in dichloromethane (200ml) for 1 hour. The mixture was allowed to warm to room temperature and left overnight. Sodium acetate (80g) in water (100ml) was added and the mixture heated at reflux for 2 hours. The organic layer was separated, washed with water and brine, dried ($MgSO_4$) and the solvent evaporated. The residue was heated at 90-100° for 16 hours in saturated aqueous sodium acetate (100ml). The reaction mixture was diluted with water and extracted with ethyl acetate. The organic extract was washed with brine, dried ($MgSO_4$) and the solvent exaporated to give the sub-title dione (7.6g).
$M^+$ 196/8; nmr ($CDCl_3$) delta 2.2(s,3H) and 6.04(s,1H).

b) 3-Amino-1-(2-chlorophenyl)-2-buten-1-one

Ammonia was bubbled through a refluxing solution of the product of step a) (12g, 61mmol) in toluene (100ml) containing ammonium acetate (1g) in a Dean-Stark apparatus. After 7 hours, the reaction mixture was cooled and the resulting solid filtered off to give the sub-title enamine (9.4g)
$M^+$ 195/7; nmr ($CDCl_3$) delta 2.04 (s,3H) and 5.38 (s,1H)

c) (2-Chlorophenyl) (2,5-dimethyl-4-phenyl-1H-pyrrol-3-yl)methanone

The product of step b) (1g, 5.1mmol) and 2-nitro-1-phenyl-1-propene (0.9g, 5.5mmol) were heated at reflux for 1 day in tert-butanol (5ml) under nitrogen. The solvent was evaporated and the residue was purified by chromatography on silica (twice) eluting with ethyl acetate/petroleum ether (60-80°) and then dichloromethane/ethyl acetate. Crystallisation from 2-propanol gave the title compound (0.175g), mp 220-222°.

5.15 Methyl 4-(4-(1H-imidazol-1-yl)phenyl)-2,5-dimethyl-1H-pyrrole-3-carboxylate

Prepared by the method of Example 5.14c) from methyl 3-amino-2-butenoate and 1-(4-(1H-imidazol)-1-yl)-phenyl)-2-nitro-2-propene, mp 216-218°.

Example 6

6.1 Methyl 2,5-dimethyl-4-(2-pyridinyl)-1H-pyrrole-3-carboxylate

Acetaldehyde (1.6g, 36.6mmol) in dimethylformamide (2ml) was added over 45 minutes to a solution of methyl 3-oxo-2-((2-pyridinyl)methylene)butanoate (2.5g, 12.2mmol), triethylamine (0.88g, 8.7mmol) and 5-(2-hydroxyethyl)-4-methyl-3-(phenylmethyl)thiazolium chloride (0.4g) in dimethylformamide (10ml) at 80° under nitrogen. After 4.5 hours at 80°, ammonium acetate (2.35g, 30.5mmol) was added and the heating continued for a further 16 hours. The solvent was evaporated and the residue was dissolved in ethyl acetate and saturated sodium bicarbonate. The organic layer was separated, dried ($Na_2SO_4$) and the solvent wa evaporated. Chromatography on silica eluting with chloroform/methanol mixtures followed by trituration with ether gave the title compound (0.95g), mp 156-158°.

Example 7

7.1 Methyl 2,5-Dimethyl-4-(2-(phenylmethyl)benzoyl)-1H-pyrrole-3-carboxylate

a) Methyl 4-Bromo-2,5-dimethyl-1H-pyrrole-3-carboxylate

A stirred solution of methyl 2,5-dimethyl-1H-pyrrole -3-carboxylate (1.5g, 9.8mmol) in chloroform (60ml) and triethylamine (2.5ml) cooled to 0° C was treated portionwise with pyridinium perbromide (3.5g, 10.94 mmol). The mixture was allowed to warm to room temperature and then silica (6g) was added in one portion and the entire mixture evaporated to dryness under reduced pressure (14 mm Hg) with gentle heating (below 50°). Chromatography on silica eluting with ethyl acetate-hexane mixtures gave the sub-title compound (1.9g), $M^+$ 231/233.

b) Methyl 4-Bromo-2,5-dimethyl-1-((2-(trimethylsilyl) ethoxy)methyl)-1H-pyrrole-3-carboxylate

The product of step a) (2g, 8.62mmol) in tetrahydrofuran (20ml) was added dropwise to a stirred and ice-cooled suspension of sodium hydride (0.3g, 12.5mmol) in tetrahydrofuran (60ml). After stirring at room temperature for 45 minutes the resulting pale yellow suspension was treated with (2-(trimethylsilyl) ethoxy)-

methyl chloride (1.7ml, 10.2mmol). After stirring for 2 hours at room temperature the reaction was quenched with water (5ml) and evaporated to near dryness. The residue was extracted with chloroform and the combined extracts dried ($MgSO_4$) and evaporated. Chromatography of the resulting oil on silica eluting with ethyl acetate-hexane mixtures gave the sub-title compound (2.67g), $M^+$361/363.

c) Methyl 2,5-Dimethyl-4-(2-(phenylmethyl)benzoyl)-1-((2-(trimethylsilyl)ethoxy)methyl)-1H-pyrrole-3-carboxylate

The product of step b) (1.3g, 3.59mmol) in tetrahydrofuran (20ml) at -78° under nitrogen was treated dropwise with n-butyl lithium (2.2ml, 1.6 M in hexane, 3.56mmol) and the resulting mixture stirred at -78° for 15 minutes. 2-(Phenylmethyl)benzoyl chloride (1g, 4.34mmol) in tetrahydrofuran (5ml) was rapidly added and the mixture evaporated to near dryness and the residue extracted with chloroform. The combined extracts were dried ($MgSO_4$) and evaporated. Chromatography of the resulting oil on silica eluted with ethyl acetate-hexane mixtures gave the sub-title compound (1.3g), $M^+$477.

d) Methyl 2,5-Dimethyl-4-(2-(phenylmethyl)benzoyl)-1H-pyrrole-3-carboxylate

Boron trifluoride etherate (1.5ml, 12.2mmol) was added dropwise to a stirred solution of the product of step c) (0.58g, 1.21mmol) at 0° under nitrogen. The mixture was warmed to room temperature, stirred for 2 hours, then quenched with saturated aqueous sodium bicarbonate (50ml). The resulting mixture was extracted with dichloromethane and the combined extracts dried ($MgSO_4$), and evaporated to give a yellow gum which dissolved in acetonitrile (30ml). This solution was treated dropwise with 10 drops of trimethyl-(phenylmethyl) ammonium hydroxide (Triton B) (40% in methanol) and stirred at room temperature overnight. The mixture was carefully evaporated and the residue extracted with chloroform. The combined extracts were washed with water, dried ($MgSO_4$) and evaporated. Chromatography of the resulting oil on silica eluting with ethyl acetate-hexane mixtures gave the title compound (0.31g) as a white solid, mp 143-144°.

7.2 Methyl 2,5-Dimethyl-4-(2-phenylbenzoyl)-1H-pyrrole -3-carboxylate

The method of Compound 7.1 using 2-phenylbenzoyl chloride gave the title compound as a whitel solid, mp 185-186°.

7.3 (E)Methyl 5-(2-cyanoethenyl)-2-methyl-4-(2-(phenyl methyl)benzoyl)-1H-pyrrole-3-carboxylate

a) Methyl 5-bromo-2-methyl-4-(2-(Phenylmethyl) benzoyl)-1-((2-(trimethylsilyl)ethoxy)methyl)-1H-pyrrole-3-carboxylate

Prepared by the method of Example 7.1b) from the compound of Example 3.4.

b) Methyl 5-formyl-2-methyl-4-(2-phenylmethyl)benzoyl) -1-((2-(trimethylsilyl)ethoxy)methyl)-1H-pyrrole-3-carboxylate

Prepared by the method of Example 7.1c) using methyl formate, nmr delta ($CDl_3$) 9.24(2,1H), 5.85(s,2H), 3.42(s,3H).

c) E-Methyl 5-(2-cyanoethenyl)-2-methyl-4-(2-(phenylmethyl)benzoyl)-1H-pyrrole-3-carboxylate

Prepared by the method of Example 7.1d), the Triton B/acetonitrile reaction was left for 3 days to give the cyanoethenyl compound, mp 180-181°.

7.4 Methyl 2,5-dimethyl-4-((2-phenylmethyl)phenyl)thio) -1H-pyrrole-3-carboxylate

a) Methyl 4-iodo-2,5-dimethyl-1H-pyrrole-3-carboxylate

Iodine (13.2g,5.2mmol) was added to a stirred solution at -40° of methyl 2,5-dimethyl-1H-pyrrole-3-carboxylate (8g,5.2mmol) and triethylamine (5.8g,57.5mmol) in dichloromethane (250ml). After 10 hours at room temperature, the solvent was evaporated and the residue was chromatographed on silica eluting with ethyl acetate/dichloromethane mixtures to give the sub-title iodo compound (7.7g).
$M^+$ 279; nmr delta ($CDCl_3$) 3.82(s,3H), 2.49(s,3H) and 2.20(s,3H).

b) O-(2-(phenylmethyl)phenyl) dimethylthiocarbamate

Sodium hydride (0.835g of 80%, 29mmol) was added to a solution of 2-(phenylmethyl)phenol (5g,27mmol) in dimethylformamide (80ml). After 20 minutes this solution was added dropwise over 10 minutes to a solution at 12° of dimethylthiocarbamoyl chloride (3.6g,29mmol) in dimethylformamide (50ml). After stirring at room temperature for 1.5 hours, the reaction mixture was poured onto water and ethyl acetate. The organic layer was separated, washed with water (3x) and brine, dried ($MgSO_4$) and the solvent was evaporated to give the sub-title compound (4.9g) as a yellow oil
$M^+$ 271; nmr delta ($CDCl_3$) 3.93 (s,2H), 3.44 (s,3H) and 3.11 (s,3H).

c) S-(2-(phenylmethyl)phenyl) dimethylthiocarbamate

The product of step b) (4.6g,17mmol) in 2,2′-oxydiethanol (50ml) was heated at 245° for 4 hours. Chromatography on silica eluting with tetrahydrofuran/petroleum ether (60-80°) gave the sub-title compounds (3.8g).
$M^+$271; nmr delta ($CDCl_3$) 4.19 (s,2H), 3.10 (s,3H) and 3.01 (s,3H).

d) 2-(Phenylmethyl)benzenethiol

The product of step c) (3.7g,14mmol), methanol (60ml) and 10% aqueous sodium hydroxide (30ml) were heated at reflux for 15 hours. The reaction mixture was poured onto ethyl acetate and dilute hydrochloric acid. The organic layer was separated, dried ($MgSO_4$) and the solvent was evaporated. Chromatography on silica eluting with hexane gave the sub-title thiol (1.5g).
Nmr delta ($CDCl_3$) 4.06 (s,2H) and 3.17 (s,1H).

e) Bis(2-(phenylmethyl)phenyl)disulphide

The product of step d) (1.0g,5mmol) was added to a stirred mixture of pyridinium chlorochromate (1.08g,5mmol) and florisil (2g) in dichloromethane (75ml). After 15 minutes, the reaction mixture was filtered through a short silica column; eluting further with tetrahydrofuran/hexane mixtures. The sub-title disulphide (0.96g) was obtained as an oil.
$M^+$ 398; nmr delta ($CDCl_3$) 4.14 (s,4H).

f) Methyl 2,5-dimethyl-4-((2-(phenylmethyl)phenyl)thio) -1-((2-(trimethylsilyl)ethoxy)methyl)-1H-pyrrole-3-carboxylate

Prepared by the method of Example 7.1b) and 7.1c) from the product of Example 7.4a) without isolation of the intermediates and reaction with the product of Example 7.4e).
$M^+$ 467; nmr delta ($CDCl_3$) 5.23 (s,2H), 4.20 (s,2H), 3.59 (s,3H), 2.62(s,3H), 2.29 (s,3H) and 0.00 (s,9H).

g) Methyl 2,5-dimethyl-4-((2-(phenylmethyl)phenyl)thio) -1H-pyrrole-3-carboxylate

Tetra-n-butyl ammonium fluoride (0.7g,2.2mmol) in tetrahydrofuran (20ml) was added to a solution of the product of step f) (0.22g, 0.46mmol) in tetrahydrofuran (20ml). After heating at 55° for 5 hours, the reaction mixture was poured onto water and ethyl acetate. The organic layer was separated, dried ($MgSO_4$) and the solvent was evaporated. HPLC eluting with tetrahydrofuran/hexane gave the title sulphide.
$M+I^+$ 352; nmr delta ($CDCl_3$) 4.20 (s,2H), 3.60 (2,3H), 2.54 (s,3H) and 2.20 (s,3H).

Example 8

8.1 Methyl 4-(((2-(3,4-dimethoxyphenyl)ethyl)amino) carbonyl)-2,5-dimethyl-1H-pyrrole-3-carboxylate

a) Methyl 2,5-dimethyl-4-(trichloroacetyl)-1H-pyrrole-3-carboxylate

Prepared by the method of Compound 1.1, mp 156-158°.

b) Methyl 4-(((2-(3,4-dimethyoxyphenyl)ethyl)amino) carbonyl)-2,5-dimethyl-1H-pyrrole-3-carboxylate

Prepared by reaction of the product of step a) with 2-(3,4-dimethyoxyphenyl)ethylamine, mp 162-164°.
Compounds 8.2-8.3 were prepared from Compound 8.1a) by methods analogous to that of Compound 8.1b) using appropriate amines.

8.2 Methyl 4-(((1H-benzimidazol-2-yl)amino)carbonyl)-2,5-dimethyl-1H-pyrrole-3-carboxylate hydrochloride hydrate

mp 213-215°

8.3 Methyl 2,5-dimethyl-4-(((2-(4-morpholinyl)ethyl)amino) carbonyl)-1H-pyrrole-3-carboxylate

Prepared as the maleate hemihydrate, mp 111-113°.

8.4 Methyl 2,5-dimethyl-4-(oxo((phenylmethyl)amino) acetyl)-1H-pyrrole-3-carboxylate

a) Ethyl 4-(methoxycarbonyl)-2,5-dimethyl-α-oxo-1H-pyrrole-3-acetate

Prepared by the method of Compound 1.1, mp 117-119°.

b) Methyl 2,5-dimethyl-4-(oxo(phenylmethyl)amino) acetyl)-1H-pyrrole-3-carboxylate

Prepared by reaction of the product of step a) with $C_6H_5CH_2NHAl(CH_3)_2$, mp 219-221°.

8.5 Methyl 4-(((2-benzothiazolyl)amino)carbonyl)-2,5-dimethyl-1H-pyrrole-3-carboxylate

a) Monomethyl 2,5-dimethyl-1H-pyrrole-3,4-dicarboxylate

The product of Example 8.1a) (0.7g, 2.34mmol) was heated at reflux for 3 hours in tetrahydrofuran (15ml), methanol (5ml) and sodium hydroxide solution (2.5ml of 1M). The solvent was evaporated after acidification and then the solid dried by azeotropic distillation with benzene to give the sub-title acid. $M+1^+$ 198; nmr delta ($D_6$DMSO) 3.78(s,3H), 2.33(s,3H) and 2.30(s,3H).

b) Methyl 4-(((2-benzothiazolyl)amino)carbonyl)-2,5-dimethyl-1H-pyrrole-3-carboxylate

Phosphorus pentachloride (0.52g, 2.5mmol) was added to a stirred suspension of the product of step a) (0.5g, 2.5mmol) in dichloromethane (30ml). After 30 minutes, the solution was filtered and 2-benzothiazolamine (0.76g, 5mmol), 4-dimethylaminopyridine (0.03g) and acetonitrile (20ml) were added to the filtrate. After 24 hours, the solvent was evaporated and the residue dissolved in ethyl acetate and saturated sodium bicarbonate. The organic layer was separated, dried ($MgSO_4$) and the solvent evaporated. Chromatography on silica eluting with dichloromethane/ethyl acetate gave the title compound (0.25g) mp>250°.

Example 9

9.1 Methyl 4-(3,4-dihydro-6,7-dimethoxy-1-isoquinolinyl)-2,5-dimethyl-1H-pyrrole-3-carboxylate

Prepared from Compound 8.1 by treatment with phosphorous oxychloride and acetonitrile, mp 258-259°.

9.2 Methyl 4-(5-anthracenyl)-2-formyl-5-methyl-1H-pyrrole-3-carboxylate

Obtained as a by-product in the preparation of Compound 3.12, mp 244° (dec).

9.3 Methyl 4-(5-anthracenyl)-5-formyl-2-methyl-1H-pyrrole-3-carboxylate

Obtained as a by-product in the preparation of Compound 3.11, mp>280°.

9.4 Methyl 4-(11H-dibenz(b.e)azepin-6-yl)-5-methyl-1H-pyrrole-3-carboxylate

a) Methyl 4-(2-((2-nitrophenyl)methyl)benzoyl)-5-methyl-1H-pyrrole-3-carboxylate

Prepared by the method of Example 1.1 $M^+$378.

b) Methyl 4-(11H-dibenz(b.e)azepin-6-yl)-5-methyl-1H-pyrrole-3-carboxylate

Prepared by hydrogenation of the product of step a), by the method of Example 12, mp 239-240°.

9.5 (i) Methyl 2,5-dimethyl-4-(2,3,4,5-tetrahydro-1H-benzazepin-2-yl)-1H-pyrrole-3-carboxylate

(ii) Methyl 2,5-dimethyl-4-(4,5-dihydro-3H-benzazepin-2-yl)-1H-pyrrole-3-carboxylate

a) Methyl 4-(1-oxo-4-(2-nitrophenyl)butyl)-2,5-dimethyl-1H-pyrrole-3-carboxylate

Prepared by the method of Compound 1.1; nmr delta ($CDCl_3$) 3.78 (s,3H), 2.41 (s,3H) and 2.24 (s,3H).

b) (i) Methyl 2,5-dimethyl-4-(2,3,4,5-tetrahydro-1H-benzazepin-2-yl-1H-pyrrole-3-carboxylate

(ii) Methyl 2,5-dimethyl-4-(4,5-dihydro-3H-benzazepin-2-yl)-1H-pyrrole-3-carboxylate

Obtained as a mixture by hydrogenation over $PtO_2$ of the product of step a), by the method of Example 12, mp (i) 164-165°, (ii) 226-227°.

9.6 Methyl 4-(3,4-dihydro-1-isoquinolinyl))-2,5-dimethyl-1H-pyrrole-3-carboxylate

a) Methyl 2.5-dimethyl-4-(2-(2-carboxyethyl)benzoyl)-1H-pyrrole-3-carboxylate

The compound of Example 2.10 (2g, 6.08mmol) was hydrogenated at 3 atmospheres in methanol for 16 hours over 10% palladium on carbon. The catalyst was filtered and the solvent was evaporated to give the acid (2.0g), nmr delta ($D_5$DMSO) 11.51(brs,1H), 2.83(t,2H), 2.44(t,2H).

b) Methyl 4-(2-(2-(((1,1-dimethylethylethoxy)carbonyl) amino)ethyl)benzoyl)-2,5-dimethyl-1H-pyrrole-3-carboxylate

Diphenylphosphoryl azide (1.6g, 5.9mmol) was added to a solution of the product of step a) (2g, 6.08mmol) and triethylamine (0.7g, 6.9mmol) in t-butanol (100ml). The mixture was heated at reflux for 4 hours and then the solvent was evaporated. Chromatography on silica eluting with ethyl acetate/petroleum ether (60-80°) followed by rechromatography on silica eluting with ethylacetate/dichloromethane gave the sub-title compound (0.16g).
$M+1^{+}$ 401; nmr delta($CDCl_3$) 3.24(s,3H), 2.43(s,3H), 2.35(s,3H) and 1.40(s,9H).

c) Methyl 4-(3,4-dihydro-1-isoquinolinyl))-2,5-dimethyl-1H-pyrrole-3-carboxylate

The product of step b) (0.16g, 0.4mmol) was heated at reflux for 15 minutes in tetrahydrofuran (5ml) and hydrochloric acid (1ml of 2M). After cooling, ethyl acetate and saturated sodium bicarbonate were added. The organic layer was separated, dried ($MgSO_4$) and the solvent evaporated. Chromatography on silica eluting with chloroform/methanol gave the title compound (0.04g), mp 138° (dec).

9.7 (±) Methyl 4-(4,5-dihydro-5-phenyl-2-oxazolyl)-2,5-dimethyl-1H-pyrrole-3-carboxylate

Methyl 2,5-dimethyl-4-(trichloroacetyl)-1H-pyrrole-3-carboxylate (1.0g, 3.35mmol) and beta-hydroxybenzeneethanamine (1g, 7.3mmol) were heated a 110° in toluene (3ml) for 4 hours. The cooled reaction mixture was dissolved in ethylacetate and dilute hydrochloric acid. The organic layer was separated, dried ($Na_2SO_4$) and the solvent was evaporated. The residue was dissolved in dichloromethane (20ml) and thionylchloride (0.37ml, 5.2mmol) was added slowly with stirring. After 2 hours at room temperature, the solvent was evaporated and the residue was dissolved in chloroform. This solution was added to saturated sodium bicabonate. The organic layer was separated, dried ($Na_2SO_4$) and the solvent was evaporated. Chromatography on silica eluting with dichloromethane/methanol followed by trituration with ether gave the title compound (0.6g), mp 165-168°.

9.8 (S) and (R) Methyl 4-(4,5-dihydro-4-phenyl-2-oxazolyl)-2,5-dimethyl-1H-pyrrole-3-carboxylate

Prepared by the method of Example 9.7.
mp (S) 153-154°, (R) 135-136°.

9.9 Methyl 2,5-dimethyl-4-(2-quinolinyl)-1H-pyrrole-3-carboxylate

a) Methyl 2,5-dimethyl-4-(3-(2-nitrophenyl)-1-oxo-2-propenyl)-1H-pyrrole-3-carboxylate

Prepared by the method of Example 1.1.
Nmr delta($CDCl_3$) 3.63(s,3H), 2.37(s,3H), and 2.24(s,3H).

b) Methyl 2,5-dimethyl-4-(3-(2-nitrophenyl)-1-oxopropyl)-1H-pyrrole-3-carboxylate

Tributyltin hydride (2ml) was added dropwise to a solution of the product of step a) (1.5g, 4.6mmol) and tetrakistriphenylphosphine palladium (0) (catalytic) in tetrahydrofuran (50ml). After 4 hours, more tributyltin hydride (1ml) was added and the mixture stirred for 16 hours. The reaction mixture was added to water and ethyl acetate. The organic layer was separated, dried ($MgSO_4$) and the solvent was evaporated. Chromatography on silica eluting with ethyl acetate/hexane gave the sub-title compound (1.5g).
$M^+$ 330; nmr delta ($CDCl_3$) 3.75(s,3H), 3.25(t,2H), 3.12(t,2H.

c) Methyl 2,5-dimethyl-4-(2-quinolinyl)-1H-pyrrole-3-carboxylate

The product of step b) was hydrogenated at 4 atmospheres in acetic acid (400ml) over 10% palladium on carbon for 16 hours. The catalyst was filtered off, the solvent was evaporated and the residue was chromatograhed on silica eluting with ethyl acetate/hexane to give the title compound (0.17g), mp 210-211°.

9.10 Methyl 2,5-dimethyl-4-(2-phenyl-1H-imidazol-4-yl)-1H- pyrrole-3-carboxylate

a) Methyl 4-(chloroacetyl)-2,5-dimethyl-1H-pyrrole-3-carboxylate

Prepared by the method of Example 1.1.
$M^+$ 229/231; nmr delta ($CDCl_3$) 4.55(s,2H), 3.84(2,3H).

b) Methyl 2,5-dimethyl-4-(2-phenyl-1H-imidazol-4-yl)-1H-pyrrole-3-carboxylate

Methyl 4-(chloroacetyl)-2,5-dimethyl-1H-pyrrole-3-carboxylate (0.5g, 2mmol), benzamidine hydrochloride (0.38g, 2mmol) and sodium bicarbonate (0.36g, 4mmol) were heated at reflux in acetonitrile (20ml) for 1.5 days. The reaction mixture was poured onto dilute hydrochloric acid and ethyl acetate. The aqueous layer was separated, basified with sodium bicarbonate and extracted with ethyl acetate. The organic extract was washed with brine, dried ($MgSO_4$) and the solvent was evaporated. Chromatography on silica eluting with dichloromethane/methanol followed by trituration with ether gave the title compound (0.26g), mp 233-234°.

Example 10

10.1 Methyl 4-((imino(phenylamino)methyl)amino) carbonyl)-2,5-dimethyl-1H-pyrrole-3-carboxylate hydrochloride

a) Methyl 4-((cyanoamino)carbonyl)-2,5-dimethyl-1H-pyrrole-3-carboxylate

Prepared by the reaction of Compound 8.1a) with sodium cyanamide, $M^+$ 221.

b) Methyl 4-((imino(phenylamino)methyl)amino) carbonyl)-2,5-dimethyl-1H-pyrrole-3-carboxylate hydrochloride

The product of step a) (1g, 4.5mmol) and aniline (5ml) were heated at 110° for 4 hours. Excess aniline was evaporated. Trituration with ether and recrystallisation from acetonitrite/methanol gave the title compound free base. The hydrochloride salt was prepared and crystallised from methanol/ethylacetate (0.39g), mp 228° (dec).

Example 11

11.1 1-Methylethyl, 2,5-Dimethyl-4-(2-(phenylmethyl) benzoyl)-1H-pyrrole-3-carboxylate

Phosphorous pentachloride (0.315g, 1.5mmol) was added to a stirred suspension of 2,5-dimethyl-4-(2-(phenylmethyl) benzoyl)-1H-pyrrole-3-carboxylic acid (0.5g, 1.5mmol) in dichloromethane (80ml). After 1 hour, 2-propanol (1ml) was added and then after a further 2 hours the reaction was quenched with potassium carbonate (2g) in water (50ml). After 2 hours of vigorous stirring, the organic layer was separated, washed with sodium carbonate solution, water and brine, dried ($MgSO_4$) and the solvent evaporated. Chromatography on silica eluting with ethylacetate/hexane followed by rechromatography eluting with tetrahydrofuran/hexane and trituration with ether gave the title compound (0.34g), mp 112-113°.

11.2 2-Methoxyethyl 2,5-Dimethyl-4-(2-(phenylmethyl) benzoyl)-1H-pyrrole-3-carboxylate

Phosphorous pentachloride (0.2g, 1mmol) was added to a suspension of 2,5-dimethyl-4-(2-(phenylmethyl)-benzoyl) -1H-pyrrole-3-carboxylic acid (0.33g, 1mmol) in dichloromethane (40ml). After 45 minutes, 2-methoxyethanol (0.075g, 1mmol) was added. After 16 hours, potassium carbonate (1g in 20ml water) was added with vigorous stirring. After 3 hours, the organic layer was separated, dried ($MgSO_4$) and the solvent was evaporated. Chromatography on silica eluting with tetrahydrofuran/hexane followed by crystallisation from ether/hexane gave the title ester (0.215g), mp 106-107°.
Compounds 11.3-11.9 were prepared from appropriate starting materials by methods analogous to those used in the preparation of Compounds 11.1 and 11.2.

11.3 Cyclopropylmethyl 2,5-Dimethyl-4-(2-(phenylmethyl) benzoyl)-1H-pyrrole-3-carboxylate

Obtained as an oil, $M^+$ 387; nmr delta ($CDCl_3$) 2.11 (s,3H), 2.43 (s,3H), 4.30 (s,2H).

11.4 2-Chloroethyl 2,5-Dimethyl-4-(2-(phenylmethyl)benzoyl) -1H-pyrrole-3-carboxylate

mp 77-78°.

11.5 2,2,2-Trifluoroethyl 2,5-Dimethyl-4-(2-(phenylmethyl) benzoyl-1H-pyrrole-3-carboxylate

mp 136-137°.

11.6 S-Methyl 2,5-Dimethyl-4-(2-(phenylmethyl)benzoyl)-1H-pyrrole-3-carbothioate

Phosphorous pentachloride (0.37g, 1.8mmol) was added with stirring to 2,5-dimethyl-4-(2-(phenylmethyl) benzoyl)-1H-pyrrole-3-carboxylic acid (0.6g, 1.8mmol) in dichloromethane (10ml). After 30 minutes, methanethiol gas was bubbled through the solution for 20 minutes. Potassium carbonate (2g) and water (50ml) were added and the mixture stirred for 2 hours. The organic layer was separated, washed with water and brine, dried ($MgSO_4$) and the solvent was evaporated. Chromatography on silica eluting with tetrahydrofuran/hexane gave the title compound (0.4g), mp 178-179°.

11.7 Cyclopentyl 4-(2-Chlorobenzoyl)-2,5-dimethyl-1H-pyrrole-3-carboxylate

mp 99-100°.

11.8 2-(Dimethylamino)ethyl 4-(2-Chlorobenzoyl)-2,5-dimethyl-1H-pyrrole-3-carboxylate

mp 174-176°.

11.9 (2-Methoxy-2-oxoethyl) 2,5-dimethyl-4-(2-phenyl methyl)benzoyl)-1H-pyrrole-3-carboxylate

mp 114-115°.

11.1(Phenylmethyl 4-(methoxycarbonyl)-2,5-dimethyl-α -oxo-1H-pyrrole-3-acetate

a) 4-(Methoxycarbonyl)-2,5-dimethyl-α-oxo-1H-pyrrole-3-acetic acid

Prepared by the method of Example 13.3 using aqueous sodium hydroxide from the compound of Example 8.4a). $M^+$ 226; nmr delta($CDCl_3$) 3.61(s,3H), 2.33(s,3H) and 2.30(s,3H).

b) Phenylmethyl (4-methoxycarbonyl)-2,5-dimethyl-α -oxo-1H-pyrrole-3-acetate

Benzyl bromide (0.37g, 2.2mmol) was added to a stirred solution of the compound of step a) (0.45g, 2 mmol) and potassium carbonate (0.3g, 2.2mmol) in dimethylformamide (5ml). After stirring for 16 hours, ethyl acetate and dilute hydrochloric acid were added. The organic layer was separated, washed with water, saturated sodium bicarbonate and brine, dried ($Na_2SO_4$) and the solvent was evaporated. Semi-prep HPLC eluting with dichloromethane/ethylacetate followed by chromatography on silica eluting with ether gave the product as a yellow oil (0.29g)
$M^+$ 315; nmr delta ($CDCl_3$) 5.26(s,2H), 3.59(s,3H), 2.42(s,3H) and 2.36(s,3H).

Example 12

12.1 Methyl 4-(2-((4-Aminophenyl)methyl)benzoyl)-2,5-dimethyl-1H-pyrrole-3-carboxylate

Methyl 2,5-dimethyl-4-(2-((4-nitrophenyl)methyl) benzoyl)-1H-pyrrole-3-carboxylate (0.7g, 1.8mmol) was hydrogenated at 1 atmosphere in dioxan/methanol (200ml of 5:1) over $PtO_2$ (30mg) for 18 hours. The solvent was evaporated and the residue purified by chromatography on silica eluting with ethyl acetate/petroleum ether (60-80°) mixtures. Trituration with hexane/ether gave the title compound (0.42g), mp 171-173°.
Compounds 12.2 and 12.3 were prepared from appropriate starting materials by methods analogous to that used in the preparation of Compound 12.1

12.2 Methyl 4-(2-(3-Aminobenzoyl)benzoyl)-2,5-dimethyl-1H-pyrrole-3-carboxylate

mp 229-230°.

12.3 Methyl 4-(2-(4-Aminophenoxy)benzoyl)-2,5-dimethyl-1H-pyrrole-3-carboxylate

$M^+$ 364; nmr ($CDCl_3$) delta 2.23 (s,3H), 2.36 (s,3H), 3.23 (s,3H)

12.4 Methyl 2-Amino-4-(2-chlorobenzoyl)-5-Methyl-1H-pyrrole-3-carboxylate

Methyl 4-(2-chlorobenzoyl)-5-methyl-2-((4-nitrophenyl) azo)-1H-pyrrole-3-carboxylate (0.2g, 0.47mmol) was hydrogenated at 1 atmosphere in ethanol (10ml) over 10% palladium on carbon for 18 hours. The reaction mixture was filtered, the solvent evaporated and the residue chromatographed in silica eluting with tetrahydrofuran/petroleum ether (60-80°) to give the title compound (0.1g), mp 207-208°.
Compounds 12.5 and 12.6 were prepared from appropriate starting materials by methods analogous to that used in the preparation of Compound 12.4.

12.5 Methyl 2-amino-5-methyl-4-(2-(phenylmethyl)benzoyl)-1H-pyrrole-3-carboxylate

mp 214-215° (dec).

12.6 Methyl 5-amino-2-methyl-4-(2-(phenylmethyl)benzoyl)-1H-pyrrole-3-carboxylate

mp 202-203° (dec).

Example 13

13.1 2,5-Dimethyl-4-(2-(phenylmethyl)benzoyl)-1H-pyrrole-3-carboxylic acid

Boron trichloride (12ml of 1M in dichloromethane, 12mmol) was added to a solution at -20° of methyl 2,5-dimethyl-4-(2-phenylmethyl)benzoyl)-1H-pyrrole-3-carboxylate (3.1g, 9mmol) in dichloromethane (150ml). After 4 days at room temperature, the reaction was quenched with methanol (5ml) and then ethyl acetate and water were added. The organic extract was washed with water and extracted with 3% sodium hydroxide solution. The aqueous extract was acidified with hydrochloric acid and the title acid (1.4g) was filtered off and dried in vacuo, mp 234-236°.

Compound 13.2 was prepared from the appropriate starting material using a method analogous to that used in the preparation of Compound 13.1.

13.2 5-Ethyl-2-methyl-4-(2-(phenylmethyl)benzoyl)-1H-pyrrole-3-carboxylic acid

$M+1^+$ 348; nmr delta ($CDCl_3$) 0.99 (t,3H), 2.38 (s,3H), 4.10 (s,2H).

13.3 Carboxymethyl 2,5-dimethyl-4-(2-(phenylmethyl) benzoyl))-1H-pyrrole-3-carboxylate

Water (5ml) was added over 2 hours to a mixture of the compound of Example 11.9 (0.6g, 1.5mmol) and potassium carbonate (1g) in methanol (30ml). After 4 hours, water and ethyl acetate were added. The aqueous layer was separated, acidified with dilute hydrochloric acid and the resulting precipitate filtered off. After drying over $P_2O_5$, the title compound (0.43g) was obtained, mp 80-90°.

13.4 4-(2-(Phenylmethyl)benzoyl)-3-methoxycarbonyl-5-methyl-1H-pyrrole-2-acetic acid

Prepared by the method of Example 13.3, mp 180-181°

13.5 E-Methyl 5-(2-carboxyethenyl)-2-methyl-4-(2-(phenylmethyl)benzoyl)-1H-pyrrole-3-carboxylate

Prepared by the method of Example 13.3 using aqueous sodium hydroxide at reflux, mp 202° (dec).

13.6 4-(2-Methoxycarbonyl)benzoyl)-2,5-dimethyl-1H-pyrrole-3-carboxylic acid

Prepared by the method of Example 13.3 on the compound of Example 1.15 using aqueous lithium hydroxide in tetrahydrofuran at room temperature; $M^+$301, nmr delta ($CDCl_3$) 3.27 (s,3H), 2.37 (s,3H) and 2.25 (s,3H).

Example 14

14.1 Methyl 4-(2-Benzoylbenzoyl)-2,5-dimethyl-1H-pyrrole-3-carboxylate

Water (20ml) was added to a solution of methyl 4-(2-(3-aminobenzoyl)benzoyl)-2,5-dimethyl-1H-pyrrole-3-carboxylate (0.67g, 1.8mmol) in methanol (20ml) containing conc. hydrochloric acid (0.2ml). The methanol was evaporated in vacuo and the aqueous suspension was diluted down with water (10ml) and conc. hydrochloric acid (0.6ml). The suspension was cooled to 5° and a solution of sodium nitrite (0.135g,

1.95mmol) in water (2ml) was added. After 15 minutes, hypophosphorous acid (20ml, 50%) was added. After stirring for 16 hours, the solid was filtered off and washed with water. The solid was dissolved in ethyl acetate and the water separated. The organic layer was dried ($Na_2SO_4$) and the solvent was evaporated. Chromatography on silica eluting with ethyl acetate/petroleum ether (60-80°) followed by rechromatography eluting with ether gave the title compound as a colourless foam (0.39g).
$M^+$ 361; nmr delta ($CDCl_3$) 3.17 (s,3H), 2.35 (s,3H), 1.74 (s,3H).
Compounds 14.2-14.4 were prepared from appropriate starting materials by methods analogous to that used in the preparation of Compound 14.1.

14.2 Methyl 2,5-Dimethyl-4-(2-phenoxybenzoyl)-1H-pyrrole-3-carboxylate

Obtained as an oil. $M^+$ 349; nmr delta ($CDCl_3$) 8.23 (br s,1H), 3.26 (s,3H), 2.39 (s,3H), 2.04 (s,3H).

14.3 Methyl 5-Methyl-4-(2-(phenylmethyl)benzoyl)-1H-pyrrole-3-carboxylate

a) Methyl 5-methyl-4-(2-((2 and 4-nitrophenyl)methyl) benzoyl)-1H-pyrrole-3-carboxylate

Prepared by the method of Compound 1.2 using methyl 5-methyl-1H-pyrrole-3-carboxylate. Obtained as a mixture of isomers. $M^+$ 378.

b) Methyl 4-(2-((4-aminophenyl)methyl)benzoyl)-1H-pyrrole-3-carboxylate

Prepared by the method of Compound 12.1. $M^+$ 348; nmr ($CDCl_3$) delta 2.13(s,3H), 3.32(s,3H), 4.16(s,2H).

c) Methyl 5-methyl-4-(2-(phenylmethyl)benzoyl)- 1H-pyrrole-3-carboxylate

mp 128-129°.

14.4 Methyl 2,5-dimethyl-4-(1-oxo-4-phenylbutyl)-1H-pyrrole-3-carboxylate

a) Methyl 2,5-dimethyl-4-(1-oxo-4-(nitrophenyl)butyl)-1H-pyrrole-3-carboxylate

Prepared by the method of Compound 1.1; nmr delta ($CDCl_3$) 3.77 (s,3H), 2.41 (s,3H) and 2.24 (s,3H).

b) Methyl 2,5-dimethyl-4-(1-oxo-4-(aminophenyl)butyl)-1H-pyrrole-3-carboxylate

Prepared from the product of step a) by the method of Compound 12.1; $M^+$314, nmr delta ($CDCl_3$) 3.73 (s,3H), 2.36 (s,3H) and 2.15 (s,3H).

c) Methyl 2,5-dimethyl-4-(1-oxo-4-phenylbutyl)-1H-pyrrole-3-carboxylate

mp 72-74°.

Example 15

15.1 i) Methyl 5-((Acetyloxy)methyl)-2-methyl-4-(2-(phenylmethyl)benzoyl)-1H-pyrrole-3-carboxylate

ii) Methyl 2-((Acetyloxy)methyl)-5-methyl-4-(2-(phenylmethyl)benzoyl)-1H-pyrrole-3-carboxylate

Methyl 2,5-dimethyl-4-(2-(phenylmethyl)benzoyl)-1H-pyrrole-3-carboxylate (2.0g, 5.8mmol) and lead tetraacetate (3.2g, 7.2mmol) were heated at 50° in acetic acid (25ml) for 24 hours. The solvent was evaporated and the residue purified by HPLC on silica (multiple fractionation) eluting with ethyl acetate/hexane. Pure 5-((acetyloxy)methyl) was obtained (0.075g) by trituration with ether/hexane, mp 90-1°. The 2-((acetyloxy)methyl) isomer was obtained contaminated with the 5-isomer.
$M+1^+$ 406; nmr delta ($CDCl_3$) 2.09 (s,3H), 2.17 (s,3H), 3.21 (s,3H), 4.28 (s,2H), 5.28 (s,2H).

Example 16

16.1 i) Methyl 5-(Hydroxymethyl)-2-methyl-4-(2-(phenylmethyl)benzoyl)-1H-pyrrole-3-carboxylate

ii) Methyl 2-(Hydroxymethyl)-5-methyl-4-(2-(phenylmethyl)benzoyl)-1H-pyrrole-3-carboxylate

To a 3:2 mixture of 5- and 2-(acetyloxy)methyl isomers from Example 15.1 (0.95g, 2.3mmol) in dichloromethane (50ml) at 0° was added boron tribromide in dichloromethane (3.1ml of 0.2g/ml). After 30 minutes at room temperature, methanol (5ml) was added and then the solvent was evaporated. Ethyl acetate was added and this solution was washed with dilute hydrochloric acid, water and brine, dried ($MgSO_4$) and the solvent was evaporated. Chromatography on silica eluting with ethyl acetate/dichloromethane gave the two pure isomers.
2-(hydroxymethyl) : oil, $M^+$ 363; nmr delta ($CDCl_3$) 1.45 (s,3H), 3.51 (s,3H), 4.02 (s,2H), 4.90 (s,2H).
5-(hydroxymethyl) : mp 164-165° (methanol).

Example 17

17.1 Methyl 4-(5-(Acetylamino)-2-chlorobenzoyl)-2,5-dimethyl-1H-pyrrole-3-carboxylate

Methyl 4-(5-amino-2-chlorobenzoyl)-2,5-dimethyl-1H-pyrrole-3-carboxylate (1.36g, 4.4mmol) was heated at 45° for 3 hours in acetic anhydride (40ml). The reaction mixture was evaporated to dryness and the residue dissolved in ethyl acetate and water. The organic extract was dried ($MgSO_4$) and the solvent evaporated. Chromatography on silica eluting with methanol/dichloromethane followed by crystallisation from acetonitrile gave the title compound (0.7g), mp 232-233°.

17.2 Methyl 4-(2-((Ethylamino)carbonyl)benzoyl)-2,5-dimethyl-1H-pyrrole-3-carboxylate

Ethyl chloroformate (0.33g, 3mmol) was added to a solution at -20° of methyl 4-(2-carboxybenzoyl)-2,5-dimethyl-1H-pyrrole-3-carboxylate (0.9g, 3mmol) and triethylamine (0.3g, 3mmol) in dichloromethane (10ml). After 2 hours, a saturated solution of ethylamine in dimethoxyethane (1,5ml, excess) was added and the mixture stirred at room temperature for 16 hours. The reaction mixture was then washed with saturated sodium bicarbonate, water, dilute hydrochloric acid and brine, dried ($MgSO_4$) and the solvent was exaporated. Chromatography on silica eluting with ethyl acetate/dichloromethane followed by crystallisation from ethyl acetate gave the title compound (0.045g), mp 185°.

17.3 N-Ethyl 4-(2-(methoxycarbonyl)benzoyl)-2,5-dimethyl -1H-pyrrole-3-carboxamide

Prepared by the method of Compound 17.2, mp 180°.

Example 18

18.1 Methyl 4-(2-Chloro-5-(methylthio)benzoyl)-2,5-dimethyl-1H-pyrrole-3-carboxylate

(a) Methyl 4-(5-amino-2-chlorobenzoyl)-2,5-dimethyl-1H-pyrrole-3-carboxylate

Prepared by the method of Example 9, mp 170-172°

(b) Methyl 4-(2-chloro-5-(methylthio)benzoyl)-2,5-dimethyl-1H-pyrrole-3-carboxylate

Amyl nitrite (0.19g, 1.6mmol) was added to a solution at 85° of the product of step a) (0.5g, 1.6mmol) in dimethyldisulphide (2ml). After 2 hours at 85°, the solution was chromatographed on silica eluting with ethylacetate (dichloromethane). Trituration with ether gave the title compound (0.2g), mp 153-154°.

Example 19

19.1 (Z)- and (E)-Methyl 5-((hydroxyimino)methyl)-2-methyl-4-(2-(phenylmethyl)benzoyl)-1H-pyrrole-3-carboxylate

Methyl 5-formyl-2-methyl-4-(2-(phenylmethyl)benzoyl) 1H-pyrrole-3-carboxylate (0.18g, 0.5mmol), hydroxylamine hydrochloride (0.035g), pyridine (0.2ml) and ethanol (2ml) were heated at reflux for 1 1/2 hours. The solvent was evaporated and the residue purified and separated using semi-prep HPLC eluting with dichloromethane/methanol to give the Z-isomer (0.075g), mp 139-40°, and E-isomer (0.070g), mp 177-8°.

19.2 (E)Methyl 5-(((aminocarbonyl)hydrazono)methyl)-2-methyl-4-(2-(phenylmethyl)benzoyl)-1H-pyrrole-3-carboxylate

Methyl 5-formyl-2-methyl-4-(2-(phenylmethyl)benzoyl)-1H-pyrrole-3-carboxylate (0.075g, 0.2mmol), semicarbazide hydrochloride (0.025g) and sodium acetate (0.225g) were heated at reflux for 4 hours in ethanol (7ml) and water (1ml). The solvent was evaporated and the residue dissolved in ethyl acetate and water. The organic layer was separated, dried ($MgSO_4$) and the solvent was evaporated. Chromatography on silica eluting with dichloromethane/ethyl acetate followed by trituration with ether gave the title compound (0.055g), mp 205-6° (dec).

19.3 Methyl 2,5-dimethyl-4-(((phenylmethylene)hydrazino carbonyl)-1H-pyrrole-3-carboxylate

a) Methyl 4-(hydrazinocarbonyl)-2,5-dimethyl-1H-pyrrole -3-carboxylate

Prepared by the method of Example 8, $M^+$211.

b) Methyl 2,5-dimethyl-4-(((phenylmethylene)hydrazino) carbonyl-1H-pyrrole-3-carboxylate

The product of step a) (1.06g, 5mmol) in methanol (25ml) was heated with benzaldehyde (1ml). After 16 hours the product was filtered off (0.95g), mp 231-5°.

Example 20

20.1 Methyl 2,5-Dimethyl-4-(2-(phenylsulphinyl)benzoyl)-1H-pyrrole-3-carboxylate

3-Chlorobenzeneperoxoic acid (0.62g, 3.6mmol) in chloroform (10ml) was added dropwise to a solution at 0° of methyl 2,5-dimethyl-4-(2-(phenylthio)benzoyl)-1H-pyrrole-3-carboxylate (1g, 2.7mmol) in chloroform (50ml). After 1 hour, saturated sodium bicarbonate was added. The organic layer was separated, dried ($Na_2SO_4$) and the solvent was evaporated. Chromatography on silica eluting with ethyl acetate followed by crystallization from ethyl acetate gave the title sulphoxide (0.76g), mp 190-191°.

20.2 Methyl 2,5-Dimethyl-4-(2-(phenylsulphonyl)benzoyl)-1H-pyrrole-3-carboxylate

3-Chlorobenzeneperoxoic acid (1.4g, 81mmol) in chloroform (20ml) was added with stirring to a solution of methyl 2,5-dimethyl-4-(2-(phenylthio)benzoyl) -1H-pyrrole-3-carboxylate (1g, 27mmol) in chloroform (50ml). After 16 hours, saturated sodium bicarbonate was added. The organic layer was separated, dried ($Na_2SO_4$) and the solvent was evaporated. Chromatography on silica eluting with chloroform/ethyl acetate mixtures followed by crystallization from ether/petroleum ether (60-80°) gave the sulphone (0.6g), mp 197-199°.
Compounds 20.3-20.12 were prepared from appropriate starting materials by methods analogous to those used in the preparation of Compounds 20.1 and 20.2.

20.3 Methyl 4-(2-Chlorobenzoyl)-2-methyl-5-(methyl sulphinyl)-1H-pyrrole-3-carboxylate

mp 152-153°.

20.4 Methyl 4-(2-Chlorobenzoyl)-2-methyl-5-(methyl sulphonyl)-1H-pyrrole-3-carboxylate

mp 217-218°.

20.5 (2-Chlorophenyl) (2,5-Dimethyl-4-(methylsulphinyl)-1H-pyrrol-3-yl)methanone

mp 173-174° (dec).

20.6 (2-Chlorophenyl) (2,5-Dimethyl-4-(methylsulphonyl)-1H-pyrrol-3-yl)methanone

mp 213-215°.

20.7 Methyl 2,5-Dimethyl-4-(2-((phenylmethyl)sulphinyl) benzoyl)-1H-pyrrole-3-carboxylate

mp 185-186°.

20.8 Methyl 2,5-Dimethyl-4-(2-((phenylmethyl)sulphonyl) benzoyl)-1H-pyrrole-3-carboxylate

mp 202-203°.

20.9 Methyl 4-(2-Chloro-5-(methylsulphinyl)benzoyl)-2,5-dimethyl-1H-pyrrole-3-carboxylate

mp 158-160°.

20.10 Methyl 4-(2-Chloro-5-(methylsulphonyl)benzoyl)-2,5-dimethyl-1H-pyrrole-3-carboxylate

mp 184-185°.

20.11 Methyl 5-Ethyl-2-methyl-4-(2-((phenylmethyl) sulfinyl)benzoyl)-1H-pyrrole-3-carboxylate

a) Methyl 5-ethyl-2-methyl-4-(2-((phenylmethylthio) benzoyl)-1H-pyrrole-3-carboxylate

Prepared by the method of Example 1.

b) Methyl 5-ethyl-2-methyl-4-(2-(phenylmethyl) sulfinyl) benzoyl)-1H-pyrrole-3-carboxylate

Prepared using the product of step a), mp 98-99°.

20.12 Methyl 2,5-dimethyl-4-((2-(phenylmethyl)phenyl) sulphinyl)-1H-pyrrole-3-carboxylate

mp 141-142°

## Claims

1. A compound of formula I,

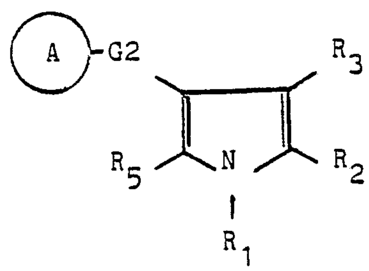

I

wherein

$R_1$ represents $-R_{11}$, $-NHR_{11}$ or $-NHCOOR_{11}$ in which $R_{11}$ is hydrogen or alkyl $C_{1-6}$,

$R_2$ and $R_5$, which may be the same or different, represent hydrogen, $-R_{21}$, -OH, $-OR_{21}$, $-NR_{22}R_{23}$, halogen, -CN, $-NO_2$, $-S(O)_qR_{21}$, $-N=NR_{24}$, $-COOR_{22}$, $-CH=R_{25}$, phenyl or pyridyl,

in which $R_{21}$ is alkyl or alkenyl $C_{1-6}$ optionally interrupted by O or S and optionally substituted by -OH, halogen, -CN, $-NO_2$, $-NR_{22}R_{23}$, $-COOR_{22}$, phenyl or $-OOCR_{26}$,

$R_{22}$ and $R_{23}$, which may be the same or different, are hydrogen or alkyl $C_{1-6}$,

$R_{24}$ is phenyl optionally substituted by $-NO_2$,

$R_{25}$ is =O, =CHCN, =NOH or $=NNHCONH_2$,

$R_{26}$ is alkyl $C_{1-6}$, and

q is 0, 1 or 2,

G2 represents a chain $-(CH_2)_z(W)_y-$, in which,

W represents C=O, $S(O)_q$ or $C=NR_{40}$,

in which $R_{40}$ is hydrogen, -OH, cycloalkyl $C_{3-8}$ or alkyl $C_{1-6}$ optionally substituted by phenyl,

y is 0, 1 or, provided W is C=O, 2,

z is 0, 1, 2 or 3, and

up to two of the methylene segments in the chain $(CH_2)_z$ are optionally replaced by -NH- and one segment is optionally replaced by -O- or $-(C=NR_{40})-$, and the chain is optionally unsaturated and optionally substituted by alkyl $C_{1-6}$ or alkoxy $C_{1-6}$,

A is a 5- or 6-membered ring or a bicyclic or tricyclic fused ring system, A being optionally substituted by one or more of halogen, -OH, $-NO_2$, $-NR_{43}R_{45}$, $-COOR_{43}$, $-S(O)_qR_{44}$, $-CONR_{43}R_{45}$, $-NHCOR_{45}$, alkyl, alkylene or alkoxy $C_{1-6}$ optionally substituted by halogen, phenyl, $-COOR_{43}$ or $-NR_{43}R_{45}$, or a group D-G3-, in which

$R_{43}$ and $R_{45}$, which may be the same or different, are hydrogen or alkyl $C_{1-6}$,

$R_{44}$ is alkyl $C_{1-6}$,

G3 is a single bond, -NH-, -O-, -CO-, $-OCH_2-$, -O-CO-, $-S(O)_q-$, $(CH_2)_p-$, $-CH_2S(O)_q-$, -CO-S-, $-SO_2-O-$ or $CH_2NH-$, in which p is 1,2 or 3, and

D represents a 5- or 6-membered ring optionally substituted by -OH, halogen, $-NO_2$, $-NR_{43}R_{45}$, alkyl $C_{1-6}$, alkoxy $C_{1-6}$ or $-SCH_2C_6H_5$, and

$R_3$ represents hydrogen, $-S(O)_qR_{31}$, $-NO_2$, -CN, -halogen, $-CH_2NR_{32}R_{33}$, $-(CO)_rR_{34}$ or an aryl group, in which

$R_{31}$ is alkyl $C_{1-6}$,

$R_{32}$ and $R_{33}$, which may be the same or different, are hydrogen, alkyl $C_{1-5}$ optionally substituted by phenyl, or phenyl optionally substituted by halogen or trihalomethyl,
r is 1 or 2,
$R_{34}$ is hydrogen, $-NR_{32}R_{33}$, alkyl $C_{1-5}$, -OH, $-OR_{35}$ or $-SR_{35}$,
$R_{35}$ is cycloalkyl $C_{3-8}$ or alkyl $C_{1-5}$ optionally substituted by alkoxy $C_{1-5}$, halogen, -OH, cycloalkyl $C_{3-8}$, $-NR_{32}R_{33}$ or $-COOR_{36}$, and
$R_{36}$ is hydrogen or alkyl $C_{1-5}$, or $R_3$ and $R_2$ together form a chain -N=CH-NH-CO-,
provided that
a) when $R_3$ represents hydrogen, then A is substituted by D-G3- where, if A is a 5-membered heterocyclic ring, D-G3- is other than phenyl, benzyl or phenyl substituted by chlorine, or, if A is phenyl or naphthyl and D is phenyl, G3 is other than $-CH_2-O-$, -S-, -O- or -NH-;
b) when $R_2$ and $R_5$ both represent hydrogen, then A is substituted by D-G3-;
c) when $R_1$ represents alkyl $C_{1-6}$ or $-NHCOOR_{11}$, and $R_2$ and $R_5$ are both alkyl $C_{1-6}$, and $R_3$ is $-COOR_{35}$ or $-NO_2$, then A is other than unsubstituted phenyl or phenyl substituted by fluorine;
d) when $R_2$ and $R_3$ both represent halogen, and A is phenyl, then A is substituted by D-G3-;
e) when one or both of $R_2$ and $R_5$ represents phenyl, and $R_3$ represents $-COOR_{35}$, $-COCH_3$, phenyl or $-NO_2$, then A is other than unsubstituted phenyl, isoxazolyl substituted by alkyl $C_{1-6}$ or phenyl, or a group

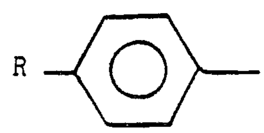

where R is halogen, alkyl $C_{1-5}$ or alkoxy $C_{1-6}$
f) when $R_5$ represents hydrogen, and $R_2$ represents alkyl $C_{1-6}$, and $R_3$ represents $-COOR_{35}$, and A is a 5- or 6-membered ring, then A is substituted by D-G3-; and
g) when $R_2$ and $R_5$ are both selected from hydrogen, $-COOR_{22}$ and alkyl $C_{1-5}$ optionally substituted by halogen, -OH, $-NR_{22}R_{23}$ or $-OOCR_{26}$ and optionally interrupted by O or S, and $R_3$ is selected from $-NO_2$, halogen, -CN, $-COR_{34}$ or $-CH_2NR_{32}R_{34}$, where $R_{34}$ is hydrogen, $-NR_{32}R_{34}$, alkyl $C_{1-6}$, -OH or $-OR_{35}$ where $R_{35}$ is alkyl $C_{1-5}$ optionally substituted by an aryl group, and $R_1$ represents hydrogen or alkyl $C_{1-6}$, and G2 represents $-(CH_2)_z(W)_y-$ in which z represents 0, 1 or 2 and none of the methylene segments are replaced by -NH-,-O- or $-(C=NR_{40})-$ and the chain is neither unsaturated not substituted by alkoxy $C_{1-6}$, then A is other than phenyl, naphthyl or benzofurazanyl optionally substituted only by halogen, -OH, $-NR_{43}R_{45}$, $-NO_2$, -COOH, alkyl or alkoxy $C_{1-6}$ optionally substituted by halogen, or D-G3- where G3 is $-SCH_2-$, $-OCH_2-$ or -O-CO- and D is phenyl optionally substituted by -OH, halogen or alkyl or alkoxy $C_{1-6}$;
and pharmaceutically acceptable derivatives thereof.

2. A compound according to Claim 1, wherein $R_3$ represents $-COOR_{35}$ or $-NO_2$.

3. A compound according to Claim 1 or Claim 2, wherein $R_2$ and $R_5$ are selected from the group consisting of hydrogen, $-R_{21}$ and halogen.

4. A compound according to any one of the preceding claims, wherein A is a 5- or 6-membered ring.

5. A compound according to Claim 4, wherein A is substituted by D-G3-.

6. A compound according to any one of the preceding claims, in which
$R_2$ and $R_5$, which may be the same or different, represent hydrogen, $-R_{21}$, or halogen,
$R_3$ represents $-NO_2$ or $-COOR_{35}$,
G2 represents C=O, -S- or a single bond, and
A represents phenyl substituted by a group D-G3-.

7. A compound according to any one of the preceding claims, in which
$R_1$ represents hydrogen,
$R_2$ and $R_5$, which may be the same or different, represent hydrogen, $-R_{21}$, or halogen,
$R_3$ represents $-NO_2$ or $-COOR_{35}$,
G2 represent C=O, and
A represents phenyl substituted by a group D-G3-, in which G3 represents -NH- or $-CH_2-$.

8. A compound according to Claim 1, which is
Methyl 2,5-Dimethyl-4-(2-((4-nitrophenyl)thio) benzoyl)-1H-pyrrole-3-carboxylate;
Methyl 2,5-Dimethyl-4-(2-((4-nitrophenyl)methyl) benzoyl)-1H-pyrrole-3-carboxylate;
Methyl 2,5-Dimethyl-4-(2-((2-nitrophenyl)methyl) benzoyl)-1H-pyrrole-3-carboxylate;
Methyl 2,5-Dimethyl-4-(2-(2-phenylethyl)benzoyl)-1H-pyrrole-3-carboxylate;

Methyl 2,5-Dimethyl-4-(2-(4-nitrophenoxy)benzoyl)-1H-pyrrole-3-carboxylate;
Methyl 2,5-Dimethyl-4-(2-(3-nitrobenzoyl)benzoyl)-1H-pyrrole-3-carboxylate;
Methyl 2,5-Dimethyl-4-(2-(((4-methylphenyl) sulphonyl)oxy)benzoyl)-1H-pyrrole-3-carboxylate;
Methyl 4-(2-Chlorobenzoyl)-2-methyl-5-(methylthio)-1H-pyrrole-3-carboxylate;
Methyl 2,5-Dimethyl-4-(2-(4-pyridinylamino)benzoyl) -1H-pyrrole-3-carboxylate;
Methyl 4-(2-(Cyclohexylmethyl)benzoyl)-2,5-dimethyl -1H-pyrrole-3-carboxylate;
Methyl 4-(2-Chlorobenzoyl)-2-methyl-5-phenyl-1H-pyrrole-3-carboxylate;
Methyl 4-(2-Chlorobenzoyl)-2,5-dimethyl-α-oxo-1H-pyrrole-3-acetate;
(2-Chlorophenyl) (2,5-Dimethyl-4-(5-methyl-1,3,4- oxadiazol-2-yl)-1H-pyrrol-3-yl)methanone;
Methyl 4-(2-(2-(Dimethylamino)ethoxy)benzoyl)-2,5-dimethyl-1H-pyrrole-3-carboxylate;
Methyl 2,5-Dimethyl-4-(2-((2-((phenylmethyl)thio) benzoyl)thio)benzoyl)-1H-pyrrole-3-carboxylate;
Methyl 4-(2-(Methoxycarbonyl)benzoyl)-2,5-dimethyl-1H-pyrrole-3-carboxylate;
Methyl 3-(2-Chlorobenzoyl)-4-(methoxycarbonyl)-5-methyl-1H-pyrrole-2-propanoate;
Methyl 4-(2-Chlorobenzoyl)-5-methyl-2-(2-nitroethyl) -1H-pyrrole-3-carboxylate;
Methyl 2,5-Dimethyl-4-(3-pyridinylcarbonyl)-1H-pyrrole-3-carboxylate;
Methyl 2,5-Dimethyl-4-(4-pyridinylcarbonyl)-1H-pyrrole-3-carboxylate;
5-(2-Chlorobenzoyl)-3,4-dihydro-6-methyl-7H-pyrrolo(2,3-d)pyrimidin-4-one;
(E)Methyl 4-(3-methoxy-1-oxo-4-phenyl-2-butenyl-2,5-dimethyl-1H-pyrrole-3-carboxylate;
(2,5-Dimethyl-1H-pyrrol-3-yl) (2-(phenylmethyl) phenyl)methanone;
Methyl 2,5-Dimethyl-4-(2-(phenylthio)benzoyl)-1H-pyrrole-3-carboxylate;
Methyl 2,5-Dimethyl-4-(2-(phenylamino)benzoyl)-1H-pyrrole-3-carboxylate;
Methyl 2,5-Dimethyl-4-(2-(2-pyridinylamino)benzoyl) -1H-pyrrole-3-carboxylate;
Methyl 5-Ethyl-2-methyl-4-(2-(phenylamino)benzoyl)-1H-pyrrole-3-carboxylate;
Methyl 2,5-dimethyl-4-(2-(cyclohexylamino)benzoyl)-1H-pyrrole-3-carboxylate;
Methyl 2,5-dimethyl-4-(2-((phenylmethyl)amino)benzoyl) -1H-pyrrole-3-carboxylate;
Methyl 2,5-dimethyl-4-(2-((phenylmethyl)amino)phenyl) ((phenylmethyl)imino)methyl)-1H-pyrrole-3-carboxylate;
Methyl 4-((2-(cyclohexylamino)phenyl) (cyclohexyl imino)methyl-2,5-dimethyl-1H-pyrrole-3-carboxylate;
(E)-Methyl 2,5-dimethyl-4-(2-(2-phenylethenyl) benzoyl)-1H-pyrrole-3-carboxylate;
E-3-(2-((4-(Methoxycarbonyl)-2,5-dimethyl-1H-pyrrol-3-yl)carbonyl)phenyl)-2-propenoic acid;
Methyl 5-(2-(methoxycarbonyl)ethenyl)-2-methyl-4-(2-(phenylmethyl)benzoyl)-1H-pyrrole-3-carboxylate;
Methyl 2-methyl-5-phenyl-4-(2-(phenylmethyl)benzoyl) -1H-pyrrole-3-carboxylate;
Methyl 2-methyl-5-(phenylmethyl)-4-(2-(phenylmethyl) benzoyl)-1H-pyrrole-3-carboxylate;
Methyl 2-Bromo-4-(2-chlorobenzoyl)-5-methyl-1H-pyrrole-3-carboxylate;
(4-Bromo-2,5-dimethyl-1H-pyrrol-3-yl) (2-(phenylmethyl) phenyl)methanone;
Methyl 2-Bromo-5-methyl-4-(2-phenylmethyl)benzoyl)-1H-pyrrole-3-carboxylate;
Methyl 5-Bromo-2-methyl-4-(2-phenylmethyl)benzoyl)-1H-pyrrole-3-carboxylate;
Methyl 4-(2-Chlorobenzoyl)-2-cyano-5-methyl-1H-pyrrole-3-carboxylate;
2,5-Dimethyl-4-(2-(phenylmethyl)benzoyl)-1H-pyrrole-3-carbonitrile;
Methyl 2-Cyano-5-methyl-4-(2-(phenylmethyl)benzoyl)-1H-pyrrole-3-carboxylate;
Methyl 5-Cyano-2-methyl-4-(2-(phenylmethyl)benzoyl)-1H-pyrrole-3-carboxylate;
2,5-Dimethyl-4-(2-(phenylmethyl)benzoyl)-1H-pyrrole-3-carboxamide;
Methyl 4-(2-Chlorobenzoyl)-2-formyl-5-methyl-1H-pyrrole-3-carboxylate;
Methyl 5-Formyl-2-methyl-4-(2-(phenylmethyl)benzoyl)-1H-pyrrole-3-carboxylate;
Methyl 2-Formyl-5-methyl-4-(2-(phenylmethyl)benzoyl)-1H-pyrrole-3-carboxylate;
2,5-Dimethyl-4-(2-phenylmethyl)benzoyl)-1H-pyrrole-3-carboxaldehyde;
Methyl 4-(2-Chlorobenzoyl)-5-methyl-2-nitro-1H-pyrrole-3-carboxylate;
(2,5-Dimethyl-4-nitro-1H-pyrrol-3-yl) (2-(phenylmethyl)phenyl)methanone;
Methyl 4-(2-Chlorobenzoyl)-5-methyl-2-((4-nitrophenyl) azo)-1H-pyrrole-3-carboxylate;
(2-Chlorophenyl) (2,5-Dimethyl-4-(methylthio)-1H-pyrrol-3-yl)methanone;
Methyl 2-methyl-5-((4-nitrophenyl)azo)-4-(2-(phenylmethyl)benzoyl)-1H-pyrrole-3-carboxylate;
Methyl 5-methyl-2-((4-nitrophenyl)azo)-4-(2-(phenylmethyl)benzoyl)-1H-pyrrole-3-carboxylate;
(4-((Dimethylamino)methyl)-2,5-dimethyl-1H-pyrrol-3-yl) ((2-phenylmethyl)phenyl)methanone;
Methyl 5-iodo-2-methyl-4-(2-(phenylmethyl)benzoyl)-1H-pyrrole-3-carboxylate;
Methyl 2-Methyl-4-(2-(phenylmethyl)benzoyl)-1H-pyrrole-3-carboxylate;
Methyl 4-(2-(phenylmethyl)benzoyl)-1H-pyrrole-3-carboxylate;
Methyl 3-(Methoxycarbonyl)-5-methyl-4-(2-(phenylmethyl)benzoyl)-1H-pyrrole-2-acetate;
Methyl 5-Ethyl-2-methyl-4-(2-(phenylmethyl)benzoyl)-1H-pyrrole-3-carboxylate;

Methyl 2,5-Dimethyl-4-((1-(phenylmethyl)-1H-pyrrol-2-yl)carbonyl)-1H-pyrrole-3-carboxylate;
Ethyl 4-(2-Chlorobenzoyl)-1-((methoxycarbonyl)amino)- 2,5-dimethyl-1H-pyrrole-3-carboxylate;
Methyl 4-(2-Chlorobenzoyl)-1-(((1,1-dimethylethoxy) carbonyl)amino)-2,5-dimethyl-1H-pyrrole-3-carboxylate;
Methyl 1-Amino-4-(2-chlorobenzoyl)-2,5-dimethyl-1H-pyrrole-3-carboxylate;
Methyl 2,5-Dimethyl-4-((2-methyl-1-piperidinyl) carbonyl)-1H-pyrrole-3-carboxylate;
Methyl 2,5-Dimethyl-4-((1-(phenylmethyl)-1H-imidazol-2-yl)carbonyl)1H-pyrrole-3-carboxylate;
Methyl 5-Ethyl-2-methyl-4-((1-(phenylmethyl)-1H-pyrrol-2-yl)carbonyl)-1H-pyrrole-3-carboxylate;
Methyl 2-methyl-4-(2-phenylmethyl)benzoyl)-5-trifluoromethyl-1H-pyrrole-3-carboxylate;
Methyl 4-(2-((4-methoxyphenyl)methyl)benzoyl)-2,5-dimethyl-1H-pyrrole-3-carboxylate;
Methyl 4-((9H-fluoren-1-yl)carbonyl)-2,5-dimethyl-1H-pyrrole-3-carboxylate;
Meithyl 2-methyl-4,5-di (2-pyridinyl)-1H-pyrrole-3-carboxylate;
(2-Chlorophenyl) (2,5-Dimethyl-4-phenyl-1H-pyrrol-3-yl)methanone;
Methyl 4-(4-(1H-imidazol-1-yl)phenyl)-2,5-dimethyl-1H-pyrrole-3-carboxylate;
Methyl 2,5-dimethyl-4-(2-pyridinyl)-1H-pyrrole-3-carboxylate;
Methyl 2,5-Dimethyl-4-(2-(phenylmethyl)benzoyl)-1H-pyrrole-3-carboxylate;
Methyl 2,5-Dimethyl-4-(2-phenylbenzoyl)-1H-pyrrole -3-carboxylate;
(E)Methyl 5-(2-cyanoethenyl)-2-methyl-4-(2-(phenyl methyl)benzoyl)-1H-pyrrole-3-carboxylate;
Methyl 2,5-dimethyl-4-((2-phenylmethyl)phenyl)thio) -1H-pyrrole-3-carboxylate;
Methyl 4-(((2-(3,4-dimethoxyphenyl)ethyl)amino) carbonyl)-2,5-dimethyl-1H-pyrrole-3-carboxylate;
Methyl 4-(((1H-benzimidazol-2-yl)amino)carbonyl)-2,5-dimethyl-1H-pyrrole-3-carboxylate;
Methyl 2,5-dimethyl-4-(((2-(4-morpholinyl)ethyl)amino) carbonyl)-1H-pyrrole-3-carboxylate;
Methyl 2,5-dimethyl-4-(oxo((phenylmethyl)amino) acetyl)-1H-pyrrole-3-carboxylate;
Methyl 4-(((2-benzothiazolyl)amino)carbonyl)-2,5-dimethyl-1H-pyrrole-3-carboxylate;
Methyl 4-(3,4-dihydro-6,7-dimethoxy-1-isoquinolinyl)-2,5-dimethyl-1H-pyrrole-3-carboxylate;
Methyl 4-(5-anthracenyl)-2-formyl-5-methyl-1H-pyrrole-3-carboxylate;
Methyl 4-(5-anthracenyl)-5-formyl-2-methyl-1H-pyrrole-3-carboxylate;
Methyl 4-(11H-dibenz(b,e)azepin-6-yl)-5-methyl-1H- pyrrole-4-carboxylate;
Methyl 2,5-dimethyl-4-(2,3,4,5-tetrahydro-1H-benzazepin-2-yl)-1H-pyrrole-3-carboxylate;
Methyl 2,5-dimethyl-4-(4,5-dihydro-3H-benzazepin-2-yl)-1H-pyrrole-3-carboxylate;
Methyl 4-(3,4-dihydro-1-isoquinolinyl))-2,5-dimethyl-1H-pyrrole-3-carboxylate;
(±) Methyl 4-(4,5-dihydro-5-phenyl-2-oxazolyl)-2,5-dimethyl-1H-pyrrole-3-carboxylate;
(S) and (R) Methyl 4-(4,5-dihydro-4-phenyl-2-oxazolyl)-2,5-dimethyl-1H-pyrrole-3-carboxylate;
Methyl 2,5-dimethyl-4-(2-quinolinyl)-1H-pyrrole-3-carboxylate;
Methyl 2,5-dimethyl-4-(2-phenyl-1H-imidazol-4-yl)-1H-pyrrole-3-carboxylate;
Methyl 4-((imino(phenylamino)methyl)amino) carbonyl)-2,5-dimethyl-1H-pyrrole-3-carboxylate;
1-Methylethyl 2,5-Dimethyl-4-(2-(phenylmethyl) benzoyl)-1H-pyrrole-3-carboxylate;
2-Methoxyethyl 2,5-Dimethyl-4-(2-(phenylmethyl) benzoyl)-1H-pyrrole-3-carboxylate;
Cyclopropylmethyl 2,5-Dimethyl-4-(2-(phenylmethyl) benzoyl)-1H-pyrrole-3-carboxylate;
2-Chloroethyl 2,5-Dimethyl-4-(2-(phenylmethyl)benzoyl) -1H-pyrrole-3-carboxylate;
2,2,2-Trifluoroethyl 2,5-Dimethyl-4-(2-(phenylmethyl) benzoyl-1H-pyrrole-3-carboxylate;
S-Methyl 2,5-Dimethyl-4-(2-(phenylmethyl)benzoyl)-1H-pyrrole-3-carbothioate;
Cyclopentyl 4-(2-Chlorobenzoyl)-2,5-dimethyl-1H-pyrrole-3-carboxylate;
2-(Dimethylamino)ethyl 4-(2-Chlorobenzoyl)-2,5-dimethyl-1H-pyrrole-3-carboxylate;
(2-Methoxy-2-oxoethyl) 2,5-dimethyl-4-(2-(phenyl methyl)benzoyl)-1H-pyrrole-3-carboxylate;
Phenylmethyl 4-(methoxycarbonyl)-2,5-dimethyl-α -oxo-1H-pyrrole-3-acetate;
Methyl 4-(2-((4-Aminophenyl)methyl)benzoyl)-2,5-dimethyl-1H-pyrrole-3-carboxylate;
Methyl 4-(2-(3-Aminobenzoyl)benzoyl)-2,5-dimethyl-1H-pyrrole-3-carboxylate;
Methyl 4-(2-(4-Aminophenoxy)benzoyl)-2,5-dimethyl-1H-pyrrole-3-carboxylate;
Methyl 2-Amino-4-(2-chlorobenzoyl)-5-Methyl-1H-pyrrole-3-carboxylate;
Methyl 2-amino-5-methyl-4-(2-(phenylmethyl)benzoyl)-1H-pyrrole-3-carboxylate;
Methyl 5-amino-2-methyl-4-(2-(phenylmethyl)benzoyl-1H-pyrrole-3-carboxylate;
2,5-Dimethyl-4-(2-(phenylmethyl)benzoyl)-1H-pyrrole- 3-carboxylic acid;
5-Ethyl-2-methyl-4-(2-(phenylmethyl)benzoyl)-1H-pyrrole-3-carboxylic acid;
Carboxymethyl 2,5-dimethyl-4-(2-(phenylmethyl) benzoyl))-1H-pyrrole-3-carboxylate;
4-(2-(Phenylmethyl)benzoyl)-3-methoxycarbonyl-5-methyl-1H-pyrrole-2-acetic acid;
E-Methyl 5-(2-carboxyethenyl)-2-methyl-4-(2-(phenylmethyl)benzoyl)-1H-pyrrole-3-carboxylate;
4-(2-(Methoxycarbonyl)benzoyl)-2,5-dimethyl-1H-pyrrole-3-carboxylic acid;
Methyl 4-(2-Benzoylbenzoyl)-2,5-dimethyl-1H-pyrrole-3-carboxylate;

Methyl 2,5-Dimethyl-4-(2-phenoxybenzoyl)-1H-pyrrole-3-carboxylate;
Methyl 5-Methyl-4-(2-(phenylmethyl)benzoyl)-1H-pyrrole-3-carboxylate;
Methyl 2,5-dimethyl-4-(1-oxo-4-phenylbutyl)-1H-pyrrole-3-carboxylate;
Methyl 5-((Acetyloxy)methyl)-2-methyl-4-(2-(phenylmethyl)benzoyl)-1H-pyrrole-3-carboxylate;
Methyl 2-((Acetyloxy)methyl)-5-methyl-4-(2-(phenylmethyl)benzoyl)-1H-pyrrole-3-carboxylate;
Methyl 5-(Hydroxymethyl)-2-methyl-4-(2-(phenyl methyl)benzoyl)-1H-pyrrole-3-carboxylate;
Methyl 2-(Hydroxymethyl)-5-methyl-4-(2-(phenyl methyl)benzoyl)-1H-pyrrole-3-carboxylate;
Methyl 4-(5-(Acetylamino)-2-chlorobenzoyl)-2,5-dimethyl-1H-pyrrole-3-carboxylate;
Methyl 4-(2-((Ethylamino)carbonyl)benzoyl)-2,5-dimethyl-1H-pyrrole-3-carboxylate;
N-Ethyl 4-(2-(methoxycarbonyl)benzoyl)-2,5-dimethyl -1H-pyrrole-3-carboxamide;
Methyl 4-(2-Chloro-5-(methylthio)benzoyl)-2,5-dimethyl-1H-pyrrole-3-carboxylate;
(Z)- and (E)-Methyl 5-((hydroxyimino)methyl)-2-methyl-4-(2-(pheynlmethyl)benzoyl)-1H-pyrrole-3-carboxylate;
(E) Methyl 5-(((aminocarbonyl)hydrazono)methyl)-2-methyl-4-(2-(phenylmethyl)benzoyl)-1H-pyrrole-3-carboxylate;
Methyl 2,5-dimethyl-4-(((phenylmethylene)hydrazino carbonyl)-1H-pyrrole-3-carboxylate;
Methyl 2,5-Dimethyl-4-(2-(phenylsulphinyl)benzoyl)-1H-pyrrole-3-carboxylate;
Methyl 2,5-Dimethyl-4-(2-(phenylsulphonyl)benzoyl)-1H-pyrrole-3-carboxylate;
Methyl 4-(2-Chlorobenzoyl)-2-methyl-5-(methyl sulphinyl)-1H-pyrrole-3-carboxylate;
Methyl 4-(2-Chlorobenzoyl)-2-methyl-5-(methyl sulphonyl)-1H-pyrrole-3-carboxylate;
(2-Chlorophenyl) (2,5-Dimethyl-4-(methylsulphinyl)-1H- pyrrole-3-yl)methanone
(2-Chlorophenyl) (2,5-Dimethyl-4-(methylsulphonyl)-1H-pyrrol-3-yl)methanone
Methyl 2,5-Dimethyl-4-(2-((phenylmethyl)sulphinyl) benzoyl)-1H-pyrrole-3-carboxylate;
Methyl 2,5-Dimethyl-4-(2-((phenylmethyl)sulphonyl) benzoyl)-1H-pyrrole-3-carboxylate;
Methyl 4-(2-Chloro-5-(methylsulphinyl)benzoyl)-2,5-dimethyl-1H-pyrrole-3-carboxylate;
Methyl 4-(2-Chloro-5-(methylsulphonyl)benzoyl)-2,5-dimethyl-1H-pyrrole-3-carboxylate;
Methyl 5-Ethyl-2-methyl-4-(2-((phenylmethyl) sulfinyl)benzoyl)-1H-pyrrole-3-carboxylate;
Methyl 2,5-dimethyl-4-((2-(phenylmethyl)phenyl) sulphinyl)-1H-pyrrole-3-carboxylate;
or a pharmaceutically acceptable derivative of any one thereof.

9. A pharmaceutical composition comprising less than 80% by weight of a compound according to any one of the preceding claims in admixture with a pharmaceutically acceptable adjuvant, diluent or carrier.

10. A process for the preparation of a compound of formula I, as defined in Claim 1, which comprises
a) producing a compound of formula I in which $R_3$ or G2 includes a carbonyl group adjacent to the pyrrole ring by reacting a compound of formula IIa or IIb,

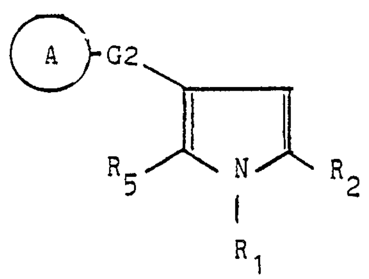

IIa

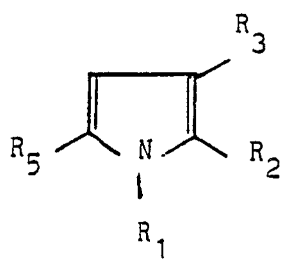

IIb

with a compound of formula IIIa or IIIb respectively,

$R_{3a}$-CO-$L_a$ IIIa

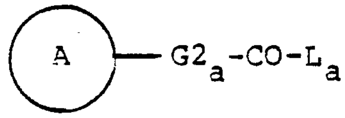

IIIb

in which $R_{3a}$CO- and -$G2_a$CO- represent groups $R_3$ and G2 respectively and $L_a$ is a leaving group, or b) producing a compound of formula I which is substituted by D-G3c- or alkylene $C_{1-6}$ optionally substituted by -$COOR_{41}$ or -$NR_{43}R_{45}$ in which -G3c-represents -NH-, -S-, -$CH_2$NH- or alkylene $C_{1-6}$, by reacting a compound of formula I substituted by a leaving group with a compound of formula IV,

$Ar_c$-H IV

in which $Ar_c$- represents D-G3c- or alkylene $C_{1-5}$ optionally substituted by -$COOR_{43}$ or -$NR_{43}R_{45}$, or

c) producing a compound of formula I which contains a substituent halogen, -CHO, -CN, -$NO_2$, -$S(O)_qR_{21}$, -$CONH_2$, -$CH_2NR_{32}R_{33}$ or -N=N-$R_{24}$, by reacting a corresponding compound containing a hydrogen atom with an appropriate electrophile and, where necessary, appropriate transformation of the product, or

d) producing a compound of formula I in which one or both of $R_2$ and $R_5$ represents hydrogen, by reacting a compound of formula V

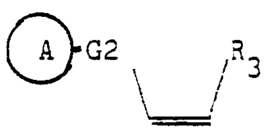

V

with a compound of formula VI

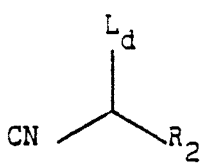

VI

in which $L_d$ represents a leaving group, or

e) reacting a compound of formula VII

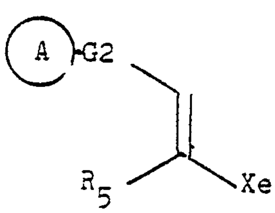

VII

with a compound of formula VIII

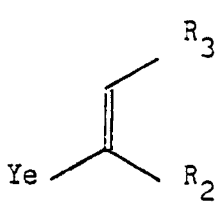

VIII

in which one of $X_e$ and $Y_e$ represents a leaving group, and the other represents $-NHR_1$, and where necessary, working up the reaction product, or

f) reacting a compound of formula IX,

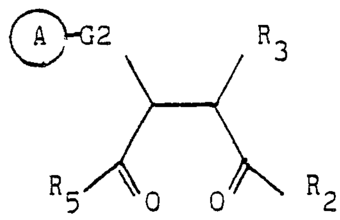

IX

with a compound of formula X,

$R_1-NH_2$ X

or

g) removal of a protecting group from a compound of formula I which bears a protecting group, or

h) producing a compound of formula I in which G2 includes a group $-(CO)_y-$ adjacent to the pyrrole ring, by reacting a compound of formula XI,

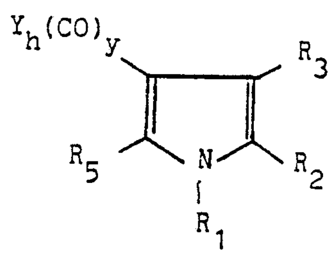

XI

with a compound of formula XII

A $G2_h-X_h$ XII

in which $G2_h$ is a group which, in combination with the group $-(CO)_y-$ forms G2 and one of $X_h$ and $Y_h$ is a leaving group and the other is hydrogen, and, if necessary, working up the reaction product, or

i) producing a compound of formula I in which A is a 5-or 6-membered ring or a bicyclic or tricyclic fusing ring system by appropriate treatment of a corresponding compound in which

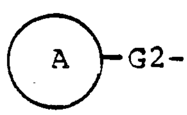

contains groups cyclisable to form such a ring or fused ring system respectively, or

j) producing a compound of formula I in which G2 represents $-NHC(=NH)NH-(CO)_y-$ by reacting a compound of formula XIII,

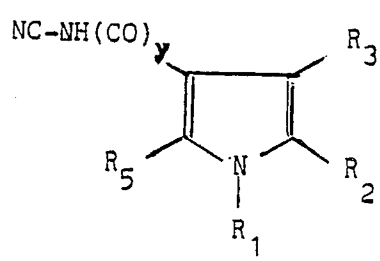

XIII

with a compound of formula XIV,

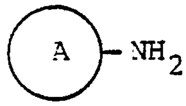

XIV

or

k) producing a compound of formula I containing a group $-COOR_k$ or $-COSR_k$ in which $R_k$ represents cycloalkyl $C_{3-8}$ or alkyl $C_{1-6}$ optionally substituted by halogen, $-NR_{32}R_{33}$ or $-COOR_{35}$, by esterification or thioesterification respectively of the corresponding compound containing a group -COOH, or

l) producing a compound of formula I containing a substituent $-NH_2$, by reduction of the corresponding compound containing a substituent $-NO_2$, or

m) producing a compound of formula I containing a group -COOH by hydrolysis of a corresponding ester, or

n) deamination of a compound of formula I containing a substituent $-NH_2$, or

o) producing a compound of formula I containing an alkyl $C_{1-6}$ group substituted by a group $-OOCR_{26}$, by alkanoyloxylation of the alkyl $C_{1-6}$ group, or

p) producing a compound of formula I containing a hydroxy group, or by reacting the corresponding compound containing a group $-OOCR_{26}$ with a hydroxylic base,

q) producing a compound of formula I containing an amido group, by acylation of the corresponding amine or by reacting a corresponding carboxylic acid or carboxylic acid derivative with an appropriate amine, or

r) producing a compound of formula I containing a group $-SR_r$ in which $R_r$ represents $C_{1-6}$ by diazotisation of the corresponding compound containing a group $-NH_2$ and subsequent displacement, or

s) producing a compound of formula I containing an aliphatic -CH=N- group by condensation of the appropriate aldehyde and amine, or

t) producing a compound of formula I containing a group -SO- or $-SO_2-$, by oxidation of the corresponding compound containing a group -S- or -SO-,

and, where necessary or desired thereafter, converting the compound of formula I so obtained to a pharmaceutically acceptable derivative thereof, or vice versa.

Claims for the following contracting State: ES

1. A process for the preparation of a compound of formula I,

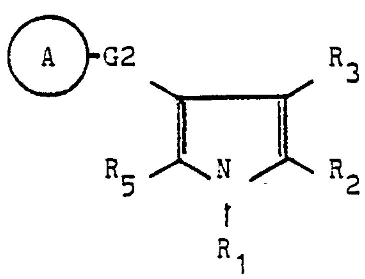

I

wherein

$R_1$ represents $-R_{11}$, $-NHR_{11}$ or $-NHCOOR_{11}$ in which $R_{11}$ is hydrogen or alkyl $C_{1-6}$,

$R_2$ and $R_5$, which may be the same or different, represent hydrogen, $-R_{21}$, -OH, $-OR_{21}$, $-NR_{22}R_{23}$, halogen, -CN, $-NO_2$, $-S(O)_qR_{21}$, $-N=NR_{24}$, $-COOR_{22}$, $-CH=R_{25}$, phenyl or pyridyl,

in which $R_{21}$ is alkyl or alkenyl $C_{1-6}$ optionally interrupted by O or S and optionally substituted by -OH, halogen, -CN, $-NO_2$, $-NR_{22}R_{23}$, $-COOR_{22}$, phenyl or $-OOCR_{26}$,

$R_{22}$ and $R_{23}$, which may be the same or different, are hydrogen or alkyl $C_{1-6}$,

$R_{24}$ is phenyl optionally substituted by $-NO_2$,

$R_{25}$ is =O, =CHCN, =NOH or $=NNHCONH_2$,

$R_{26}$ is alkyl $C_{1-6}$, and
q is 0, 1 or 2,
G2 represents a chain $-(CH_2)_z(W)_y-$, in which,
W represents C = O, $S(O)_q$ or $C = NR_{40}$,
in which $R_{40}$ is hydrogen, -OH, cycloalkyl $C_{3-8}$ or alkyl $C_{1-6}$ optionally substituted by phenyl,
y is 0, 1 or, provided W is C = O, 2,
z is 0, 1, 2 or 3, and
up to two of the methylene segments in the chain $(CH_2)_z$ are optionally replaced by -NH- and one segment is optionally replaced by -O- or $-(C = NR_{40})-$, and the chain is optionally unsaturated and optionally substituted by alkyl $C_{1-6}$ or alkoxy $C_{1-6}$,
A is a 5- or 6-membered ring or a bicyclic or tricyclic fused ring system, A being optionally substituted by one or more of halogen, -OH, $-NO_2$, $-NR_{43}R_{45}$, $-COOR_{43}$, $-S(O)_qR_{44}$, $-CONR_{43}R_{45}$, $-NHCOR_{45}$, alkyl, alkylene or alkoxy $C_{1-6}$ optionally substituted by halogen, phenyl, $-COOR_{43}$ or $-NR_{43}R_{45}$, or a group D-G3-, in which
$R_{43}$ and $R_{45}$, which may be the same or different, are hydrogen or alkyl $C_{1-6}$,
$R_{44}$ is alkyl $C_{1-6}$,
G3 is a single bond, -NH-, -O-, -CO-, $-OCH_2-$, -O-CO-, $-S(O)_q-$, $(CH_2)_p-$, $-CH_2S(O)_q-$, -CO-S-, $-SO_2-O-$ or $CH_2NH-$, in which p is 1,2 or 3, and
D represents a 5- or 6-membered ring optionally substituted by -OH, halogen, $-NO_2$, $-NR_{43}R_{45}$, alkyl $C_{1-6}$, alkoxy $C_{1-6}$ or $-SCH_2C_6H_5$, and
$R_3$ represents hydrogen, $-S(O)_qR_{31}$, $-NO_2$, -CN, -halogen, $-CH_2NR_{32}R_{33}$, $-(CO)_rR_{34}$ or an aryl group, in which
$R_{31}$ is alkyl $C_{1-6}$,
$R_{32}$ and $R_{33}$, which may be the same or different, are hydrogen, alkyl $C_{1-6}$ optionally substituted by phenyl, or phenyl optionally substituted by halogen or trihalomethyl,
r is 1 or 2,
$R_{34}$ is hydrogen, $-NR_{32}R_{33}$, alkyl $C_{1-6}$, -OH, $-OR_{35}$ or $-SR_{35}$,
$R_{35}$ is cycloalkyl $C_{3-8}$ or alkyl $C_{1-6}$ optionally substituted by alkoxy $C_{1-6}$, halogen, -OH, cycloalkyl $C_{3-8}$, $-NR_{32}R_{33}$ or $-COOR_{36}$, and
$R_{36}$ is hydrogen or alkyl $C_{1-6}$, or $R_3$ and $R_2$ together form a chain -N = CH-NH-CO-,
provided that
a) when $R_3$ represents hydrogen, then A is substituted by D-G3- where, if A is a 5-membered heterocyclic ring, D-G3- is other than phenyl, benzyl or phenyl substituted by chlorine, or, if A is phenyl or naphthyl and D is phenyl, G3 is other than $-CH_2-O-$, -S-, -O-or -NH-;
b) when $R_2$ and $R_5$ both represent hydrogen, then A is substituted by D-G3-;
c) when $R_1$ represents alkyl $C_{1-6}$ or $-NHCOOR_{11}$, and $R_2$ and $R_5$ are both alkyl $C_{1-6}$, and $R_3$ is $-COOR_{35}$ or $-NO_2$, then A is other than unsubstituted phenyl or phenyl substituted by fluorine;
d) when $R_2$ and $R_3$ both represent halogen, and A is phenyl, then A is substituted by D-G3-;
e) when one or both of $R_2$ and $R_5$ represents phenyl, and $R_3$ represents $-COOR_{35}$, $-COCH_3$, phenyl or $-NO_2$, then A is other than unsubstituted phenyl, isoxazolyl substituted by alkyl $C_{1-6}$ or phenyl, or a group

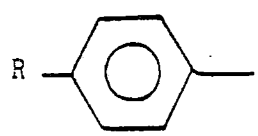

where R is halogen, alkyl $C_{1-6}$ or alkoxy $C_{1-6}$
f) when $R_5$ represents hydrogen, and $R_2$ represents alkyl $C_{1-6}$, and $R_3$ represents $-COOR_{35}$, and A is a 5- or 6-membered ring, then A is substituted by D-G3-; and
g) when $R_2$ and $R_5$ are both selected from hydrogen, $-COOR_{22}$ and alkyl $C_{1-6}$ optionally substituted by halogen, -OH, $-NR_{22}R_{23}$ or $-OOCR_{26}$ and optionally interrupted by O or S, and $R_3$ is selected from $-NO_2$, halogen, -CN, $-COR_{34}$ or $-CH_2NR_{32}R_{34}$, where $R_{34}$ is hydrogen, $-NR_{32}R_{34}$, alkyl $C_{1-6}$, -OH or $-OR_{35}$ where $R_{35}$ is alkyl $C_{1-6}$ optionally substituted by an aryl group, and $R_1$ represents hydrogen or alkyl $C_{1-6}$, and G2 represents $-(CH_2)_z(W)_y-$ in which z represents 0, 1 or 2 and none of the methylene segments are replaced by -NH-,-O- or $-(C = NR_{40})-$ and the chain is neither unsaturated not substituted by alkoxy $C_{1-6}$, then A is other than phenyl, naphthyl or benzofurazanyl optionally substituted only by halogen, -OH, $-NR_{43}R_{45}$, $-NO_2$, -COOH, alkyl or alkoxy $C_{1-6}$ optionally substituted by halogen, or D-G3- where G3 is

-$SCH_2$-, -$OCH_2$- or -O-CO- and D is phenyl optionally substituted by -OH, halogen or alkyl or alkoxy $C_{1-6}$; or a pharmaceutically acceptable derivative thereof, which process comprises

a) producing a compound of formula I in which $R_3$ or G2 includes a carbonyl group adjacent to the pyrrole ring by reacting a compound of formula IIa or IIb,

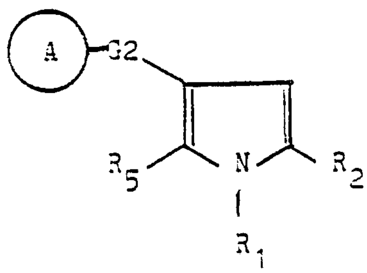

IIa

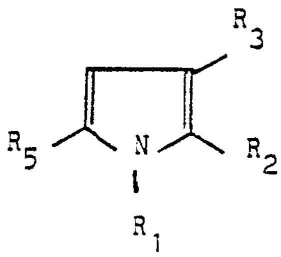

IIb

with a compound of formula IIIa or IIIb respectively,

$R_{3a}$-CO-$L_a$ IIIa

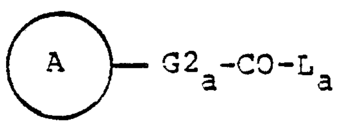

IIIb

in which $R_{3a}$CO- and $G2_a$CO- represent groups $R_3$ and G2 respectively and $L_a$ is a leaving group, or

b) producing a compound of formula I which is substituted by D-G3c- or alkylene $C_{1-6}$ optionally substituted by -$COOR_{41}$ or -$NR_{43}R_{45}$ in which -G3c- represents -NH-, -S-, -$CH_2$NH- or alkylene $C_{1-6}$, by reacting a compound of formula I substituted by a leaving group with a compound of formula IV,

$Ar_c$-H IV

in which $Ar_c$- represents D-G3c- or alkylene $C_{1-6}$ optionally substituted by -$COOR_{43}$ or -$NR_{43}R_{45}$, or

c) producing a compound of formula I which contains a substituent halogen, -CHO, -CN, -$NO_2$, -$S(O)_qR_{21}$, -$CONH_2$, -$CH_2NR_{32}R_{33}$ or -N=N-$R_{24}$, by reacting a corresponding compound containing a hydrogen atom with an appropriate electrophile and, where necessary, appropriate transformation of the product, or

d) producing a compound of formula I in which one or both of $R_2$ and $R_5$ represents hydrogen, by reacting a compound of formula V

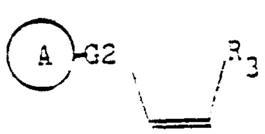

V

with a compound of formula VI

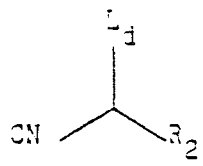

VI

in which $L_d$ represents a leaving group, or

e) reacting a compound of formula VII

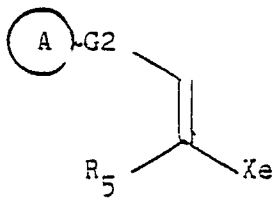

VII

with a compound of formula VIII

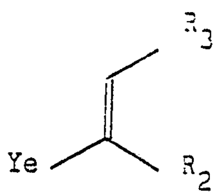

VIII

in which one of $X_e$ and $Y_e$ represents a leaving group, and the other represents $-NHR_1$, and where necessary, working up the reaction product, or

f) reacting a compound of formula IX,

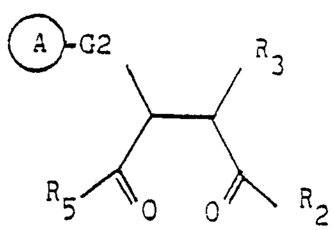

IX

with a compound of formula X,

$R_1-NH_2$ X

or

g) removal of a protecting group from a compound of formula I which bears a protecting group, or

h) producing a compound of formula I in which G2 includes a group $-(CO)_y-$ adjacent to the pyrrole ring, by reacting a compound of formula XI,

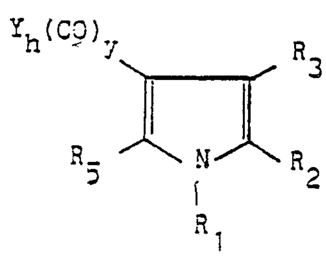

XI

with a compound of formula XII

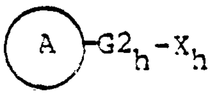

XII

in which $G2_h$ is a group which, in combination with the group $-(CO)_y-$ forms G2 and one of $X_h$ and $Y_h$ is a leaving group and the other is hydrogen, and, if necessary, working up the reaction product, or

i) producing a compound of formula I in which A is a 5-or 6-membered ring or a bicyclic or tricyclic fusing ring system by appropriate treatment of a corresponding compound in which

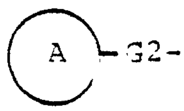

contains groups cyclisable to form such a ring or fused ring system respectively, or

j) producing a compound of formula I in which G2 represents $-NHC(=NH)NH-(CO)_y-$
by reacting a compound of formula XIII,

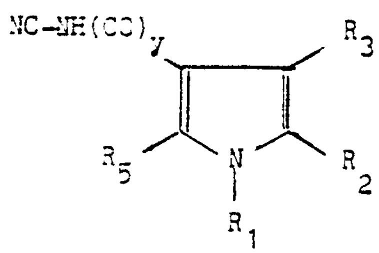

XIII

with a compound of formula XIV,

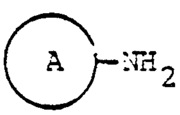

XIV

or

k) producing a compound of formula I containing a group $-COOR_k$ or $-COSR_k$ in which $R_k$ represents cycloalkyl $C_{3-3}$ or alkyl $C_{1-6}$ optionally substituted by halogen, $-NR_{32}R_{33}$ or $-COOR_{35}$,
by esterification or thioesterification respectively of the corresponding compound containing a group -COOH, or

l) producing a compound of formula I containing a substituent $-NH_2$,
by reduction of the corresponding compound containing a substituent $-NO_2$, or

m) producing a compound of formula I containing a group -COOH
by hydrolysis of a corresponding ester, or

n) deamination of a compound of formula I containing a substituent $-NH_2$, or

o) producing a compound of formula I containing an alkyl $C_{1-6}$ group substituted by a group $-OOCR_{26}$,
by alkanoyloxylation of the alkyl $C_{1-6}$ group, or

p) producing a compound of formula I containing a hydroxy group, or
by reacting the corresponding compound containing a group $-OOCR_{26}$ with a hydroxylic base,

q) producing a compound of formula I containing an amido group,
by acylation of the corresponding amine or by reacting a corresponding carboxylic acid or carboxylic acid derivative with an appropriate amine, or

r) producing a compound of formula I containing a group $-SR_r$ in which $R_r$ represents $C_{1-6}$ by diazotisation of the corresponding compound containing a group $-NH_2$ and subsequent displacement, or

s) producing a compound of formula I containing an aliphatic -CH = N- group by condensation of the appropriate aldehyde and amine, or

t) producing a compound of formula I containing a group -SO- or $-SO_2-$,

by oxidation of the corresponding compound containing a group -S- or -SO-,

and, where necessary or desired thereafter, converting the compound of formula I so obtained to a pharmaceutically acceptable derivative thereof, or vice versa.

2. A process according to Claim 1, wherein $R_3$ represents $-COOR_{35}$ or $-NO_2$.

3. A process according to Claim 1 or Claim 2, wherein $R_2$ and $R_5$ are selected from the group consisting of hydrogen, $-R_{21}$ and halogen.

4. A process according to any one of the preceding claims, wherein A is a 5- or 6-membered ring.

5. A process according to Claim 4, wherein A is substituted by D-G3-.

6. A process according to any one of the preceding claims, in which

$R_2$ and $R_5$, which may be the same or different, represent hydrogen, $-R_{21}$, or halogen,

$R_3$ represents $-NO_2$ or $-COOR_{35}$,

G2 represents C = O, -S- or a single bond, and

A represents phenyl substituted by a group D-G3-.

7. A process according to any one of the preceding claims, in which

$R_1$ represents hydrogen,

$R_2$ and $R_5$, which may be the same or different, represent hydrogen, $-R_{21}$, or halogen,

$R_3$ represents $-NO_2$ or $-COOR_{35}$,

G2 represent C = O, and

A represents phenyl substituted by a group D-G3-, in which G3 represents -NH- or $-CH_2-$.

8. A process according to Claim 1, in which the compound of formula I is

Methyl 2,5-Dimethyl-4-(2-((4-nitrophenyl)thio) benzoyl)-1H-pyrrole-3-carboxylate;
Methyl 2,5-Dimethyl-4-(2-((4-nitrophenyl)methyl) benzoyl)-1H-pyrrole-3-carboxylate;
Methyl 2,5-Dimethyl-4-(2-((2-nitrophenyl)methyl) benzoyl)-1H-pyrrole-3-carboxylate;
Methyl 2,5-Dimethyl-4-(2-(2-phenylethyl)benzoyl)-1H-pyrrole-3-carboxylate;
Methyl 2,5-Dimethyl-4-(2-(4-nitrophenoxy)benzoyl)-1H-pyrrole-3-carboxylate;
Methyl 2,5-Dimethyl-4-(2-(3-nitrobenzoyl)benzoyl)-1H-pyrrole-3-carboxylate;
Methyl 2,5-Dimethyl-4-(2-(((4-methylphenyl) sulphonyl)oxy)benzoyl)-1H-pyrrole-3-carboxylate;
Methyl 4-(2-Chlorobenzoyl)-2-methyl-5-(methylthio)-1H-pyrrole-3-carboxylate;
Methyl 2,5-Dimethyl-4-(2-(4-pyridinylamino)benzoyl) -1H-pyrrole-3-carboxylate;
Methyl 4-(2-(Cyclohexylmethyl)benzoyl)-2,5-dimethyl -1H-pyrrole-3-carboxylate;
Methyl 4-(2-Chlorobenzoyl)-2-methyl-5-phenyl-1H-pyrrole-3-carboxylate;
Methyl 4-(2-Chlorobenzoyl)-2,5-dimethyl-α-oxo-1H-pyrrole-3-acetate;
(2-Chlorophenyl) (2,5-Dimethyl-4-(5-methyl-1,3,4-oxadiazol-2-yl)-1H-pyrrol-3-yl)methanone;
Methyl 4-(2-(2-(Dimethylamino)ethoxy)benzoyl)-2,5-dimethyl-1H-pyrrole-3-carboxylate;
Methyl 2,5-Dimethyl-4-(2-((2-((phenylmethyl)thio) benzoyl)thio)benzoyl)-1H-pyrrole-3-carboxylate;
Methyl 4-(2-(Methoxycarbonyl)benzoyl)-2,5-dimethyl-1H-pyrrole-3-carboxylate;
Methyl 3-(2-Chlorobenzoyl)-4-(methoxycarbonyl)-5-methyl-1H-pyrrole-2-propanoate;
Methyl 4-(2-Chlorobenzoyl)-5-methyl-2-(2-nitroethyl) -1H-pyrrole-3-carboxylate;
Methyl 2,5-Dimethyl-4-(3-pyridinylcarbonyl)-1H-pyrrole-3-carboxylate;
Methyl 2,5-Dimethyl-4-(4-pyridinylcarbonyl)-1H-pyrrole-3-carboxylate;
5-(2-Chlorobenzoyl)-3,4-dihydro-6-methyl-7H- pyrrolo(2,3-d)pyrimidin-4-one;
(E)Methyl 4-(3-methoxy-1-oxo-4-phenyl-2-butenyl)-2,5-dimethyl-1H-pyrrole-3-carboxylate;
(2,5-Dimethyl-1H-pyrrol-3-yl) (2-(phenylmethyl) phenyl)methanone;
Methyl 2,5-Dimethyl-4-(2-(phenylthio)benzoyl)-1H-pyrrole-3-carboxylate;
Methyl 2,5-Dimethyl-4-(2-(phenylamino)benzoyl)-1H-pyrrole-3-carboxylate;
Methyl 2,5-Dimethyl-4-(2-(2-pyridinylamino)benzoyl) -1H-pyrrole-3-carboxylate;
Methyl 5-Ethyl-2-methyl-4-(2-(phenylamino)benzoyl)-1H-pyrrole-3-carboxylate;
Methyl 2,5-dimethyl-4-(2-(cyclohexylamino)benzoyl)-1H-pyrrole-3-carboxylate;
Methyl 2,5-dimethyl-4-(2-((phenylmethyl)amino)benzoyl) -1H-pyrrole-3-carboxylate;
Methyl 2,5-dimethyl-4-(2-((phenylmethyl)amino)phenyl) ((phenylmethyl)imino)methyl)-1H-pyrrole-3-carboxylate;
Methyl 4-((2-(cyclohexylamino)phenyl) (cyclohexyl imino)methyl-2,5-dimethyl-1H-pyrrole-3-carboxylate;
(E)-Methyl 2,5-dimethyl-4-(2-(2-phenylethenyl) benzoyl)-1H-pyrrole-3-carboxylate;
E-3-(2-((4-(Methoxycarbonyl)-2,5-dimethyl-1H-pyrrol-3-yl)carbonyl)phenyl)-2-propenoic acid;
Methyl 5-(2-(methoxycarbonyl)ethenyl)-2-methyl-4-(2-(phenylmethyl)benzoyl)-1H-pyrrole-3-carboxylate;

Methyl 2-methyl-5-phenyl-4-(2-(phenylmethyl)benzoyl) -1H-pyrrole-3-carboxylate;
Methyl 2-methyl-5-(phenylmethyl)-4-(2-(phenylmethyl) benzoyl)-1H-pyrrole-3-carboxylate;
Methyl 2-Bromo-4-(2-chlorobenzoyl)-5-methyl-1H-pyrrole-3-carboxylate;
(4-Bromo-2,5-dimethyl-1H-pyrrol-3-yl) (2-(phenylmethyl) phenyl)methanone;
Methyl 2-Bromo-5-methyl-4-(2-phenylmethyl)benzoyl)-1H-pyrrole-3-carboxylate;
Methyl 5-Bromo-2-methyl-4-(2-phenylmethyl)benzoyl)-1H-pyrrole-3-carboxylate;
Methyl 4-(2-Chlorobenzoyl)-2-cyano-5-methyl-1H-pyrrole-3-carboxylate;
2,5-Dimethyl-4-(2-(phenylmethyl)benzoyl)-1H-pyrrole-3-carbonitrile;
Methyl 2-Cyano-5-methyl-4-(2-(phenylmethyl)benzoyl)-1H-pyrrole-3-carboxylate;
Methyl 5-Cyano-2-methyl-4-(2-(phenylmethyl)benzoyl)-1H-pyrrole-3-carboxylate;
2,5-Dimethyl-4-(2-(phenylmethyl)benzoyl)-1H-pyrrole-3-carboxamide;
Methyl 4-(2-Chlorobenzoyl)-2-formyl-5-methyl-1H- pyrrole-3-carboxylate;
Methyl 5-Formyl-2-methyl-4-(2-(phenylmethyl)benzoyl)-1H-pyrrole-3-carboxylate;
Methyl 2-Formyl-5-methyl-4-(2-(phenylmethyl)benzoyl)-1H-pyrrole-3-carboxylate;
2,5-Dimethyl-4-(2-phenylmethyl)benzoyl)-1H-pyrrole-3-carboxaldehyde;
Methyl 4-(2-Chlorobenzoyl)-5-methyl-2-nitro-1H-pyrrole-3-carboxylate;
(2,5-Dimethyl-4-nitro-1H-pyrrol-3-yl) (2-(phenylmethyl)phenyl)methanone;
Methyl 4-(2-Chlorobenzoyl)-5-methyl-2-((4-nitrophenyl) azo)-1H-pyrrole-3-carboxylate;
(2-Chlorophenyl) (2,5-Dimethyl-4-(methylthio)-1H-pyrrol-3-yl)methanone;
Methyl 2-methyl-5-((4-nitrophenyl)azo)-4-(2-(phenylmethyl)benzoyl)-1H-pyrrole-3-carboxylate;
Methyl 5-methyl-2-((4-nitrophenyl)azo)-4-(2-(phenylmethyl)benzoyl)-1H-pyrrole-3-carboxylate;
(4-((Dimethylamino)methyl)-2,5-dimethyl-1H-pyrrol-3-yl) ((2-phenylmethyl)phenyl)methanone;
Methyl 5-iodo-2-methyl-4-(2-(phenylmethyl)benzoyl)-1H-pyrrole-3-carboxylate;
Methyl 2-Methyl-4-(2-(phenylmethyl)benzoyl)-1H-pyrrole-3-carboxylate;
Methyl 4-(2-(phenylmethyl)benzoyl)-1H-pyrrole-3-carboxylate;
Methyl 3-(Methoxycarbonyl)-5-methyl-4-(2-(phenylmethyl)benzoyl)-1H-pyrrole-2-acetate;
Methyl 5-Ethyl-2-methyl-4-(2-(phenylmethyl)benzoyl)-1H-pyrrole-3-carboxylate;
Methyl 2,5-Dimethyl-4-((1-(phenylmethyl)-1H-pyrrol-2-yl)carbonyl)-1H-pyrrole-3-carboxylate;
Ethyl 4-(2-Chlorobenzoyl)-1-((methoxycarbonyl)amino)-2,5-dimethyl-1H-pyrrole-3-carboxylate;
Methyl 4-(2-Chlorobenzoyl)-1-(((1,1-dimethylethoxy) carbonyl)amino)-2,5-dimethyl-1H-pyrrole-3-carboxylate;
Methyl 1-Amino-4-(2-chlorobenzoyl)-2,5-dimethyl-1H-pyrrole-3-carboxylate;
Methyl 2,5-Dimethyl-4-((2-methyl-1-piperidinyl) carbonyl)-1H-pyrrole-3-carboxylate;
Methyl 2,5-Dimethyl-4-((1-(phenylmethyl)-1H-imidazol-2-yl)carbonyl)1H-pyrrole-3-carboxylate;
Methyl 5-Ethyl-2-methyl-4-((1-(phenylmethyl)-1H-pyrrol-2-yl)carbonyl)-1H-pyrrole-3-carboxylate;
Methyl 2-methyl-4-(2-phenylmethyl)benzoyl)-5-trifluoromethyl-1H-pyrrole-3-carboxylate;
Methyl 4-(2-((4-methoxyphenyl)methyl)benzoyl)-2,5-dimethyl-1H-pyrrole-3-carboxylate;
Methyl 4-((9H-fluoren-1-yl)carbonyl)-2,5-dimethyl-1H- pyrrole-3-carboxylate;
Methyl 2-methyl-4,5-di (2-pyridinyl)-1H-pyrrole-3-carboxylate;
(2-Chlorophenyl) (2,5-Dimethyl-4-phenyl-1H-pyrrol-3-yl)methanone;
Methyl 4-(4-(1H-imidazol-1-yl)phenyl)-2,5-dimethyl-1H-pyrrole-3-carboxylate;
Methyl 2,5-dimethyl-4-(2-pyridinyl)-1H-pyrrole-3-carboxylate;
Methyl 2,5-Dimethyl-4-(2-(phenylmethyl)benzoyl)-1H-pyrrole-3-carboxylate;
Methyl 2,5-Dimethyl-4-(2-phenylbenzoyl)-1H-pyrrole -3-carboxylate;
(E)Methyl 5-(2-cyanoethenyl)-2-methyl-4-(2-(phenyl methyl)benzoyl)-1H-pyrrole-3-carboxylate;
Methyl 2,5-dimethyl-4-((2-phenylmethyl)phenyl)thio) -1H-pyrrole-3-carboxylate;
Methyl 4-(((2-(3,4-dimethoxyphenyl)ethyl)amino) carbonyl)-2,5-dimethyl-1H-pyrrole-3-carboxylate;
Methyl 4-(((1H-benzimidazol-2-yl)amino)carbonyl)-2,5-dimethyl-1H-pyrrole-3-carboxylate;
Methyl 2,5-dimethyl-4-(((2-(4-morpholinyl)ethyl)amino) carbonyl)-1H-pyrrole-3-carboxylate;
Methyl 2,5-dimethyl-4-(oxo((phenylmethyl)amino) acetyl)-1H-pyrrole-3-carboxylate;
Methyl 4-(((2-benzothiazolyl)amino)carbonyl)-2,5-dimethyl-1H-pyrrole-3-carboxylate;
Methyl 4-(3,4-dihydro-6,7-dimethoxy-1-isoquinolinyl)-2,5-dimethyl-1H-pyrrole-3-carboxylate;
Methyl 4-(5-anthracenyl)-2-formyl-5-methyl-1H-pyrrole-3-carboxylate;
Methyl 4-(5-anthracenyl)-5-formyl-2-methyl-1H-pyrrole-3-carboxylate;
Methyl 4-(11H-dibenz(b.e)azepin-6-yl)-5-methyl-1H-pyrrole-3-carboxylate;
Methyl 2,5-dimethyl-4-(2,3,4,5-tetrahydro-1H-benzazepin-2-yl)-1H-pyrrole-3-carboxylate;
Methyl 2,5-dimethyl-4-(4,5-dihydro-3H-benzazepin-2-yl)-1H-pyrrole-3-carboxylate;
Methyl 4-(3,4-dihydro-1-isoquinolinyl))-2,5-dimethyl-1H-pyrrole-3-carboxylate;
(±) Methyl 4-(4,5-dihydro-5-phenyl-2-oxazolyl)-2,5-dimethyl-1H-pyrrole-3-carboxylate;

(S) and (R) Methyl 4-(4,5-dihydro-4-phenyl-2-oxazolyl)-2,5-dimethyl-1H-pyrrole-3-carboxylate;
Methyl 2,5-dimethyl-4-(2-quinolinyl)-1H-pyrrole-3-carboxylate;
Methyl 2,5-dimethyl-4-(2-phenyl-1H-imidazol-4-yl)-1H-pyrrole-3-carboxylate;
Methyl 4-((imino(phenylamino)methyl)amino) carbonyl)-2,5-dimethyl-1H-pyrrole-3-carboxylate;
1-Methylethyl 2,5-Dimethyl-4-(2-(phenylmethyl) benzoyl)-1H-pyrrole-3-carboxylate;
2-Methoxyethyl 2,5-Dimethyl-4-(2-(phenylmethyl) benzoyl)-1H-pyrrole-3-carboxylate;
Cyclopropylmethyl 2,5-Dimethyl-4-(2-(phenylmethyl) benzoyl)-1H-pyrrole-3-carboxylate;
2-Chloroethyl 2,5-Dimethyl-4-(2-(phenylmethyl)benzoyl) -1H-pyrrole-3-carboxylate;
2,2,2-Trifluoroethyl 2,5-Dimethyl-4-(2-(phenylmethyl) benzoyl-1H-pyrrole-3-carboxylate;
S-Methyl 2,5-Dimethyl-4-(2-(phenylmethyl)benzoyl)-1H-pyrrole-3-carbothioate;
Cyclopentyl 4-(2-Chlorobenzoyl)-2,5-dimethyl-1H-pyrrole-3-carboxylate;
2-(Dimethylamino)ethyl 4-(2-Chlorobenzoyl)-2,5-dimethyl-1H-pyrrole-3-carboxylate;
(2-Methoxy-2-oxoethyl) 2,5-dimethyl-4-(2-(phenyl methyl)benzoyl)-1H-pyrrole-3-carboxylate;
Phenylmethyl 4-(methoxycarbonyl)-2,5-dimethyl-α -oxo-1H-pyrrole-3-acetate;
Methyl 4-(2-((4-Aminophenyl)methyl)benzoyl)-2,5-dimethyl-1H-pyrrole-3-carboxylate;
Methyl 4-(2-(3-Aminobenzoyl)benzoyl)-2,5-dimethyl-1H-pyrrole-3-carboxylate;
Methyl 4-(2-(4-Aminophenoxy)benzoyl)-2,5-dimethyl-1H-pyrrole-3-carboxylate;
Methyl 2-Amino-4-(2-chlorobenzoyl)-5-Methyl-1H-pyrrole-3-carboxylate;
Methyl 2-amino-5-methyl-4-(2-(phenylmethyl)benzoyl)-1H-pyrrole-3-carboxylate;
Methyl 5-amino-2-methyl-4-(2-(phenylmethyl)benzoyl-1H-pyrrole-3-carboxylate;
2,5-Dimethyl-4-(2-(phenylmethyl)benzoyl)-1H-pyrrole3-carboxylic acid;
5-Ethyl-2-methyl-4-(2-(phenylmethyl)benzoyl)-1H-pyrrole-3-carboxylic acid;
Carboxymethyl 2,5-dimethyl-4-(2-(phenylmethyl) benzoyl))-1H-pyrrole-3-carboxylate;
4-(2-(Phenylmethyl)benzoyl)-3-methoxycarbonyl-5-methyl-1H-pyrrole-2-acetic acid;
E-Methyl 5-(2-carboxyethenyl)-2-methyl-4-(2-(phenylmethyl)benzoyl)-1H-pyrrole-3-carboxylate;
4-(2-(Methoxycarbonyl)benzoyl)-2,5-dimethyl-1H-pyrrole-3-carboxylic acid;
Methyl 4-(2-Benzoylbenzoyl)-2,5-dimethyl-1H-pyrrole-3-carboxylate;
Methyl 2,5-Dimethyl-4-(2-phenoxybenzoyl)-1H-pyrrole-2-carboxylate;
Methyl 5-Methyl-4-(2-(phenylmethyl)benzoyl)-1H- pyrrole-3-carboxylate;
Methyl 2,5-dimethyl-4-(1-oxo-4-phenylbutyl)-1H-pyrrole-3-carboxylate;
Methyl 5-((Acetyloxy)methyl)-2-methyl-4-(2-(phenylmethyl)benzoyl)-1H-pyrrole-3-carboxylate;
Methyl 2-((Acetyloxy)methyl)-5-methyl-4-(2-(phenylmethyl)benzoyl-1H-pyrrole-3-carboxylate;
Methyl 5-(Hydroxymethyl)-2-methyl-4-(2-(phenyl methyl)benzoyl)-1H-pyrrole-3-carboxylate;
Methyl 2-(Hydroxymethyl)-5-methyl-4-(2-(phenyl methyl)benzoyl)-1H-pyrrole-3-carboxylate;
Methyl 4-(5-(Acetylamino)-2-chlorobenzoyl)-2,5-dimethyl-1H-pyrrole-3-carboxylate;
Methyl 4-(2-((Ethylamino)carbonyl)benzoyl)-2,5-dimethyl-1H-pyrrole-3-carboxylate;
N-Ethyl 4-(2-(methoxycarbonyl)benzoyl)2,5-dimethyl -1H-pyrrole-3-carboxamide;
Methyl 4-(2-Chloro-5-(methylthio)benzoyl)-2,5-dimethyl-1H-pyrrole-3-carboxylate;
(Z)- and (E)-Methyl 5-((hydroxyimino)methyl)-2-methyl-4-(2-(pheynlmethyl)benzoyl)-1H-pyrrole-3-carboxylate;
(E) Methyl 5-(((aminocarbonyl)hydrazono)methyl)-2-methyl-4-(2-(phenylmethyl)benzoyl)-1H-pyrrole-3-carboxylate;
Methyl 2,5-dimethyl-4-(((phenylmethylene)hydrazino carbonyl)-1H-pyrrole-3-carboxylate;
Methyl 2,5-Dimethyl-4-(2-(phenylsulphinyl)benzoyl)-1H-pyrrole-3-carboxylate;
Methyl 2,5-Dimethyl-4-(2-(phenylsulphonyl)benzoyl)-1H-pyrrole-3-carboxylate;
Methyl 4-(2-Chlorobenzoyl)-2-methyl-5-(methyl sulphinyl)-1H-pyrrole-3-carboxylate;
Methyl 4-(2-Chlorobenzoyl)-2-methyl-5-(methyl sulphonyl)-1H-pyrrole-3-carboxylate;
(2-Chlorophenyl) (2,5-Dimethyl-4-(methylsulphinyl)-1H-pyrrole-3-yl)methanone
(2-Chlorophenyl) (2,5-Dimethyl-4-(methylsulphonyl)-1H-pyrrol-3-yl)methanone
Methyl 2,5-Dimethyl-4-(2-((phenylmethyl)sulphinyl) benzoyl)-1H-pyrrole-3-carboxylate;
Methyl 2,5-Dimethyl-4-(2-((phenylmethyl)sulphonyl) benzoyl)-1H-pyrrole-3-carboxylate;
Methyl 4-(2-Chloro-5-(methylsulphinyl)benzoyl)-2,5-dimethyl-1H-pyrrole-3-carboxylate;
Methyl 4-(2-Chloro-5-(methylsulphonyl)benzoyl)-2,5-dimethyl-1H-pyrrole-3-carboxylate;
Methyl 5-Ethyl-2-methyl-4-(2-((phenylmethyl) sulfinyl)benzoyl)-1H-pyrrole-3-carboxylate;
Methyl 2,5-dimethyl-4-((2-(phenylmethyl)phenyl) sulphinyl)-1H-pyrrole-3-carboxylate;
or a pharmaceutically acceptable derivative of any one thereof.

Claims for the following contracting State: GR

1. A process for the preparation of a compound of formula I,

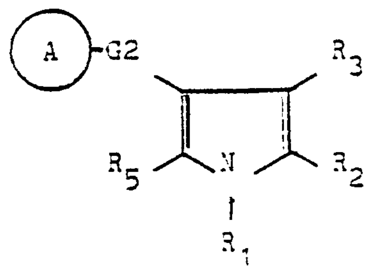

I

wherein
$R_1$ represents $-R_{11}$, $-NHR_{11}$ or $-NHCOOR_{11}$ in which $R_{11}$ is hydrogen or alkyl $C_{1-6}$,
$R_2$ and $R_5$, which may be the same or different, represent
hydrogen, $-R_{21}$, -OH, $-OR_{21}$, $-NR_{22}R_{23}$, halogen, -CN, $-NO_2$, $-S(O)_qR_{21}$, $-N=NR_{24}$, $-COOR_{22}$, $-CH=R_{25}$, phenyl or pyridyl,
in which $R_{21}$ is alkyl or alkenyl $C_{1-6}$ optionally interrupted by O or S and optionally substituted by -OH, halogen, -CN, $-NO_2$, $-NR_{22}R_{23}$, $-COOR_{22}$, phenyl or $-OOCR_{26}$,
$R_{22}$ and $R_{23}$, which may be the same or different, are hydrogen or alkyl $C_{1-6}$,
$R_{24}$ is phenyl optionally substituted by $-NO_2$,
$R_{25}$ is =O, =CHCN, =NOH or $=NNHCONH_2$,
$R_{26}$ is alkyl $C_{1-6}$, and
q is 0, 1 or 2,
G2 represents a chain $-(CH_2)_z(W)_y-$, in which,
W represents C=O, $S(O)_q$ or $C=NR_{40}$,
in which $R_{40}$ is hydrogen, -OH, cycloalkyl $C_{3-8}$ or alkyl $C_{1-6}$ optionally substituted by phenyl,
y is 0, 1 or, provided W is C=O, 2,
z is 0, 1, 2 or 3, and
up to two of the methylene segments in the chain $(CH_2)_z$ are optionally replaced by -NH- and one segment is optionally replaced by -O- or $-(C=NR_{40})-$, and the chain is optionally unsaturated and optionally substituted by alkyl $C_{1-6}$ or alkoxy $C_{1-6}$,
A is a 5- or 6-membered ring or a bicyclic or tricyclic fused ring system, A being optionally substituted by one or more of halogen, -OH, $-NO_2$, $-NR_{43}R_{45}$, $-COOR_{43}$, $-S(O)_qR_{44}$, $-CONR_{43}R_{45}$, $-NHCOR_{45}$, alkyl, alkylene or alkoxy $C_{1-6}$ optionally substituted by halogen, phenyl, $-COOR_{43}$ or $-NR_{43}R_{45}$, or a group D-G3-, in which
$R_{43}$ and $R_{45}$, which may be the same or different, are hydrogen or alkyl $C_{1-6}$,
$R_{44}$ is alkyl $C_{1-6}$,
G3 is a single bond, -NH-, -O-, -CO-, $-OCH_2-$, -O-CO-, $-S(O)_q-$, $(CH_2)_p-$, $-CH_2S(O)_q-$, -CO-S-, $-SO_2-O-$ or $CH_2NH-$, in which p is 1,2 or 3, and
D represents a 5- or 6-membered ring optionally substituted by -OH, halogen, $-NO_2$, $-NR_{43}R_{45}$, alkyl $C_{1-6}$, alkoxy $C_{1-6}$ or $-SCH_2C_6H_5$, and
$R_3$ represents hydrogen, $-S(O)_qR_{31}$, $-NO_2$, -CN, -halogen, $-CH_2NR_{32}R_{33}$, $-(CO)_rR_{34}$ or an aryl group, in which
$R_{31}$ is alkyl $C_{1-6}$,
$R_{32}$ and $R_{33}$, which may be the same or different, are hydrogen, alkyl $C_{1-6}$ optionally substituted by phenyl, or phenyl optionally substituted by halogen or trihalomethyl,
r is 1 or 2,
$R_{34}$ is hydrogen, $-NR_{32}R_{33}$, alkyl $C_{1-6}$, -OH, $-OR_{35}$ or $-SR_{35}$,
$R_{35}$ is cycloalkyl $C_{3-8}$ or alkyl $C_{1-6}$ optionally substituted by alkoxy $C_{1-6}$, halogen, -OH, cycloalkyl $C_{3-8}$, $-NR_{32}R_{33}$ or $-COOR_{36}$, and
$R_{36}$ is hydrogen or alkyl $C_{1-6}$, or $R_3$ and $R_2$ together form a chain -N=CH-NH-CO-,
<u>provided that</u>
a) when $R_3$ represents hydrogen, <u>then</u> A is substituted by D-G3- where, if A is a 5-membered heterocyclic ring, D-G3- is other than phenyl, benzyl or phenyl substituted by chlorine, or, if A is phenyl or naphthyl and D is phenyl, G3 is other than $-CH_2O-$, -S-, -O-or -NH-;

b) when $R_2$ and $R_5$ both represent hydrogen, then A is substituted by D-G3-;

c) when $R_1$ represents alkyl $C_{1-6}$ or $-NHCOOR_{11}$, and $R_2$ and $R_5$ are both alkyl $C_{1-6}$, and $R_3$ is $-COOR_{35}$ or $-NO_2$, then A is other than unsubstituted phenyl or phenyl substituted by fluorine;

d) when $R_2$ and $R_3$ both represent halogen, and A is phenyl, then A is substituted by D-G3-;

e) when one or both of $R_2$ and $R_5$ represents phenyl, and $R_3$ represents $-COOR_{35}$, $-COCH_3$, phenyl or $-NO_2$, then A is other than unsubstituted phenyl, isoxazolyl substituted by alkyl $C_{1-6}$ or phenyl, or a group

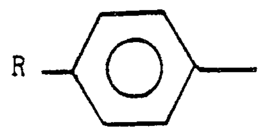

where R is halogen, alkyl $C_{1-6}$ or alkoxy $C_{1-6}$

f) when $R_5$ represents hydrogen, and $R_2$ represents alkyl $C_{1-6}$, and $R_3$ represents $-COOR_{35}$, and A is a 5- or 6-membered ring, then A is substituted by D-G3-; and

g) when $R_2$ and $R_5$ are both selected from hydrogen, $-COOR_{22}$ and alkyl $C_{1-6}$ optionally substituted by halogen, -OH, $-NR_{22}R_{23}$ or $-OOCR_{26}$ and optionally interrupted by O or S, and $R_3$ is selected from $-NO_2$, halogen, -CN, $-COR_{34}$ or $-CH_2NR_{32}R_{34}$, where $R_{34}$ is hydrogen, $-NR_{32}R_{34}$, alkyl $C_{1-6}$, -OH or $-OR_{35}$ where $R_{35}$ is alkyl $C_{1-6}$ optionally substituted by an aryl group, and $R_1$ represents hydrogen or alkyl $C_{1-6}$, and G2 represents $-(CH_2)_z(W)_y-$ in which z represents 0, 1 or 2 and none of the methylene segments are replaced by -NH-,-O- or $-(C=NR_{40})-$ and the chain is neither unsaturated not substituted by alkoxy $C_{1-6}$, then A is other than phenyl, naphthyl or benzofurazanyl optionally substituted only by halogen, -OH, $-NR_{43}R_{45}$, $-NO_2$, -COOH, alkyl or alkoxy $C_{1-6}$ optionally substituted by halogen, or D-G3- where G3 is $-SCH_2-$, $-OCH_2-$ or -O-CO- and D is phenyl optionally substituted by -OH, halogen or alkyl or alkoxy $C_{1-6}$;

or a pharmaceutically acceptable derivative thereof, which process comprises

a) producing a compound of formula I in which $R_3$ or G2 includes a carbonyl group adjacent to the pyrrole ring by reacting a compound of formula IIa or IIb,

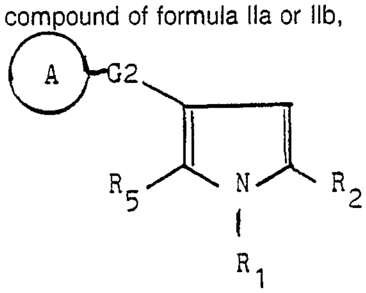

IIa

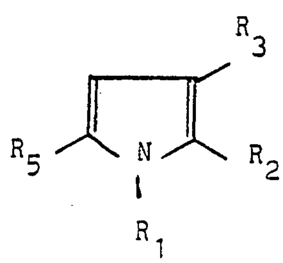

IIb

with a compound of formula IIIa or IIIb respectively,

$R_{3a}-CO-L_a$ IIIa

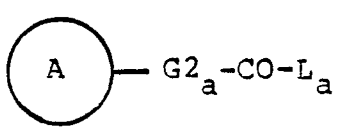

IIIb

in which $R_{3a}CO$- and $G2_aCO$- represent groups $R_3$ and G2 respectively and $L_a$ is a leaving group, or
b) producing a compound of formula I which is substituted by D-G3c- or alkylene $C_{1-6}$ optionally substituted by $-COOR_{4}$· or $-NR_{43}R_{45}$ in which -G3c- represents -NH-, -S-, $-CH_2NH$- or alkylene $C_{1-6}$,
by reacting a compound of formula I substituted by a leaving group with a compound of formula IV,

$Ar_c$-H IV

in which $Ar_c$- represents D-G3c- or alkylene $C_{1-6}$ optionally substituted by $-COOR_{43}$ or $-NR_{43}R_{45}$, or
c) producing a compound of formula I which contains a substituent halogen, -CHO, -CN, $-NO_2$, $-S(O)_qR_{21}$, $-CONH_2$, $-CH_2NR_{32}R_{33}$ or $-N=N-R_{24}$,
by reacting a corresponding compound containing a hydrogen atom with an appropriate electrophile and, where necessary, appropriate transformation of the product, or
d) producing a compound of formula I in which one or both of $R_2$ and $R_5$ represents hydrogen,
by reacting a compound of formula V

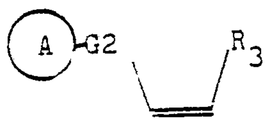

V

with a compound of formula VI

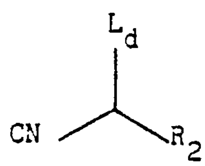

VI

in which $L_d$ represents a leaving group, or
e) reacting a compound of formula VII

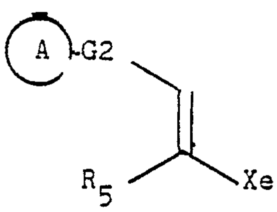

VII

with a compound of formula VIII

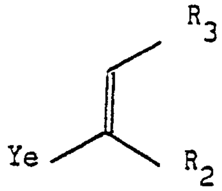

VIII

in which one of $X_e$ and $Y_e$ represents a leaving group, and the other represents $-NHR_1$, and where necessary, working up the reaction product, or
f) reacting a compound of formula IX,

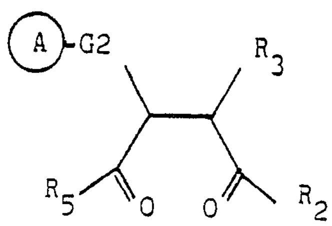

IX

with a compound of formula X,

$R_1$-$NH_2$ X

or

g) removal of a protecting group from a compound of formula I which bears a protecting group, or

h) producing a compound of formula I in which G2 includes a group $-(CO)_y-$ adjacent to the pyrrole ring, by reacting a compound of formula XI,

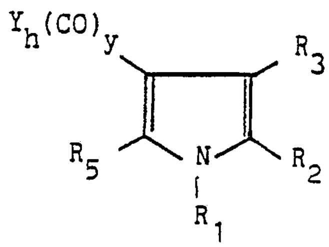

XI

with a compound of formula XII

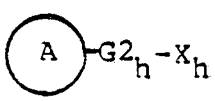

XII

in which $G2_h$ is a group which, in combination with the group $-(CO)_y-$ forms G2 and one of $X_h$ and $Y_h$ is a leaving group and the other is hydrogen, and, if necessary, working up the reaction product, or

i) producing a compound of formula I in which A is a 5-or 6-membered ring or a bicyclic or tricyclic fusing ring system by appropriate treatment of a corresponding compound in which

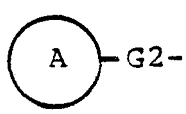

contains groups cyclisable to form such a ring or fused ring system respectively, or

j) producing a compound of formula I in which G2 represents $-NHC(=NH)NH-(CO)_y-$ by reacting a compound of formula XIII,

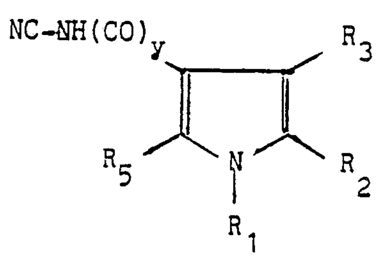

XIII

with a compound of formula XIV,

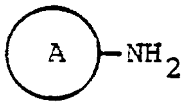

XIV

or

k) producing a compound of formula I containing a group $-COOR_k$ or $-COSR_k$ in which $R_k$ represents cycloalkyl $C_{3-8}$ or alkyl $C_{1-6}$ optionally substituted by halogen, $-NR_{32}R_{33}$ or $-COOR_{35}$,
by esterification or thioesterification respectively of the corresponding compound containing a group -COOH, or
l) producing a compound of formula I containing a substituent - $NH_2$,
by reduction of the corresponding compound containing a substituent $-NO_2$, or
m) producing a compound of formula I containing a group -COOH
by hydrolysis of a corresponding ester, or
n) deamination of a compound of formula I containing a substituent $-NH_2$, or
o) producing a compound of formula I containing an alkyl $C_{1-6}$ group substituted by a group $-OOCR_{26}$,
by alkanoyloxylation of the alkyl $C_{1-6}$ group, or
p) producing a compound of formula I containing a hydroxy group, or
by reacting the corresponding compound containing a group $-OOCR_{26}$ with a hydroxylic base,
q) producing a compound of formula I containing an amido group,
by acylation of the corresponding amine or by reacting a corresponding carboxylic acid or carboxylic acid derivative with an appropriate amine, or
r) producing a compound of formula I containing a group $-SR_r$ in which $R_r$ represents $C_{1-6}$ by diazotisation of the corresponding compound containing a group $-NH_2$ and subsequent displacement, or
s) producing a compound of formula I containing an aliphatic -CH=N- group by condensation of the appropriate aldehyde and amine, or
t) producing a compound of formula I containing a group -SO- or $-SO_2-$,
by oxidation of the corresponding compound containing a group -S- or -SO-,
and, where necessary or desired thereafter, converting the compound of formula I so obtained to a pharmaceutically acceptable derivative thereof, or vice versa.

2. A process according to Claim 1, wherein $R_3$ represents $-COOR_{35}$ or $-NO_2$.

3. A process according to Claim 1 or Claim 2, wherein $R_2$ and $R_5$ are selected from the group consisting of hydrogen, $-R_{21}$ and halogen.

4. A process according to any one of the preceding claims, wherein A is a 5- or 6-membered ring.

5. A process according to Claim 4, wherein A is substituted by D-G3-.

6. A process according to any one of the preceding claims, in which
$R_2$ and $R_5$, which may be the same or different, represent hydrogen, $-R_{21}$, or halogen,
$R_3$ represents $-NO_2$ or $-COOR_{35}$,
G2 represents C=O, -S- or a single bond, and
A represents phenyl substituted by a group D-G3-.

7. A process according to any one of the preceding claims, in which
$R_1$ represents hydrogen,
$R_2$ and $R_5$, which may be the same or different, represent hydrogen, $-R_{21}$, or halogen,
$R_3$ represents $-NO_2$ or $-COOR_{35}$,
G2 represent C=O, and
A represents phenyl substituted by a group D-G3-, in which G3 represents -NH- or $-CH_2-$.

8. A process according to Claim 1, in which the compound of formula I is
Methyl 2,5-Dimethyl-4-(2-((4-nitrophenyl)thio) benzoyl)-1H-pyrrole-3-carboxylate;
Methyl 2,5-Dimethyl-4-(2-((4-nitrophenyl)methyl) benzoyl)-1H-pyrrole-3-carboxylate;
Methyl 2,5-Dimethyl-4-(2-((2-nitrophenyl)methyl) benzoyl)-1H-pyrrole-3-carboxylate;
Methyl 2,5-Dimethyl-4-(2-(2-phenylethyl)benzoyl)-1H-pyrrole-3-carboxylate;
Methyl 2,5-Dimethyl-4-(2-(4-nitrophenoxy)benzoyl)-1H-pyrrole-3-carboxylate;
Methyl 2,5-Dimethyl-4-(2-(3-nitrobenzoyl)benzoyl)-1H-pyrrole-3-carboxylate;
Methyl 2,5-Dimethyl-4-(2-(((4-methylphenyl) sulphonyl)oxy)benzoyl)-1H-pyrrole-3-carboxylate;
Methyl 4-(2-Chlorobenzoyl)-2-methyl-5-(methylthio)-1H-pyrrole-3-carboxylate;

Methyl 2,5-Dimethyl-4-(2-(4-pyridinylamino)benzoyl) -1H-pyrrole-3-carboxylate;
Methyl 4-(2-(Cyclohexylmethyl)benzoyl)-2,5-dimethyl -1H-pyrrole-3-carboxylate;
Methyl 4-(2-Chlorobenzoyl)-2-methyl-5-phenyl-1H-pyrrole-3-carboxylate;
Methyl 4-(2-Chlorobenzoyl)-2,5-dimethyl-α-oxo-1H-pyrrole-3-acetate;
(2-Chlorophenyl) (2,5-Dimethyl-4-(5-methyl-1,3,4-oxadiazol-2-yl)-1H-pyrrol-3-yl)methanone;
Methyl 4-(2-(2-(Dimethylamino)ethoxy)benzoyl)-2,5-dimethyl-1H-pyrrole-3-carboxylate;
Methyl 2,5-Dimethyl-4-(2-((2-((phenylmethyl)thio) benzoyl)thio)benzoyl)-1H-pyrrole-3-carboxylate;
Methyl 4-(2-(Methoxycarbonyl)benzoyl)-2,5-dimethyl-1H-pyrrole-3-carboxylate;
Methyl 3-(2-Chlorobenzoyl)-4-(methoxycarbonyl)-5-methyl-1H-pyrrole-2-propanoate;
Methyl 4-(2-Chlorobenzoyl)-5-methyl-2-(2-nitroethyl) -1H-pyrrole-3-carboxylate;
Methyl 2,5-Dimethyl-4-(3-pyridinylcarbonyl)-1H-pyrrole-3-carboxylate;
Methyl 2,5-Dimethyl-4-(4-pyridinylcarbonyl)-1H-pyrrole-3-carboxylate;
5-(2-Chlorobenzoyl)-3,4-dihydro-6-methyl-7H- pyrrolo(2,3-d)pyrimidin-4-one;
(E)Methyl 4-(3-methoxy-1-oxo-4-phenyl-2-butenyl)-2,5-dimethyl-1H-pyrrole-3-carboxylate;
(2,5-Dimethyl-1H-pyrrol-3-yl) (2-(phenylmethyl) phenyl)methanone;
Methyl 2,5-Dimethyl-4-(2-(phenylthio)benzoyl)-1H-pyrrole-3-carboxylate;
Methyl 2,5-Dimethyl-4-(2-(phenylamino)benzoyl)-1H-pyrrole-3-carboxylate;
Methyl 2,5-Dimethyl-4-(2-(2-pyridinylamino)benzoyl) -1H-pyrrole-3-carboxylate;
Methyl 5-Ethyl-2-methyl-4-(2-(phenylamino)benzoyl)-1H-pyrrole-3-carboxylate;
Methyl 2,5-dimethyl-4-(2-(cyclohexylamino)benzoyl)-1H-pyrrole-3-carboxylate;
Methyl 2,5-dimethyl-4-(2-((phenylmethyl)amino)benzoyl) -1H-pyrrole-3-carboxylate;
Methyl 2,5-dimethyl-4-(2-((phenylmethyl)amino)phenyl) ((phenylmethyl)imino)methyl)-1H-pyrrole-3-carboxylate;
Methyl 4-((2-(cyclohexylamino)phenyl) (cyclohexyl imino)methyl-2,5-dimethyl-1H-pyrrole-3-carboxylate;
(E)-Methyl 2,5-dimethyl-4-(2-(2-phenylethenyl) benzoyl)-1H-pyrrole-3-carboxylate;
E-3-(2-((4-(Methoxycarbonyl)-2,5-dimethyl-1H-pyrrol-3-yl)carbonyl)phenyl)-2-propenoic acid;
Methyl 5-(2-(methoxycarbonyl)ethenyl)-2-methyl-4-(2-(phenylmethyl)benzoyl)-1H-pyrrole-3-carboxylate;
Methyl 2-methyl-5-phenyl-4-(2-(phenylmethyl)benzoyl) -1H-pyrrole-3-carboxylate;
Methyl 2-methyl-5-(phenylmethyl)-4-(2-(phenylmethyl) benzoyl)-1H-pyrrole-3-carboxylate;
Methyl 2-Bromo-4-(2-chlorobenzoyl)-5-methyl-1H-pyrrole-3-carboxylate;
(4-Bromo-2,5-dimethyl-1H-pyrrol-3-yl) (2-(phenylmethyl) phenyl)methanone;
Methyl 2-Bromo-5-methyl-4-(2-phenylmethyl)benzoyl)-1H-pyrrole-3-carboxylate;
Methyl 5-Bromo-2-methyl-4-(2-phenylmethyl)benzoyl)-1H-pyrrole-3-carboxylate;
Methyl 4-(2-Chlorobenzoyl)-2-cyano-5-methyl-1H-pyrrole-3-carboxylate;
2,5-Dimethyl-4-(2-(phenylmethyl)benzoyl)-1H-pyrrole-3-carbonitrile;
Methyl 2-Cyano-5-methyl-4-(2-(phenylmethyl)benzoyl)-1H-pyrrole-3-carboxylate;
Methyl 5-Cyano-2-methyl-4-(2-(phenylmethyl)benzoyl)-1H-pyrrole-3-carboxylate;
2,5-Dimethyl-4-(2-(phenylmethyl)benzoyl)-1H-pyrrole-3-carboxamide;
Methyl 4-(2-Chlorobenzoyl)-2-formyl-5-methyl-1H- pyrrole-3-carboxylate;
Methyl 5-Formyl-2-methyl-4-(2-(phenylmethyl)benzoyl)-1H-pyrrole-3-carboxylate;
Methyl 2-Formyl-5-methyl-4-(2-(phenylmethyl)benzoyl)-1H-pyrrole-3-carboxylate;
2,5-Dimethyl-4-(2-phenylmethyl)benzoyl)-1H-pyrrole-3-carboxaldehyde;
Methyl 4-(2-Chlorobenzoyl)-5-methyl-2-nitro-1H-pyrrole-3-carboxylate;
(2,5-Dimethyl-4-nitro-1H-pyrrol-3-yl) (2-(phenylmethyl)phenyl)methanone;
Methyl 4-(2-Chlorobenzoyl)-5-methyl-2-((4-nitrophenyl) azo)-1H-pyrrole-3-carboxylate;
(2-Chlorophenyl) (2,5-Dimethyl-4-(methylthio)-1H-pyrrol-3-yl)methanone;
Methyl 2-methyl-5-((4-nitrophenyl)azo)-4-(2-(phenylmethyl)benzoyl)-1H-pyrrole-3-carboxylate;
Methyl 5-methyl-2-((4-nitrophenyl)azo)-4-(2-(phenylmethyl)benzoyl)-1H-pyrrole-3-carboxylate;
(4-((Dimethylamino)methyl)-2,5-dimethyl-1H-pyrrol-3-yl) ((2-phenylmethyl)phenyl)methanone;
Methyl 5-iodo-2-methyl-4-(2-(phenylmethyl)benzoyl)-1H-pyrrole-3-carboxylate;
Methyl 2-Methyl-4-(2-(phenylmethyl)benzoyl)-1H-pyrrole-3-carboxylate;
Methyl 4-(2-(phenylmethyl)benzoyl)-1H-pyrrole-3-carboxylate;
Methyl 3-(Methoxycarbonyl)-5-methyl-4-(2-(phenylmethyl)benzoyl)-1H-pyrrole-2-acetate;
Methyl 5-Ethyl-2-methyl-4-(2-(phenylmethyl)benzoyl)-1H-pyrrole-3-carboxylate;
Methyl 2,5-Dimethyl-4-((1-(phenylmethyl)-1H-pyrrol-2-yl)carbonyl)-1H-pyrrole-3-carboxylate;
Ethyl 4-(2-Chlorobenzoyl)-1-((methoxycarbonyl)amino)-2,5-dimethyl-1H-pyrrole-3-carboxylate;
Methyl 4-(2-Chlorobenzoyl)-1-(((1,1-dimethylethoxy) carbonyl)amino)-2,5-dimethyl-1H-pyrrole-3-carboxylate;
Methyl 1-Amino-4-(2-chlorobenzoyl)-2,5-dimethyl-1H-pyrrole-3-carboxylate;

Methyl 2,5-Dimethyl-4-((2-methyl-1-piperidinyl) carbonyl)-1H-pyrrole-3-carboxylate;
Methyl 2,5-Dimethyl-4-((1-(phenylmethyl)-1H-imidazol-2-yl)carbonyl)1H-pyrrole-3-carboxylate;
Methyl 5-Ethyl-2-methyl-4-((1-(phenylmethyl)-1H-pyrrol-2-yl)carbonyl)-1H-pyrrole-3-carboxylate;
Methyl 2-methyl-4-(2-phenylmethyl)benzoyl)-5-trifluoromethyl-1H-pyrrole-3-carboxylate;
Methyl 4-(2-((4-methoxyphenyl)methyl)benzoyl)-2,5-dimethyl-1H-pyrrole-3-carboxylate;
Methyl 4-((9H-fluoren-1-yl)carbonyl)-2,5-dimethyl-1H- pyrrole-3-carboxylate;
Methyl 2-methyl-4,5-di (2-pyridinyl)-1H-pyrrole-3-carboxylate;
(2-Chlorophenyl) (2,5-Dimethyl-4-phenyl-1H-pyrrol-3-yl)methanone;
Methyl 4-(4-(1H-imidazol-1-yl)phenyl)-2,5-dimethyl-1H-pyrrole-3-carboxylate;
Methyl 2,5-dimethyl-4-(2-pyridinyl)-1H-pyrrole-3-carboxylate;
Methyl 2,5-Dimethyl-4-(2-(phenylmethyl)benzoyl)-1H-pyrrole-3-carboxylate;
Methyl 2,5-Dimethyl-4-(2-phenylbenzoyl)-1H-pyrrole -3-carboxylate;
(E)Methyl 5-(2-cyanoethenyl)-2-methyl-4-(2-(phenyl methyl)benzoyl)-1H-pyrrole-3-carboxylate;
Methyl 2,5-dimethyl-4-((2-phenylmethyl)phenyl)thio) -1H-pyrrole-3-carboxylate;
Methyl 4-(((2-(3,4-dimethoxyphenyl)ethyl)amino) carbonyl)-2,5-dimethyl-1H-pyrrole-3-carboxylate;
Methyl 4-(((1H-benzimidazol-2-yl)amino)carbonyl)-2,5-dimethyl-1H-pyrrole-3-carboxylate;
Methyl 2,5-dimethyl-4-(((2-(4-morpholinyl)ethyl)amino) carbonyl)-1H-pyrrole-3-carboxylate;
Methyl 2,5-dimethyl-4-(oxo((phenylmethyl)amino) acetyl)-1H-pyrrole-3-carboxylate;
Methyl 4-(((2-benzothiazolyl)amino)carbonyl)-2,5-dimethyl-1H-pyrrole-3-carboxylate;
Methyl 4-(3,4-dihydro-6,7-dimethoxy-1-isoquinolinyl)-2,5-dimethyl-1H-pyrrole-3-carboxylate;
Methyl 4-(5-anthracenyl)-2-formyl-5-methyl-1H-pyrrole-3-carboxylate;
Methyl 4-(5-anthracenyl)-5-formyl-2-methyl-1H-pyrrole-3-carboxylate;
Methyl 4-(11H-dibenz(b.e)azepin-6-yl)-5-methyl-1H-pyrrole-3-carboxylate;
Methyl 2,5-dimethyl-4-(2,3,4,5-tetrahydro-1H-benzazepin-2-yl)-1H-pyrrole-3-carboxylate;
Methyl 2,5-dimethyl-4-(4,5-dihydro-3H-benzazepin-2-yl)-1H-pyrrole-3-carboxylate;
Methyl 4-(3,4-dihydro-1-isoquinolinyl))-2,5-dimethyl-1H-pyrrole-3-carboxylate;
(±) Methyl 4-(4,5-dihydro-5-phenyl-2-oxazolyl)-2,5-dimethyl-1H-pyrrole-3-carboxylate;
(S) and (R) Methyl 4-(4,5-dihydro-4-phenyl-2-oxazolyl)-2,5-dimethyl-1H-pyrrole-3-carboxylate;
Methyl 2,5-dimethyl-4-(2-quinolinyl)-1H-pyrrole-3-carboxylate;
Methyl 2,5-dimethyl-4-(2-phenyl-1H-imidazol-4-yl)-1H-pyrrole-3-carboxylate;
Methyl 4-((imino(phenylamino)methyl)amino) carbonyl)-2,5-dimethyl-1H-pyrrole-3-carboxylate;
1-Methylethyl 2,5-Dimethyl-4-(2-(phenylmethyl) benzoyl)-1H-pyrrole-3-carboxylate;
2-Methoxyethyl 2,5-Dimethyl-4-(2-(phenylmethyl) benzoyl)-1H-pyrrole-3-carboxylate;
Cyclopropylmethyl 2,5-Dimethyl-4-(2-(phenylmethyl) benzoyl)-1H-pyrrole-3-carboxylate;
2-Chloroethyl 2,5-Dimethyl-4-(2-(phenylmethyl)benzoyl) -1H-pyrrole-3-carboxylate;
2,2,2-Trifluoroethyl 2,5-Dimethyl-4-(2-(phenylmethyl) benzoyl-1H-pyrrole-3-carboxylate;
S-Methyl 2,5-Dimethyl-4-(2-(phenylmethyl)benzoyl)-1H-pyrrole-3-carbothioate;
Cyclopentyl 4-(2-Chlorobenzoyl)-2,5-dimethyl-1H-pyrrole-3-carboxylate;
2-(Dimethylamino)ethyl 4-(2-Chlorobenzoyl)-2,5-dimethyl-1H-pyrrole-3-carboxylate;
(2-Methoxy-2-oxoethyl) 2,5-dimethyl-4-(2-(phenyl methyl)benzoyl)-1H-pyrrole-3-carboxylate;
Phenylmethyl 4-(methoxycarbonyl)-2,5-dimethyl-α -oxo-1H-pyrrole-3-acetate;
Methyl 4-(2-((4-Aminophenyl)methyl)benzoyl)-2,5-dimethyl-1H-pyrrole-3-carboxylate;
Methyl 4-(2-(3-Aminobenzoyl)benzoyl)-2,5-dimethyl-1H-pyrrole-3-carboxylate;
Methyl 4-(2-(4-Aminophenoxy)benzoyl)-2,5-dimethyl-1H-pyrrole-3-carboxylate;
Methyl 2-Amino-4-(2-chlorobenzoyl)-5-Methyl-1H-pyrrole-3-carboxylate;
Methyl 2-amino-5-methyl-4-(2-(phenylmethyl)benzoyl)-1H-pyrrole-3-carboxylate;
Methyl 5-amino-2-methyl-4-(2-(phenylmethyl)benzoyl-1H-pyrrole-3-carboxylate;
2,5-Dimethyl-4-(2-(phenylmethyl)benzoyl)-1H-pyrrole3-carboxylic acid;
5-Ethyl-2-methyl-4-(2-(phenylmethyl)benzoyl)-1H-pyrrole-3-carboxylic acid;
Carboxymethyl 2,5-dimethyl-4-(2-(phenylmethyl) benzoyl))-1H-pyrrole-3-carboxylate;
4-(2-(Phenylmethyl)benzoyl)-3-methoxycarbonyl-5-methyl-1H-pyrrole-2-acetic acid;
E-Methyl 5-(2-carboxyethenyl)-2-methyl-4-(2-(phenylmethyl)benzoyl)-1H-pyrrole-3-carboxylate;
4-(2-(Methoxycarbonyl)benzoyl)-2,5-dimethyl-1H-pyrrole-3-carboxylic acid;
Methyl 4-(2-Benzoylbenzoyl)-2,5-dimethyl-1H-pyrrole-3-carboxylate;
Methyl 2,5-Dimethyl-4-(2-phenoxybenzoyl)-1H-pyrrole-2-carboxylate;
Methyl 5-Methyl-4-(2-(phenylmethyl)benzoyl)-1H- pyrrole-3-carboxylate;
Methyl 2,5-dimethyl-4-(1-oxo-4-phenylbutyl)-1H-pyrrole-3-carboxylate;
Methyl 5-((Acetyloxy)methyl)-2-methyl-4-(2-(phenylmethyl)benzoyl)-1H-pyrrole-3-carboxylate;

Methyl 2-((Acetyloxy)methyl)-5-methyl-4-(2-(phenylmethyl)benzoyl-1H-pyrrole-3-carboxylate;
Methyl 5-(Hydroxymethyl)-2-methyl-4-(2-(phenyl methyl)benzoyl)-1H-pyrrole-3-carboxylate;
Methyl 2-(Hydroxymethyl)-5-methyl-4-(2-(phenyl methyl)benzoyl)-1H-pyrrole-3-carboxylate;
Methyl 4-(5-(Acetylamino)-2-chlorobenzoyl)-2,5-dimethyl-1H-pyrrole-3-carboxylate;
Methyl 4-(2-((Ethylamino)carbonyl)benzoyl)-2,5-dimethyl-1H-pyrrole-3-carboxylate;
N-Ethyl 4-(2-(methoxycarbonyl)benzoyl)2,5-dimethyl -1H-pyrrole-3-carboxamide;
Methyl 4-(2-Chloro-5-(methylthio)benzoyl)-2,5-dimethyl-1H-pyrrole-3-carboxylate;
(Z)- and (E)-Methyl 5-((hydroxyimino)methyl)-2-methyl-4-(2-(pheynlmethyl)benzoyl)-1H-pyrrole-3-carboxylate;
(E) Methyl 5-(((aminocarbonyl)hydrazono)methyl)-2-methyl-4-(2-(phenylmethyl)benzoyl)-1H-pyrrole-3-carboxylate;
Methyl 2,5-dimethyl-4-(((phenylmethylene)hydrazino carbonyl)-1H-pyrrole-3-carboxylate;
Methyl 2,5-Dimethyl-4-(2-(phenylsulphinyl)benzoyl)-1H-pyrrole-3-carboxylate;
Methyl 2,5-Dimethyl-4-(2-(phenylsulphonyl)benzoyl)-1H-pyrrole-3-carboxylate;
Methyl 4-(2-Chlorobenzoyl)-2-methyl-5-(methyl sulphinyl)-1H-pyrrole-3-carboxylate;
Methyl 4-(2-Chlorobenzoyl)-2-methyl-5-(methyl sulphonyl)-1H-pyrrole-3-carboxylate;
(2-Chlorophenyl) (2,5-Dimethyl-4-(methylsulphinyl)-1H-pyrrole-3-yl)methanone
(2-Chlorophenyl) (2,5-Dimethyl-4-(methylsulphonyl)-1H-pyrrol-3-yl)methanone
Methyl 2,5-Dimethyl-4-(2-((phenylmethyl)sulphinyl) benzoyl)-1H-pyrrole-3-carboxylate;
Methyl 2,5-Dimethyl-4-(2-((phenylmethyl)sulphonyl) benzoyl)-1H-pyrrole-3-carboxylate;
Methyl 4-(2-Chloro-5-(methylsulphinyl)benzoyl)-2,5-dimethyl-1H-pyrrole-3-carboxylate;
Methyl 4-(2-Chloro-5-(methylsulphonyl)benzoyl)-2,5-dimethyl-1H-pyrrole-3-carboxylate;
Methyl 5-Ethyl-2-methyl-4-(2-((phenylmethyl) sulfinyl)benzoyl)-1H-pyrrole-3-carboxylate;
Methyl 2,5-dimethyl-4-((2-(phenylmethyl)phenyl) sulphinyl)-1H-pyrrole-3-carboxylate;
or a pharmaceutically acceptable derivative of any one thereof.

9. A compound of formula I, as defined in Claim 1, for use as an intermediate in the synthesis of another chemical compound.

European Patent Office

**PARTIAL EUROPEAN SEARCH REPORT**
which under Rule 45 of the European Patent Convention shall be considered, for the purposes of subsequent proceedings, as the European search report

Application number
EP 88 30 6464

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 001 534 (A. ROLLAND S.A.) |  | C 07 D 207/34 |
|  | * Whole document * | 1-10 | C 07 D 207/333 |
|  | -- |  | C 07 D 403/06 |
| Y | EP-A-0 002 392 (TEIJIN LTD) |  | C 07 D 405/06 |
|  | * Abstract * | 1-10 | C 07 D 409/06 |
|  | -- |  | A 61 K 31/40 |
| Y | US-A-3 644 631 (I.J. PACHTER) |  |  |
|  | * Columns 1-3 * | 1-10 |  |
|  | -- |  |  |
| Y | GB-A-1 161 638 (ENDO LAB. INC.) |  |  |
|  | * Whole document * | 1-10 |  |
|  | -- |  |  |
| Y | FR-A-2 405 936 (A. ROLAND S.A.) |  |  |
|  | * Whole document * | 1-10 | TECHNICAL FIELDS SEARCHED (Int. Cl.4) |
|  | -- |  |  |
| X | CHEMICAL ABSTRACTS, vol. 106, 1987, page 701, ref.no. 176098j; Columbus, Ohio, US; ./. |  | C 07 D 207/00<br>C 07 D 401/00<br>C 07 D 405/00<br>C 07 D 409/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely: 1-10
Claims not searched:
Reason for the limitation of the search:

The considerably long list of substituents with their often numerous and/or cascading significances coupled with the extensive use - if not abuse - of the disclaimer technique makes that the present application hardly meets - if at all - the requirements set forth in either Art. 83 and Art. 84 ("description and claims shall be clear and concise") or art. 82 ("unity of invention") of the European Patent Convention.

| Place of search | Date of completion of the search | Examiner |
|---|---|---|
| The Hague | 21-09-1988 | MAISONNEUVE |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

European Patent Office

# PARTIAL EUROPEAN SEARCH REPORT

Application number EP 88 30 6464



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | M.R. PAVIA: "An improved preparation of 1,2,5-trimethyl-3-nitropyrrole. Friedel-Crafts acylation with 2-fluorobenzoyl chloride" & ORG. PREP. PROCED. INT. 1987, 19 (1), 48-52<br>* Whole abstract *<br>--------------- | 10 | |
| | | | TECHNICAL FIELDS SEARCHED (Int Cl.4) |